# DEMANDE DE BREVET EUROPEEN

(11) **EP 3 461 833 A1**
(43) Date de publication de la demande: **03.04.2019**
(21) Numéro de dépôt: 18205305.8
(22) Date de dépôt: 20.05.2015
(51) Int. Cl.: C07J 5/00, C07J 17/00, C07J 31/00, C07J 41/00, C07J 43/00, A61K 31/57, A61K 31/573, A61P 3/04, A61P 21/06

(54) **COMPOSÉS CHIMIQUES ET LEUR UTILISATION POUR L'AMÉLIORATION DE LA QUALITÉ MUSCULAIRE**

(30) Priorité: 20.05.2014 FR 1454538
(62) Demande divisionnaire de: 15732785.9
(71) Demandeur: Biophytis, 75001 Paris (FR); SORBONNE UNIVERSITE, 75006 Paris (FR)
(72) Inventeur: LAFONT, René, 75016 PARIS (FR); DIOH, Waly, 91220 BRETIGNY SUR ORGE (FR); RAYNAL, Sophie, 75016 PARIS (FR); VEILLET, Stanislas, 91600 Savigny sur Orge (FR); LEPIFRE, Franck, 91400 SACLAY (FR); DURAND, Jean-Denis, 93100 MONTREUIL SOUS BOIS (FR)
(74) Mandataire: Ipside

(57) **Abrégé**

La présente invention concerne des composés chimiques de la formula (I) (où Q represente un groupement >C=NOR5 ou >CH-NR2R3) et ces composés pour l'utilisation thérapeutique, en particulier pour l'amélioration de la qualité musculaire chez les mammifères. Plus particulièrement l'invention permet d'améliorer la qualité musculaire des mammifères sarcopéniques et de traiter et/ou prévenir la sarcopénie et en particulier l'obésité sarcopénique, ses complications et/ou pathologies associées telles que la perte de force, de masse musculaire, de performances et capacité physiques et de mobilité. L'invention permet également d'améliorer la qualité musculaire chez les mammifères obèses et traiter et/ou prévenir l'obésité et ses complications et/ou pathologies associées, avantageusement le diabète de type 2 et le syndrome métabolique.

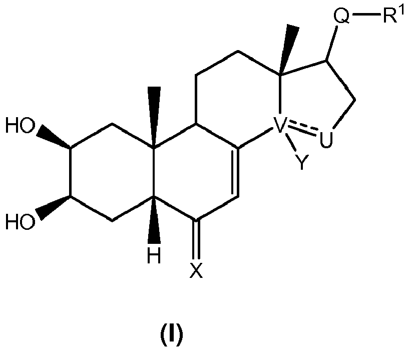

## Description

### Domaine de l'invention

La présente invention concerne des composés chimiques et leur utilisation thérapeutique, en particulier pour l'amélioration de la qualité musculaire chez les mammifères.

Plus particulièrement, l'invention permet d'améliorer la qualité musculaire chez les mammifères obèses.

L'invention permet également d'améliorer la qualité musculaire des mammifères sarcopéniques.

L'invention concerne également l'utilisation de ces composés chimiques dans le traitement et/ou la prévention de l'obésité chez le mammifère.

### Etat de la technique

L'atrophie musculaire peut résulter de plusieurs causes différentes : dénutrition, non utilisation des muscles (ex. immobilisation consécutive à une fracture), cancer ou autre maladie grave (insuffisance cardiaque ou rénale) induisant la cachexie, ou résultant naturellement du vieillissement des individus (sarcopénie). Cette atrophie peut résulter d'une réduction de la protéosynthèse ou/et d'une augmentation de la protéolyse et, selon les cas, s'accompagner d'une fibrose ou/et d'une infiltration par du tissu adipeux. L'identification des facteurs et mécanismes contrôlant la protéosynthèse et la protéolyse musculaires représente donc un pré-requis pour concevoir des traitements appropriés de ces pathologies.

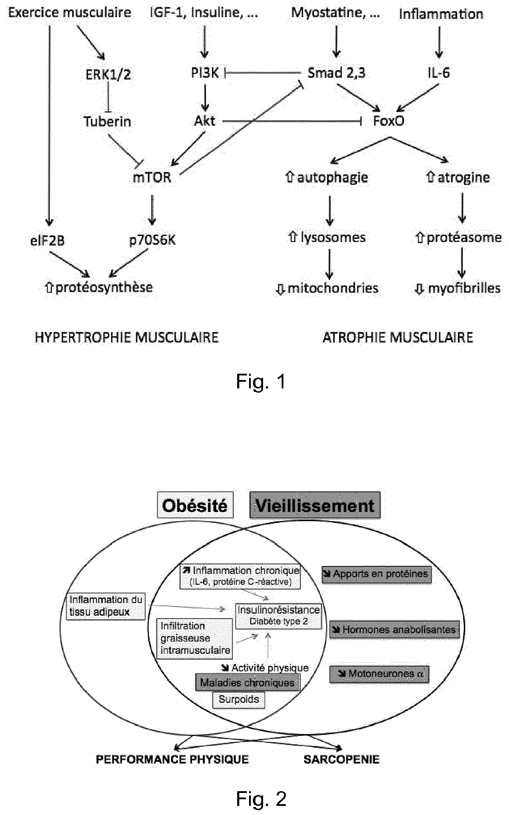

La Figure 1, faisant partie de l'état de la technique, montre les principales voies de la protéosynthèse et de la protéolyse dans les muscles (recomposé d'après Zhao et al., 2008 et Little et al., 2009).

La protéosynthèse musculaire est indispensable, et est essentiellement contrôlée au niveau traductionnel. Elle nécessite bien évidemment un apport nutritionnel adéquat en acides aminés. Elle est stimulée par l'activité physique et régulée par de nombreux facteurs, au premier rang desquels l'IGF-1 et les androgènes (Little et al., 2009).

**Tableau 1 : Facteurs et molécules agissant sur la protéosynthèse et la protéolyse dans les muscles**

| Facteur | Protéosynthèse | | Protéolyse | |
|---|---|---|---|---|
| | Stimulation | Inhibition | Stimulation | Inhibition |
| Exercice | + | | (+) | |
| Dénervation | | - | | |
| Jeûne, anorexie | | - | + | |
| Acides aminés | + | | | - |
| Insuline | + | | | - |
| GH/IGF-1 | + | | | - |
| FGF | + | | + | |
| Vitamine D | + | | | |
| Adrénaline | + | | | - |
| Acétylcholine | | | | - |
| Ocytocine | + | | | |
| Apeline | + | | | |
| Testostérone | + | | | - |
| OEstradiol | + | | | - |
| Triiodothyronine (T3) | + | | | |
| Myostatine | | - | + | |
| TGFβ | | - | + | |
| Follistatine | | | | - |
| Angiotensine II | | - | + | |
| Angiotensine-(1-7) | | | | - |
| Glucocorticoïdes | | - | + | |
| PIF | | - | + | |
| IL-1β | | | + | |
| IL-6 | | (-) | + | |
| TNF-α, IFN-γ | | - | + | |
| Antiinflammatoires | | | | - |

La protéolyse des myofibrilles s'effectue via le protéasome, tandis que les mitochondries sont détruites par autophagie (Zhao et al., 2008). Des mécanismes d'apoptose des cellules satellites sont également décrits (Murphy et al., 2010).

La myostatine, produite de façon autocrine par les muscles eux-mêmes, représente un facteur particulièrement important, car elle agit à la fois en stimulant la protéolyse et en inhibant la protéosynthèse. Elle stimule également la fibrose (Li et al., 2008).

Le vieillissement s'accompagne d'une modification des divers facteurs régulateurs (Walston et al., 2012) : l'activité physique est souvent réduite, la nutrition protéique/vitaminique peut être insuffisante et, suite aux repas, les teneurs en acides aminés circulants, dont l'augmentation est nécessaire pour stimuler la protéosynthèse, présentent une augmentation réduite qui peut être due à une séquestration splanchnique (Boirie et al., 1997). Par ailleurs, le vieillissement s'accompagne de modifications hormonales importantes : on notera en particulier une augmentation de la myostatine (Léger et al., 2008), une réduction des androgènes (Seidman, 2007), de l'hormone de croissance (Macell et al., 2001 ; Sattler, 2013), ainsi qu'une augmentation des marqueurs de l'inflammation (IL-6, TNF-α ... Schaap et al., 2009; Verghese et al., 2011). Ces diverses modifications sont défavorables pour la protéosynthèse, tandis qu'elles favorisent la protéolyse, d'où la réduction progressive de la taille des muscles (sarcopénie). Elles provoquent également une modification dans la distribution des types de fibres musculaires au détriment des fibres rapides, ce qui se traduit par une diminution de la force des muscles (dynapénie). On assiste enfin au développement de tissu conjonctif au sein des muscles (fibrose).

Dans un contexte d'obésité, la situation est aggravée pour plusieurs raisons complémentaires : l'infiltration graisseuse des muscles aggrave le contexte inflammatoire, l'insulino-résistance réduit l'effet de l'IGF-1 sur la protéosynthèse, sans compter que la mobilité est réduite par le surpoids (Stenholm et al., 2009).

La Figure 2, faisant partie de l'état de la technique, illustre l'aggravation de la sarcopénie dans un contexte d'obésité (d'après Quillot et al., 2013).

Dans tous les cas, en absence de traitement, la sarcopénie est un processus qui ne peut que s'aggraver, jusqu'à la perte totale de mobilité. La sarcopénie n'est toutefois pas le seul processus conduisant à une atrophie des muscles squelettiques. Une atrophie se produit également lors d'une immobilisation (ex. suite à une fracture), lors d'un jeûne prolongé (ou d'un régime amaigrissant), ou lors de pathologies graves (ex. cancers, SIDA) qui provoquent une cachexie. On peut également citer les diverses dystrophies musculaires d'origine génétique. Ces différentes situations présentent un certain nombre de caractéristiques communes avec la sarcopénie, mais avec un poids respectif différent des facteurs déclencheurs (Tisdale, 2007 ; Saini et al., 2009).

### Les traitements possibles connus

Différentes méthodes de prévention/traitement de la sarcopénie ont donc été envisagées et testées. Il s'agit en premier lieu de l'exercice physique, dont l'efficacité est établie (Bonnefoy et al, 2000 ; Bonnefoy, 2008 ; Ryan et al., 2013). Ainsi, suite à des exercices réalisés sur une durée de 8 semaines, des augmentations de la force musculaire de 180 % et de la masse musculaire de 11 % ont été observées (Fiatarone et al. 1990). Toutefois, une efficacité optimale nécessiterait plusieurs heures d'exercices physiques par jour, ce qui est difficilement envisageable sur de longues périodes.

Un apport accru des substrats de la synthèse protéique, que ce soit en donnant des protéines à digestion rapide, et selon une chronologie optimisée (Coëffier et al., 2009 ; Aussel et al., 2013), ainsi qu'un complément en certains acides aminés ou leurs métabolites (leucine, HMB [β-hydroxy-β-méthylbutyrate], citrulline, ornithine) peuvent augmenter la protéosynthèse musculaire (Li & Heber, 2011).

Divers traitements pharmaceutiques visent à corriger les modifications du contexte hormonal liées au vieillissement (Crenn, 2013). Ils comprennent :
- des hormones sexuelles comme la testostérone (White et al., 2013) ou ses variantes, les SARM (Selective Androgen Receptor Modulators), ou d'hormones non sexuelles comme l'hormone de croissance (Liu et al., 2003) et l'IGF-1, la ghréline ou la progranuline, voire la vitamine D
- des inhibiteurs de la myostatine (anticorps dirigés contre la molécule ou son récepteur, ou peptide précurseur de la myostatine) (Murphy et al., 2010 ; Han & Mitch, 2011)
- des molécules ciblant le système rénine-angiotensine comme les inhibiteurs d'ACE ou l'angiotensine 1-7 (Dalla Libera et al., 2001 ; Shiuchi et al., 2004 ; Kalupahana & Moustaid-Moussa, 2012 ; Allen et al., 2013)
- des agonistes des récepteurs β-adrénergiques (Ryall et al., 2004, 2007)
- des substances naturelles variées, voire des extraits plus complexes d'origine végétale (ex. isoflavones : Aubertin-Leheudre et al., 2007 ; extrait d'huile d'olive : Pierno et al., 2014 ; resvératrol : Shadfar et al., 20011; Bennett et al., 2013).

La grande diversité de ces traitements témoigne de la difficulté à traiter une pathologie multifactorielle, dont les facteurs déclencheurs ne sont pas totalement identifiés. De plus, plusieurs molécules candidates présentent des effets secondaires (cas des hormones sexuelles, des SARM ou des β-agonistes, par exemple), ou n'ont encore été étudiées que sur des modèles animaux. Tous ces éléments expliquent l'absence de médicaments disponibles sur le marché.

A ce jour, les recherches ciblent plus particulièrement la myostatine, en inhibant son action avec par exemple des anticorps anti-myostatine ou des anticorps anti-récepteurs (Dumonceaux et al., 2010 ; Greenberg, 2012 ; Sakuma & Yamaguchi, 2012 ; Arounleut et al., 2013 ; Buehring & Binkley, 2013 ; Collins-Hooper et al., 2014 ; White & Le Brasseur, 2014).

Les phytoecdysones, et plus particulièrement la 20-hydroxyecdysone (20E), ont fait l'objet de nombreuses études pharmacologiques, qui ont commencé au Japon puis en Ouzbékistan, et se sont ensuite développées dans divers autres pays.

Ces études ont mis en avant les propriétés antidiabétiques et anabolisantes de cette molécule. Ses effets stimulants sur les synthèses protéiques dans les muscles sont observés chez le rat *in vivo* (Syrov, 2000; Tóth et al., 2008; Lawrence, 2012) et sur des myotubes murins C2C12 *in vitro* (Gorelick-Feldman et al., 2008). Il s'agit d'un effet au niveau de la traduction, qui implique la phosphorylation de la protéine ribosomale p70S6K, à l'issue d'une cascade où intervient la protéine kinase Akt/PkB, une voie également utilisée par l'IGF-1 pour stimuler la protéosynthèse.

A l'aide des mêmes cellules C2C12, Zubeldia et al. (2012) ont par ailleurs montré qu'un extrait d'*Ajuga turkestanica* enrichi en phytoecdysones (20-hydroxyecdysone et turkestérone) inhibe la transcription de la myostatine et de la caspase 3 (une protéine impliquée dans les processus d'apoptose).

Par ailleurs, la 20-hydroxyecdysone possède des propriétés antifibrotiques, qui n'ont pas été démontrées sur les muscles, mais au niveau des reins, où les mécanismes de fibrose procèdent de façon très similaire (Hung et al., 2012). Ils s'opposent ainsi aux effets du TGFβ, une protéine proche de la myostatine, et en particulier à la stimulation de Smad 2,3 que provoque cette substance. On peut donc penser que la 20-hydroxyecdysone pourrait avoir des effets similaires sur les muscles (ou le coeur).

La 20-hydroxyecdysone réduit l'accumulation de masse grasse chez des souris nourries avec un régime enrichi en graisses (Kizelsztein et al., 2009; Foucault et al., 2012) ou chez des rattes ovariectomisées, un modèle de ménopause (Seidlova-Wuttke et al., 2010).

Certains des effets décrits ci-dessus dans des modèles animaux ont été retrouvés dans des études cliniques, encore peu nombreuses. Ainsi, la 20-hydroxyecdysone augmente la capacité physique (Azizov et al., 1995; Gadhzieva et al., 1995) et la masse musculaire (Simakin et al., 1988) et provoque une perte de masse grasse abdominale chez des volontaires obèses et en surpoids (Wuttke et al., 2013 ; Foucault et al., 2014 ; demande de brevet PCT WO 2013/068704).

Cependant la 20E aussi bien que ses métabolites sont faiblement biodisponibles chez la souris (Dzhukharova et al., 1987 ; Hikino et al., 1972), chez le rat, (Kapur et al., 2010 et Seidlova-Wuttke et al., 2010) et chez l'homme (Brandt 2003 ; Bolduc, 2006). Leur performance globale n'est entre autres pas entièrement satisfaisante en lien avec des applications d'amélioration de la qualité musculaire.

Plusieurs études ont montré que la turkestérone (11α,20-dihydroxyecdysone), métabolite dérivé de la 20E, montre une activité supérieure à celle de la 20E in vivo (Syrov et al., 2001 : Bathori et al., 2008). Il existe encore aujourd'hui, pour des applications thérapeutiques visant une amélioration de la qualité musculaire tant chez les mammifères obèses que chez les mammifères sarcopéniques, un besoin de nouveaux composés ayant une bonne biodisponibilité, s'exprimant plus particulièrement en termes de coefficient d'exposition plasmatique élevé tout en présentant une activité globalement supérieure à celle de la 20E sur l'amélioration de la qualité musculaire, cette activité globale s'exprimant en termes de performance d'inhibition de l'expression génique de la myostatine combinée à l'augmentation de la synthèse protéique chez le mammifère.

### Exposé de l'invention

Les inventeurs ont maintenant découvert que, de manière tout à fait inattendue, certains composés de la famille des stéroïdes répondant à une formule générale particulière, dont la structure diffère de celle de la 20E et de ses métabolites, présentent un coefficient d'exposition plasmatique supérieur à celui de cette dernière et des effets égaux ou supérieurs à ceux de la 20 hydroxyecdysone (20E) pour l'inhibition de la myostatine et la stimulation de la protéosynthèse par phosphorylation de la protéine S6K1. Ces effets permettent d'améliorer la qualité et/ou la force musculaire chez les mammifères sarcopéniques et obèses sarcopéniques.

Les composés de l'invention n'interagissent pas avec les récepteurs nucléaires stéroïdes de la sphère sexuelle (récepteurs aux androgènes et aux oestrogènes). Ils montrent une bonne stabilité chimique dans le plasma et dans des microsomes. Plusieurs d'entre eux présentent enfin un profil pharmacocinétique nettement amélioré par rapport à la 20-hydroxyecdysone. Ils induisent en outre une meilleure inhibition de l'expression génique de la myostatine et une meilleure amélioration de la synthèse protéique.

L'invention propose ainsi un composé de formule générale **(I)** suivante : 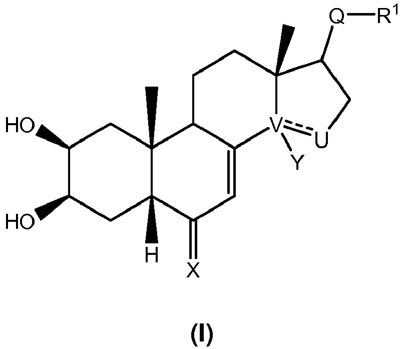 dans laquelle :
**V-U** est une liaison simple carbone-carbone et **Y** est un groupement hydroxyle ou un hydrogène, ou **V-U** est une liaison éthylénique C=C ;
**X** est choisi parmi : un oxygène ; un groupement N-O**R⁵**,
   **R⁵** étant alors choisi parmi : un hydrogène ; un groupement alkyle en C₁-C₆ possédant ou non des insaturations sur la chaîne ; un groupement (C₁-C₆)CO₂**R⁶** avec **R⁶** pouvant être un hydrogène ou un groupement en C₁-C₆; un groupement (C₁-C₆)O**R⁷**, **R⁷** étant un cycle aromatique ou hétéroaromatique mono ou polysubstitué ou non par un groupement alkyl ou alkoxyl, CF₃, Cl ;un groupement (C₁-C₆)N**R⁸R⁹**, **R⁸** et **R⁹** étant des groupements en C₁-C₆, ou des groupements en (C₁-C₆)N(C₁-C₆) ou des groupements en (C₁-C₆)N(C₁-C₆)O**R⁶** avec **R⁶** défini comme ci-dessus, N**R⁸R⁹** peut aussi être un hétérocycle ;
   et dans laquelle :
   - **Q** est un groupement carbonyle ;
      avec **R¹** étant choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A, A** représentant un hétérocycle éventuellement substitué par un groupement de type OH, OMe, (C₁-C₆), N(C₁-C₆), CO₂(C₁-C₆) ; un groupement CH₂Br ;
      **W** étant un hétéroatome choisi parmi N, O et S ;
   ou,
   - **Q** est un groupement CHOH ;
      avec **R¹** étant choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆) ;
      **W** étant un hétéroatome choisi parmi N et S ;
   ou,
   - **Q** est choisi parmi : un groupement C=N**OR⁵**, **R⁵** étant défini comme précédemment ; un groupement CH**NR²R³**,
      avec **R¹** étant un groupement alkyle en (C₁-C₆) ;
      avec **R²** et **R³** identiques ou différents étant choisis chacun parmi : un atome d'hydrogène ; un groupement alkyle en (C₁-C₆) ; un groupement (C₁-C₆)**W**(C₁-C₆) ; un groupement cycloalkyle ; un groupement (C₁-C₆)CHF₂ ; un groupement (C₁-C₆)**A** avec **A** représentant un hétérocycle défini comme précédemment ; un groupement de type **COR⁴,**
      **R⁴** étant choisi parmi : un groupement alkyle ou cycloalkyle en (C₁-C₆) éventuellement insaturé ; un groupement hétérocyclique de type **A** comme défini précédemment, un groupement aromatique ou hétéroaromatique substitué ou non par un groupement de type OH, OMe, (C₁-C₆), N(C₁-C₆), CO₂(C₁-C₆), CF₃, OCF₃, CN, Cl, F ; un groupement (C₁-C₆)**W**(C₁-C₆) ;
      **W** étant un hétéroatome choisi parmi N, O et S ;

le composé étant sous forme d'un énantiomère, d'un diastéréoisomère, d'un hydrate, d'un solvate, d'un tautomère, d'un mélange racémique ou d'un sel pharmaceutiquement acceptable.

Une autre forme particulière de l'invention met en oeuvre le composé de formule générale (I) cités précédemment dans lequel **Q** représente un groupement carbonyle.

Une forme particulière de l'invention met en oeuvre le composé de formule générale (I), dans lequel :
**X** est un oxygène ;
**V-U** est une liaison simple carbone-carbone ;
**Y** est un groupement hydroxyle ;
**Q** est un groupement carbonyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A, A** représentant un hétérocycle éventuellement substitué par un groupement de type OH, OMe, (C₁-C₆), N(C₁-C₆), CO₂(C₁-C₆) ;
**W** étant un hétéroatome choisi parmi N, O et S.

Une autre forme particulière de l'invention met en oeuvre le composé de formule générale (I) dans lequel **Q** représente un groupement CH**NR²R³**, avec **R²** et **R³** étant choisis parmi : un atome d'hydrogène ; un groupement alkyle en (C₁-C₆) ; un groupement (C₁-C₆)**W**(C₁-C₆); un groupement cycloalkyle ; un groupement (C₁-C₆)CHF₂ ; un groupement (C₁-C₆)**A,** avec **A** représentant un hétérocycle défini comme précédemment ; un groupement de type **COR⁴,**
**R⁴** étant choisi parmi : un groupement alkyle ou cycloalkyle en (C₁-C₆) éventuellement insaturé ; un groupement hétérocyclique de type **A** comme défini précédemment, un groupement aromatique ou hétéroaromatique substitué ou non par un groupement de type OH, OMe, (C₁-C₆), N(C₁-C₆), CO₂(C₁-C₆), CF₃, OCF₃, CN, Cl, F; un groupement (C₁-C₆)**W**(C₁-C₆).

Une autre forme particulière de l'invention met en oeuvre le composé de formule générale (I), dans lequel :
**X** est un oxygène ;
**V-U** est une liaison simple carbone-carbone ;
**Y** est un groupement hydroxyle ;
**R¹** est un groupe méthyle ;
**Q** est un groupement CH**NR²R³**,
   avec **R²** et **R³** étant choisis parmi : un atome d'hydrogène ; un groupement alkyle en (C₁-C₆) ; un groupement (C₁-C₆)**W**(C₁-C₆); un groupement cycloalkyle ; un groupement (C₁-C₆)CHF₂ ; un groupement (C₁-C₆)**A,** avec **A** représentant un hétérocycle défini comme précédemment ; un groupement de type **COR⁴,**
   **R⁴** étant choisi parmi : un groupement alkyle ou cycloalkyle en (C₁-C₆) éventuellement insaturé ; un groupement hétérocyclique de type **A** comme défini précédemment, un groupement aromatique ou hétéroaromatique substitué ou non par un groupement de type OH, OMe, (C₁-C₆), N(C₁-C₆), CO₂(C₁-C₆), CF₃, OCF₃, CN, Cl, F ; un groupement (C₁-C₆)**W**(C₁-C₆) ;
**W** étant un hétéroatome choisi parmi N, O et S.

Une autre forme particulière de l'invention met en oeuvre le composé de formule générale (I) dans lequel **Q** représente un groupement C=N**OR⁵**, **R⁵** étant défini comme précédemment.

Une autre forme particulière de l'invention met en oeuvre le composé de formule générale (I), dans lequel :
**X** est un oxygène ;
**V-U** est une liaison simple carbone-carbone ;
**Y** est un groupement hydroxyle ;
**R¹** est un groupe méthyle ;
**Q** est un groupement C=N**OR⁵**, **R⁵** étant défini comme précédemment.

Une autre forme particulière de l'invention met en oeuvre le composé de formule générale (I) dans lequel **V-U** est une liaison éthylénique C=C.

Une autre forme particulière de l'invention met en oeuvre le composé de formule générale (I) dans lequel **X** est un groupement N-O**R⁵**, **R⁵** étant défini comme précédemment.

Une autre forme particulière de l'invention met en oeuvre le composé de formule générale (I) choisi parmi les composés suivants :
- **n°28** : (2S,3R,5R,10R,13R,14S,17S)-17-(N-but-3-énoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one
- **n°32** : (2S,3R,5R,10R,13R,14S,17S)-17-(N-(2-diéthylaminoéthoxy)-C-méthyl-carbonimidoyl)-2,3,14-trihydroxy-10,13-diméthyl-2,3,4,5,9,11,12,15,16,17-décahydro-1H-cyclopenta[a]phénanthrèn-6-one
- **n°41** : 2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide

- **n°42** : (2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one
- **n°43** : (2S,3R,5R,10R,13R,14S,17S)-2,3,14-trihydroxy-10,13-diméthyl-17-[1-(3-pyridylméthylamino)éthyl]-2,3,4,5,9,11,12,15,16,17-décahydro-1H-cyclopenta[a]phénanthrèn-6-one
- **n°46** : (2S,3R,5R,10R,13R,14S,17S)-2,3,14-trihydroxy-10,13-diméthyl-17-[1-(tétrahydrofuran-2-ylméthylamino)éthyl]-2,3,4,5,9,11,12,15,16,17-décahydro-1H-cyclopenta[a]phénanthrèn-6-one
- **n°51** : 2-éthyl-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]butanamide
- **n°62** : 2-méthoxy-N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide
- **n°63** : N-(tetratétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide
- **n°67** : N-(2,2-difluoroéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide
- **n°76** : (2S,3R,5R,10R,13R,14S,17S)-2,3,14-trihydroxy-17-[1-(2-méthoxyéthyl(méthyl)amino)éthyl]-10,13-diméthyl-2,3,4,5,9,11,12,15,16,17-décahydro-1H-cyclopenta[a]phénanthrèn-6-one
- **n°81** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one
- **n°86** :(2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one
- **n°88** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one
- **n°89** : (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one
- **n°91** : (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one
- **n°92** : 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle
- **n°93** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one
- **n°94** : (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one ;

Un autre objet de l'invention concerne l'utilisation d'un composé de formule générale (I) en tant que médicament, notamment, dans un véhicule pharmaceutiquement acceptable.

Un autre objet de l'invention met en oeuvre le composé de formule générale (I) pour son utilisation dans le traitement et/ou la prévention de la sarcopénie et de l'obésité sarcopénique, de ses complications et/ou pathologies associées telles que la perte de force, de masse musculaire, de performances et capacité physiques et de mobilité chez le mammifère. Les performances et capacité physiques peuvent être caractérisées par des tests de marche et d'effort physique.

Un autre objet de l'invention met en oeuvre le composé de formule générale (I) pour son utilisation dans le traitement et/ou la prévention de l'obésité et de ses complications et/ou des pathologies associées, avantageusement du diabète de type 2 ou du syndrome métabolique chez le mammifère.

### Brève description des dessins

- La Figure 1 faisant partie de l'état de la technique, illustre les principales voies de la protéosynthèse et de la protéolyse dans les muscles (composé d'après Zhao et al., 2008 et Little et al., 2009).
- La Figure 2 faisant partie de l'état de la technique, illustre l'aggravation de la sarcopénie dans un contexte d'obésité (d'après Quillot et al., 2013).
- La Figure 3A illustre les effets de la 20E (composé comparatif) et de composés conformes à l'invention n° 51 et 93 sur le poids de souris C57BL/6 soumises à un régime hyperlipidique pendant 6 semaines.
- La Figure 3B illustre les effets de la 20E (composé comparatif) et de composés conformes à l'invention n° 51 et 93 sur la quantité de protéines du muscle *Soleus* de souris C57BL/6 soumises à un régime hyperlipidique pendant 6 semaines.
- La Figure 4 illustre les effets de la 20E (composé comparatif) et de composés conformes à l'invention n° 51 et 93 sur le transcrit de la myostatine du muscle *Soleus* de souris C57BL/6 soumises à un régime hyperlipidique pendant 6 semaines.
- La Figure 5A illustre les effets de la 20E (composé comparatif) et de composés conformes à l'invention n° 51 et 93 sur les transcrits de MyoD de souris C57BL/6 soumises à un régime hyperlipidique pendant 6 semaines.
- La Figure 5B illustre les effets de la 20E (composé comparatif) et de composés conformes à l'invention n° 51 et 93 sur les transcrits de la Myogénine de souris C57BL/6 soumises à un régime hyperlipidique pendant 6 semaines.
- La Figure 6 illustre sous forme de tableau les résultats obtenus pour des composés de la présente invention lors des expériences d'analyse de l'expression génique de la Myostatine et de la synthèse protéique.

### Description détaillée

L'objet de l'invention est de développer des nouveaux composés chimiques répondant notamment aux objectifs fixés ci-avant, en rapport avec des applications thérapeutiques pour le traitement et/ou la prévention de l'obésité et/ou de la sarcopénie chez le mammifère. Ces derniers composés sont nouveaux car inexistants dans les bases de données chimiques. Ils peuvent avantageusement être synthétisés selon des processus industrialisables, c'est-à-dire avec un minimum d'étapes de synthèse et un rendement optimal. Ils ont des effets supérieurs à ceux de la 20E pour l'inhibition de la myostatine, la stimulation de la protéosynthèse par la phosphorylation de la protéine S6K1. Ils montrent une bonne stabilité chimique dans le plasma et dans des microsomes. Ils ont un profil pharmacocinétique amélioré et une posologie définie. Ils stimulent l'anabolisme musculaire dans les cellules C2C12 et montrent un effet anti-hyperglycémique.

Dans le cadre de la présente invention on entend par « groupe aryle », un cycle aromatique ayant 5 à 8 atomes de carbones ou plusieurs cycles aromatiques fusionnés ayant 5 à 14 atomes de carbones. En particulier, les groupes aryles peuvent être des groupes monocycliques ou bicycliques, de préférence phényle ou naphthyle. Avantageusement il s'agit d'un groupe phényle (Ph).

Dans le cadre de la présente invention on entend par « groupe hétéroaryle », tout groupe aromatique hydrocarboné de 3 à 9 atomes contenant un ou plusieurs hétéroatomes, tels que par exemple des atomes de soufre, azote ou oxygène. L'hétéroaryle selon la présente invention peut être constitué par un ou plusieurs cycles fusionnés. Des exemples de groupe hétéroaryle sont les groupes furyle, isoxazyle, pyridyle, thiazolyle, pyrimidyle, benzimidazole, benzoxazole, benzothiazole. Avantageusement le groupe hétéroaryle est choisi parmi les groupes furyle, pyridyle et thiazolyle. De façon avantageuse il s'agit du groupe furyle.

Dans le cadre de la présente invention on entend par « atome d'halogène » tout atome d'halogène, avantageusement choisi parmi Cl, Br, I ou F, en particulier choisi parmi F, Cl ou Br, en particulier F ou Cl.

Dans le cadre de la présente invention on entend par « groupe alkyle en C₁-C₆ », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle. Dans le cadre de la présente invention on entend par « groupe cycloalkyle en C₃-C₆ », tout cycle saturé et hydrocarboné comprenant de 3 à 6 atomes de carbones, en particulier, le groupe cyclopropyle, cyclobutyle, cyclopentyle ou cyclohexyle. Avantageusement il s'agit d'un groupe cyclopropyle ou cyclohexyle.

Dans le cadre de la présente invention on entend par « (groupe alkyle en C₁-C₆)aryle », tout groupe aryle tel que défini ci-dessus, lié par l'intermédiaire d'un groupe alkyle en C₁-C₆ tel que défini ci-dessus. En particulier un exemple de (groupe alkyle en C₁-C₆)aryle est un groupe benzyle ou -(CH₂)₂phényle.

Dans le cadre de la présente invention on entend par «pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Dans le cadre de la présente invention on entend par « sels pharmaceutiquement acceptables d'un composé » des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzène-sulfonique, l'acide benzoïque, l'acide camphre-sulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans le cadre de la présente invention, on entend par « solvate d'un composé », tout composé obtenu par addition d'une molécule de solvant inerte sur le composé selon l'invention, le solvate se formant en raison de leur force d'attraction mutuelle. Les solvates sont par exemple des alcoolates du composé. Un hydrate est un solvate dans lequel le solvant inerte utilisé est l'eau. Il peut être mono-, di- ou tri-hydraté.

Dans le cadre de la présente invention, on entend par « tautomère » tout isomère de constitution des composés selon la présente invention qui sont interconvertibles par la réaction chimique réversible appelée tautomérisation. Dans la plupart des cas, la réaction se produit par migration d'un atome d'hydrogène accompagnée d'un changement de localisation d'une double liaison. Dans une solution d'un composé capable de tautomérisation, un équilibre entre les 2 tautomères se crée. Le rapport entre tautomères est alors fonction du solvant, de la température et du pH. La tautomérie est donc la transformation d'un groupement fonctionnel en un autre, le plus souvent par déplacement concomitant d'un atome d'hydrogène et d'une liaison π (liaison double ou triple). Des tautomères courants sont par exemple les paires aldéhydes / cétones - alcools ou plus précisément énol; amides - acides imidiques; lactames - lactimes ; imines - énamines ; énamines - énamines. En particulier, il peut inclure une tautomérie cycle - chaîne qui a lieu lorsque le mouvement du proton est accompagné par la transformation d'une structure ouverte à un cycle.

### Description des synthèses et schémas généraux

Les composés de formule générale (I) peuvent être préparés par application ou adaptation de toute méthode connue en soi de l'homme du métier et/ou à la portée de ce dernier, notamment celles décrites par Larock (1989), ou par application ou adaptation des procédés décrits dans les procédures qui suivent.

Les différents groupements font référence aux définitions précédemment données.
**Schéma A :** La 20-hydroxyecdysone A₁ peut être réduite en composé A₂ par action du zinc dans l'acide acétique comme décrit dans Zhu et al.(2002). Ce composé A₂ peut subir une coupure oxydante en C20-C22 de la chaîne par réaction du PCC dans la pyridine pour donner le composé A₃. Les alkyloximes de type R⁵ONH₂ réagissent sur le carbonyle en C20 pour donner les imines A₄ correspondantes ainsi que le composé A₅ de double réaction en C20 et C6. 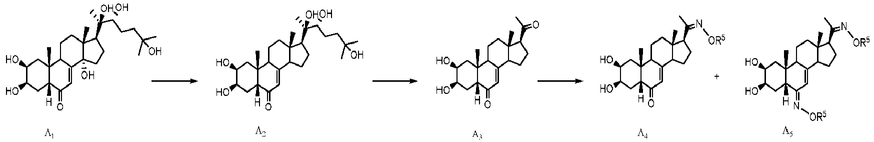
**Schéma B :** Les alkyloximes de type R⁵ONH₂ réagissent sur le carbonyle en C6 du composé A₁ pour donner l'oxime B₁ ainsi que, éventuellement les composés B₂ (conformère Z) et B'₂ (conformère E) d'élimination de l'hydroxyle en C14-C15. Ces 3 composés peuvent subir indépendamment une cassure de chaîne comme décrite en schéma A pour donner les composés B₃ et B₄, avec comme sous-produit le composé (Z)-oxime B'₃. Des alkyloximes de type R⁵ONH₂ réagissent sur le carbonyle en C6 des composés B₃ ou B₄ pour donner les composés B₅ et B₆. 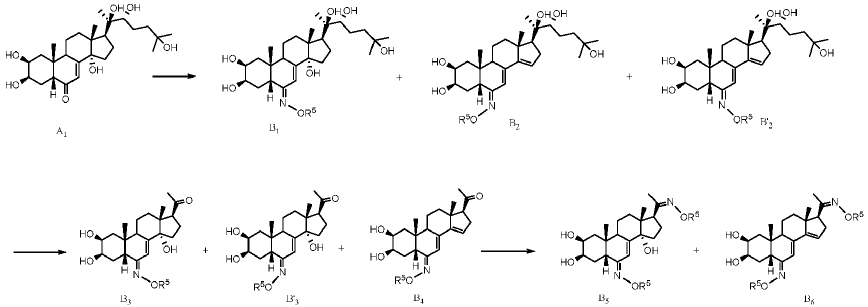
**Schéma C :**
   Le composé A₁ peut subir une coupure oxydante comme décrite sur le schéma A pour donner le composé C₁. Ce composé nommé Poststérone dans la littérature peut subir l'action d'une alkoxime de type R⁵ONH₂ sur le carbonyle en C20, ce qui permet d'obtenir le composé C₂, le composé C₃ de double réaction en C6 et C20 et le composé C₄ d'élimination de l'hydroxyle en C14-C15. 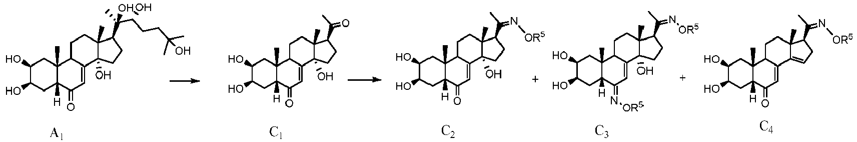
**Schéma D :**
   Le mélange de conformères (E) et (Z) ; B₃ et B'₃ issus du schéma B est mis en réaction avec du chlorure de titane, qui a pour action la déshydratation du composé (Z) ; B'₃ pour obtenir le D₁. Le carbonyle en C17 du composé B₃ isolé à l'étape précédente subit une amination réductrice avec R³NH₂en présence de cyanoborohydrure de sodium pour donner le composé D₂ qui peut être acylé par un chlorure d'acide R⁴COCl, permettant l'obtention du composé D₃. 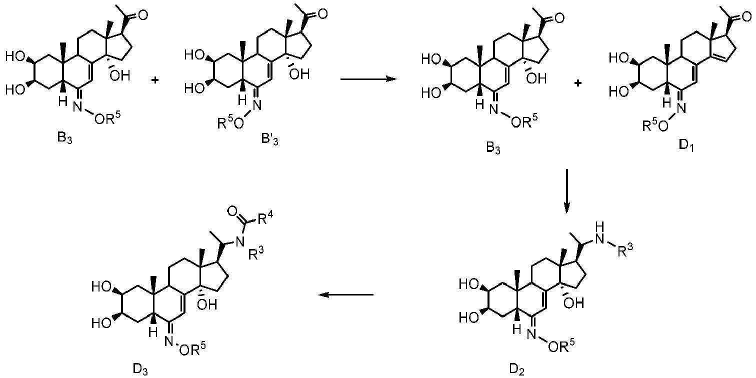
**Schéma E :**
   La Poststérone C₁ subit une amination réductrice puis une acylation du même type que celles décrites au schéma D et permet l'obtention des composés E₁, puis E₂. 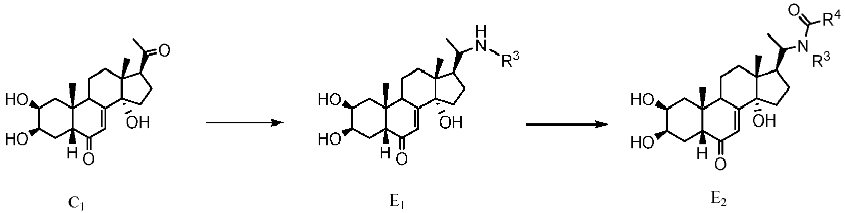
**Schéma F :**
   L'amine secondaire du composé E₁ issu du schéma F est alkylée par un composé bromoalkyl pour donner l'amine tertiaire F₂. 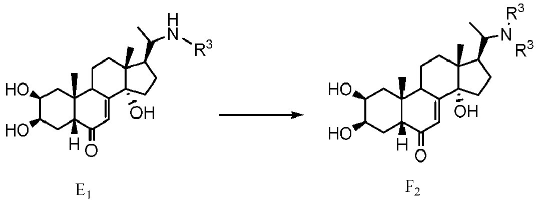
**Schéma G :**
   La Poststérone C₁ peut être bromée en C21 à l'aide de brome pour donner le composé bromé G₁ qui peut être alkylé par un nucléophile WR, W pouvant être une amine ou un thiol et donner le composé G₂. 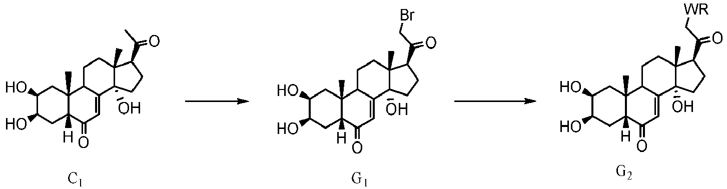
**Schéma H :**
   Le composé bromé G₁ obtenu au schéma G peut réagir avec des composés alcoolates de type OR afin d'obtenir les composés éthérés H₁. 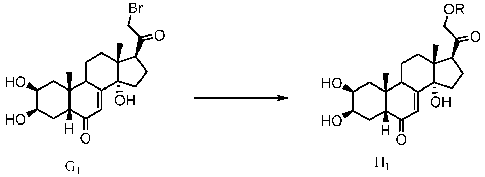
**Schéma I :**
   Les composés G₂ issus du schéma G peuvent subir une réduction du carbonyle en C20 à l'aide du borohydrure de sodium pour donner les alcools I₂. 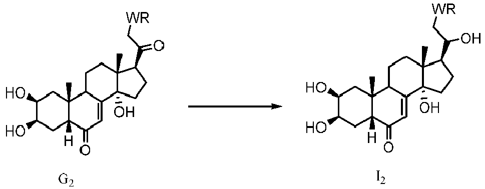
**Schéma J :**
   Les composés G₂ issus du schéma G peuvent subir la réaction en C20 d'une alkoxamine de type R⁵ONH₂ comme décrit au schéma C et permet d'obtenir le composé J₁.

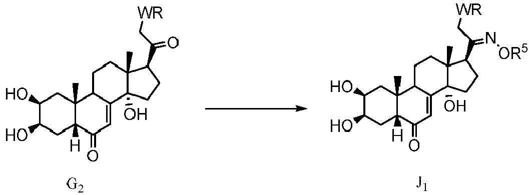

### EXEMPLES :

### Matériels et méthodes

Les spectres de résonance magnétique nucléaire (**RMN**) du proton (**¹H**) sont effectués sur un appareil Bruker Avance DPX300 (300,16 MHz). Les déplacements chimiques (δ) sont mesurés en partie par million (**ppm**). Les spectres sont calibrés sur le déplacement chimique du solvant deutéré utilisé. Les constantes de couplage (**J**) sont exprimées en Hertz (**Hz**) et la multiplicité est représentée de la manière suivante, singulet (s), doublet (d), doublet de doublet (dd), triplet (t), triplet de doublet (td), quadruplet (q), multiplet (m). Les spectres de masse (**SM**) sont réalisés par un spectromètre Agilent Technologies MSD, type G1946A, les échantillons sont ionisés par une source « Atmospheric pressure chemical ionization » (**APCI**).

### Abréviations

- TBAF: tétrabutylammonium fluoride
- THF: tétrahydrofurane
- DMF: diméthylformamide
- CDCl₃: chloroforme deutéré
- CD₃OD: méthanol deutéré
- DMSO-*d₆*: diméthylsulfoxyde deutéré
- PyBop: (Benzotriazol-1-yloxy)tripyrrolidinophosphonium hexafluorophosphate
- Boc: tert-Butyloxycarbonyl
- mmol: millimole(s)
- µM: micromolaire
- mL: millilitre(s)
- g: gramme(s)
- M: mole / litre
- N: normal(e)
- nm: nanomètre(s)
- min: minute(s)
- h: heure(s)
- j: jour(s)
- t.a.: température ambiante
- UV: ultra violet
- ctrl: contrôle
- PM: Poids Moléculaire
- SM: Spectrométrie de Masse

A titre d'exemples illustratifs de l'invention, les composés représentés dans le tableau 2 ont été synthétisés.

**Tableau 2 : Liste des composés dont la synthèse est exemplifiée.**

| **N°** | **Structure chimique** | **Nom chimique** |
|---|---|---|
| **1** | 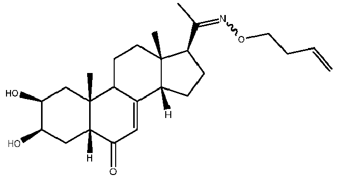 | (2S,3R,5R,10R,13S,14S,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one |
| **2** | 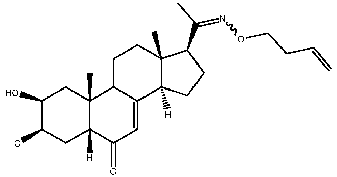 | (2S,3R,5R,10R,13S,14R,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one |
| **3** | 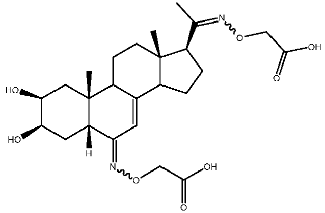 | Acide2-[[(2S,3R,5R,10R,13S,17S)-17-(N-(carboxyméthyloxy)-C-méthyl-carbonimidoyl)-2,3-dihydroxy-10,13-diméthyl-1,2,3,4,5,9,11,12,14,15,16,17-dodécahydrocyclopenta[a]phénanthrèn-6-ylidène]amino]oxyacétique |
| **4** | 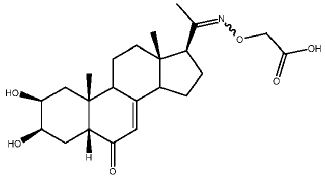 | Acide2-[1-[(2S,3R,5R,10R,13S,17S)-2,3-dihydroxy-10,13-diméthyl-6-oxo-1,2,3,4,5,9,11,12,14,15,16,17-dodécahydrocyclopenta[a]phénanthrèn-17-yl]éthylidèneamino]oxyacétique |
| **5** | 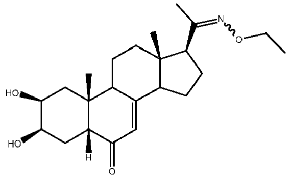 | (2S,3R,5R,10R,13S,17S)-17-(N-éthoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one |
| **6** | 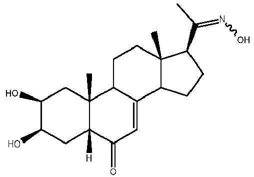 | (2S,3R,5R,10R,13S,17S)-2,3-dihydroxy-17-(N-hydroxy-C-méthyl-carbonimidoyl)-10,13-diméthyl-1,2,3,4,5,9,11,12,14,15,16,17-dodécahydrocyclopenta[a]phénanthrèn-6-one |
| **7** | 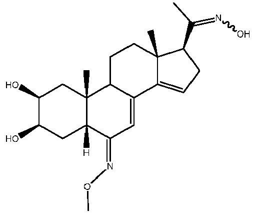 | 1-[(2S,3R,5R,6Z,10R,13R,17S)-2,3-dihydroxy-6-methoxyimino-10,13-dimethyl-1,2,3,4,5,9,11,12,16,17-decahydrocyclopenta[a]phenanthren-17-yl]ethanone oxime |
| **19** | 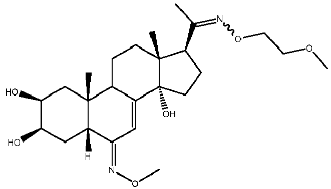 | (2S,3R,5R,6E,10R,13R,14S,17S)-17-(N-(2-méthoxyéthoxy)-C-méthyl-carbonimidoyl)-6-méthoxyimino-10,13-diméthyl-2,3,4,5,9,11,12,15,16,17-décahydro-1H-cyclopenta[a]phénanthrène-2,3,14-triol |
| **21** | 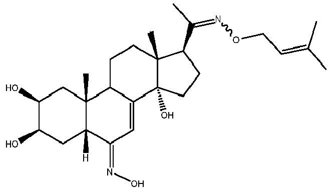 | (2S,3R,5R,10R,13R,14S,17S)-2,3,14-trihydroxy-10,13-diméthyl-17-(C-méthyl-N-(3-méthylbut-2-énoxy)carbonimidoyl)-2,3,4,5,9,11,12,15,16,17-décahydro-1H-cyclopenta[a]phénanthrèn-6-one oxime |
| **23** | 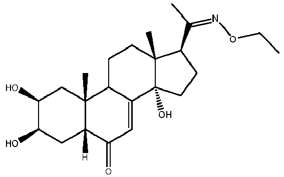 | (2S,3R,5R,10R,13R,14S,17S)-17-(N-éthoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one |
| **24** | 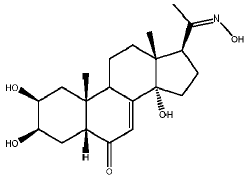 | (2S,3R,5R,10R,13R,14S,17S)-2,3,14-trihydroxy-17-(N-hydroxy-C-méthyl-carbonimidoyl)-10,13-diméthyl-2,3,4,5,9,11,12,15,16,17-décahydro-1H-cyclopenta[a]phénanthrèn-6-one |
| **25** | 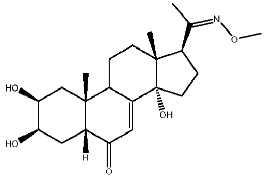 | (2S,3R,5R,10R,13R,14S,17S)-17-(N-méthoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one |
| **26** | 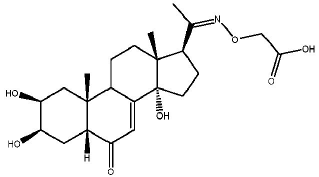 | Acide 2-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthylidèneamino]oxyacétique |
| **27** | 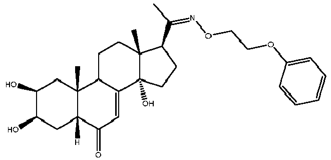 | (2S,3R,5R,10R,13R,14S,17S)-2,3,14-trihydroxy-10,13-diméthyl-17-(C-méthyl-N-(2-phénoxyéthoxy)carbonimidoyl)-2,3,4,5,9,11,12,15,16,17-décahydro-1H-cyclopenta[a]phénanthrèn-6-one |
| **28** | 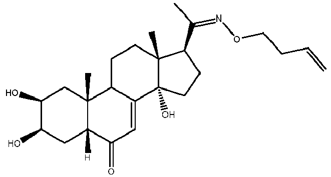 | (2S,3R,5R,10R,13R,14S,17S)-17-(N-but-3-énoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one |
| **29** | 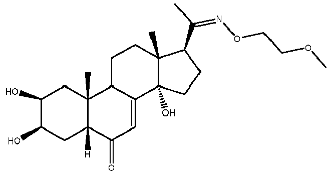 | (2S,3R,5R,10R,13R,14S,17S)-2,3,14-trihydroxy-17-( N-(2-méthoxyéthoxy)-C-méthyl-carbonimidoyl)-10,13-diméthyl-2,3,4,5,9,11,12,15,16,17-décahydro-1H-cyclopenta[a]phénanthrèn-6-one |
| **30** | 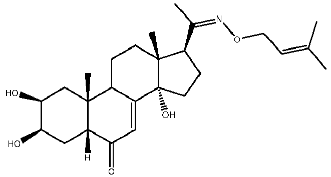 | (2S,3R,5R,10R,13R,14S,17S)-2,3,14-trihydroxy-10,13-diméthyl-17-(C-méthyl-N-(3-méthylbut-2-énoxy)carbonimidoyl)-2,3,4,5,9,11,12,15,16,17-décahydro-1H-cyclopenta[a]phénanthrèn-6-one |
| **31** | 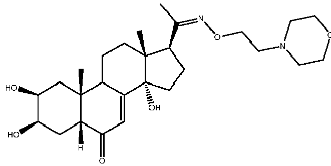 | (2S,3R,5R,10R,13R,14S,17S)-2,3,14-trihydroxy-10,13-diméthyl-17-(C-méthyl-N-(2-morpholinoéthoxy)carbonimidoyl)-2,3,4,5,9,11,12,15,16,17-décahydro-1H-cyclopenta[a]phénanthrèn-6-one |
| **32** | 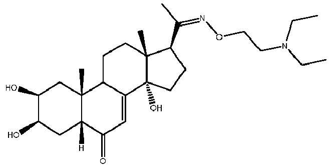 | (2S,3R,5R,10R,13R,14S,17S)-17-(N-(2-diéthylaminoéthoxy)-C-méthyl-carbonimidoyl)-2,3,14-trihydroxy-10,13-diméthyl-2,3,4,5,9,11,12,15,16,17-décahydro-1H-cyclopenta[a]phénanthrèn-6-one |
| **33** | 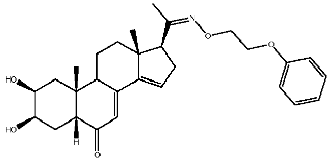 | (2S,3R,5R,10R,13R,17S)-2,3-dihydroxy-10,13-diméthyl-17-(C-méthyl-N-(2-phénoxyéthoxy)carbonimidoyl)-1,2,3,4,5,9,11,12,16,17-décahydrocyclopenta[a]phénanthrèn-6-one |
| **34** | 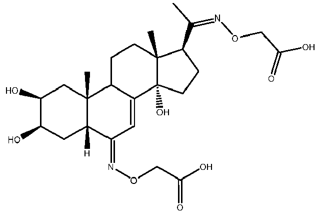 | Acide 2-[[(2S,3R,5R,10R,13R,14S,17S)-17-(N-(carboxyméthyloxy)-C-méthyl-carbonimidoyl)-2,3,14-trihydroxy-10,13-diméthyl-2,3,4,5,9,11,12,15,16,17-décahydro-1H-cyclopenta[a]phénanthrèn-6-ylidène]amino]oxyacétique |
| **35** | 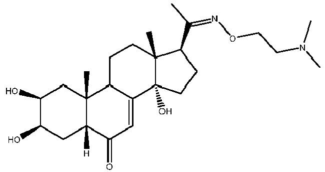 | (2S,3R,5R,10R,13R,14S,17S)-17-(N-(2-diméthylaminoéthoxy)-C-méthyl-carbonimidoyl)-2,3,14-trihydroxy-10,13-diméthyl-2,3,4,5,9,11,12,15,16,17-décahydro-1H-cyclopenta[a]phénanthrèn-6-one |
| **36** | 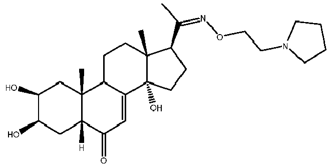 | (2S,3R,5R,10R,13R,14S,17S)-2,3,14-trihydroxy-10,13-diméthyl-17-(C-méthyl-N-(2-pyrrolidin-1-yléthoxy)carbonimidoyl)-2,3,4,5,9,11,12,15,16,17-décahydro-1H-cyclopenta[a]phénanthrèn-6-one |
| **37** | 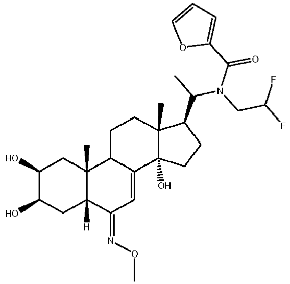 | N-(2,2-difluoroéthyl)-N-[1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthyl]furan-2-carboxamide |
| **38** | 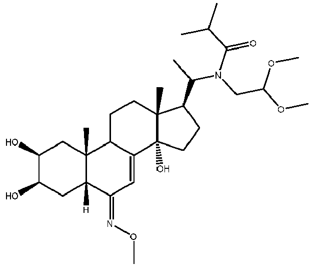 | N-(2,2-diméthoxyéthyl)-2-méthyl-N-[1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthyl]propanamide |
| **39** | 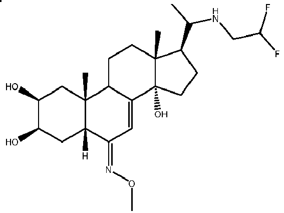 | (2S,3R,5R,6E,10R,13R,14S,17S)-17-[1-(2,2-difluoroéthylamino)éthyl]-6-méthoxyimino-10,13-diméthyl-2,3,4,5,9,11,12,15,16,17-décahydro-1H-cyclopenta[a]phénanthrène-2,3,14-triol |
| **40** | 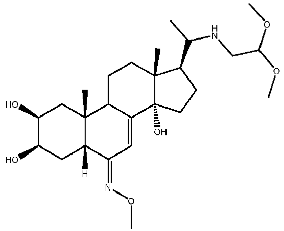 | (2S,3R,5R,6E,10R,13R,14S,17S)-17-[1-(2,2-diméthoxyéthylamino)éthyl]-6-méthoxyimino-10,13-diméthyl-2,3,4,5,9,11,12,15,16,17-décahydro-1H-cyclopenta[a]phénanthrène-2,3,14-triol |
| **41** | 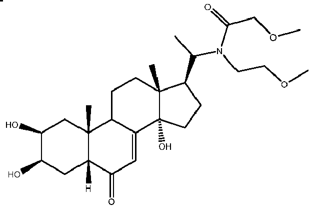 | 2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide |
| **42** | 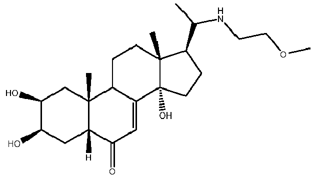 | (2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one |
| **43** | 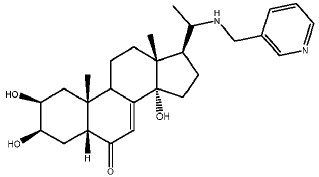 | (2S,3R,5R,10R,13R,14S,17S)-2,3,14-trihydroxy-10,13-diméthyl-17-[1-(3-pyridylméthylamino)éthyl]-2,3,4,5,9,11,12,15,16,17-décahydro-1H-cyclopenta[a]phénanthrèn-6-one |
| **44** | 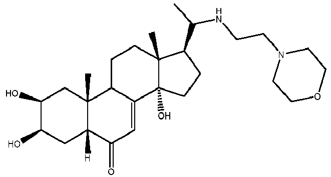 | (2S,3R,5R,10R,13R,14S,17S)-2,3,14-trihydroxy-10,13-diméthyl-17-[1-(2-morpholinoéthylamino)éthyl]-2,3,4,5,9,11,12,15,16,17-décahydro-1H-cyclopenta[a]phénanthrèn-6-one |
| **45** | 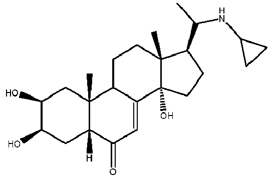 | (2S,3R,5R,10R,13R,14S,17S)-17-[1-(cyclopropylamino)éthyl]-2,3,14-trihydroxy-10,13-diméthyl-2,3,4,5,9,11,12,15,16,17-décahydro-1H-cyclopenta[a]phénanthrèn-6-one |
| **46** | 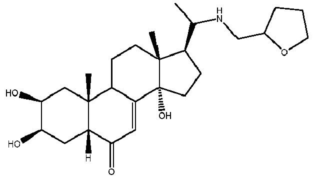 | (2S,3R,5R,10R,13R,14S,17S)-2,3,14-trihydroxy-10,13-diméthyl-17-[1-(tétrahydrofuran-2-ylméthylamino)éthyl]-2,3,4,5,9,11,12,15,16,17-décahydro-1H-cyclopenta[a]phénanthrèn-6-one |
| **47** | 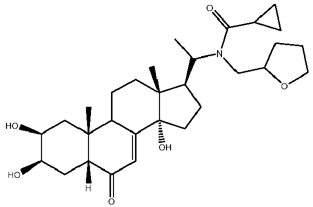 | N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]cyclopropanecarboxamide |
| **48** | 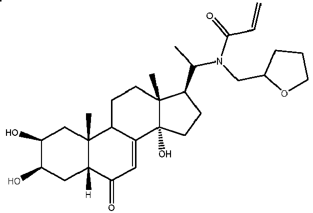 | N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]prop-2-ènamide |
| **49** | 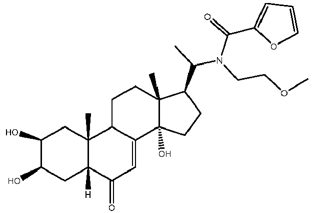 | N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide |
| **50** | 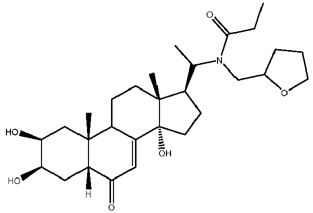 | N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]propanamide |
| **51** | 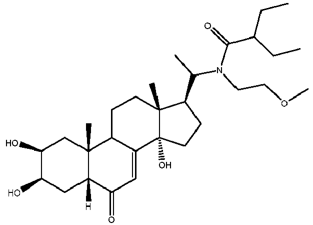 | 2-éthyl-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]butanamide |
| **52** | 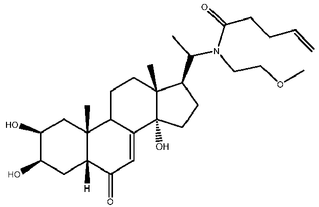 | N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]pent-4-ènamide |
| **53** | 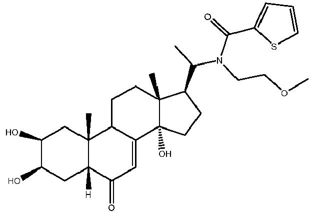 | N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]thiophène-2-carboxamide |
| **54** | 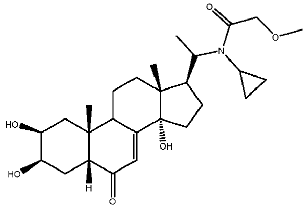 | N-cyclopropyl-2-méthoxy-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acetacétamide |
| **55** | 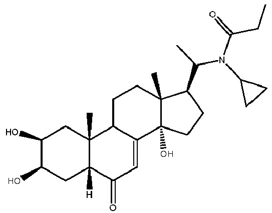 | N-cyclopropyl-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]propanamide |
| **56** | 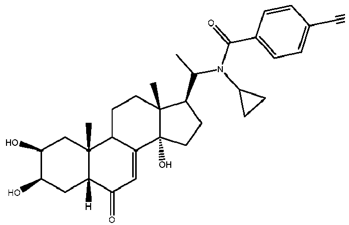 | 4-cyano-N-cyclopropyl-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]benzamide |
| **57** | 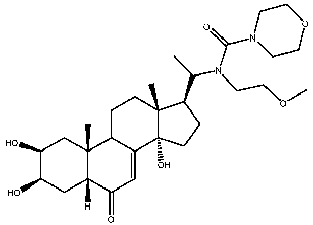 | N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]morpholine-4-carboxamide |
| **58** | 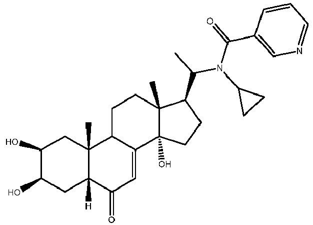 | N-cyclopropyl-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]pyridine-3-carboxamide |
| **59** | 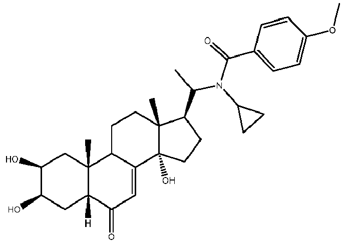 | N-cyclopropyl-4-méthoxy-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]benzamide |
| **60** | 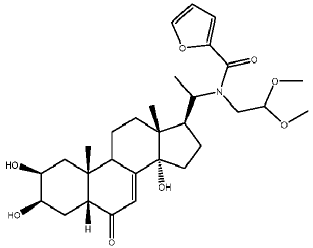 | N-(2,2-diméthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide |
| **61** | 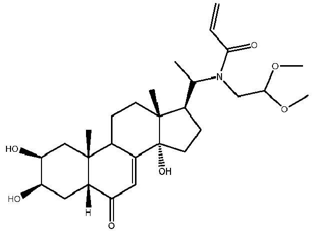 | N-(2,2-diméthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]prop-2-ènamide |
| **62** | 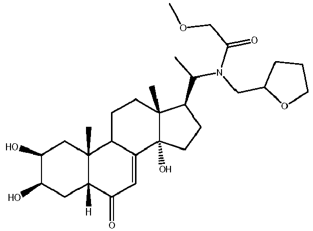 | 2-méthoxy-N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide |
| **63** | 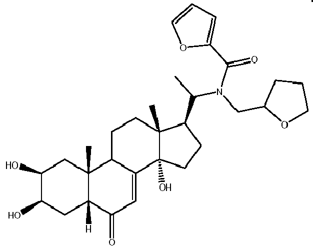 | N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide |
| **64** | 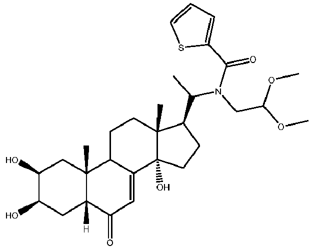 | N-(2,2-diméthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]thiophène-2-carboxamide |
| **65** | 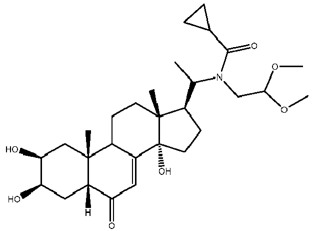 | N-(2,2-diméthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]cyclopropanecarboxamide |
| **66** | 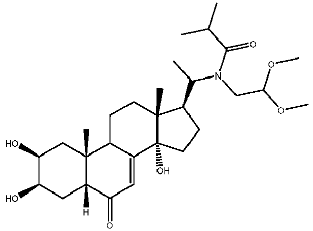 | N-(2,2-diméthoxyéthyl)-2-méthyl-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]propanamide |
| **67** | 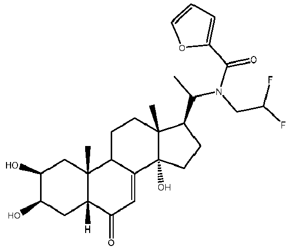 | N-(2,2-difluoroéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide |
| **68** | 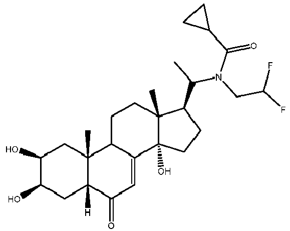 | N-(2,2-difluoroéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]cyclopropanecarboxamide |
| **69** | 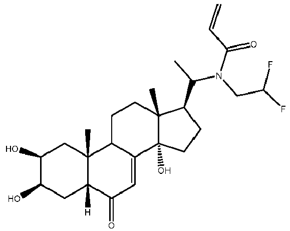 | N-(2,2-difluoroéthyl)-N-[1-[(2S,3R,5R,10R,13R,14S,17S)-2,3,14-trihydroxy-10,13-diméthyl-6-oxo-2,3,4,5,9,11,12, 15,16,17-décahydro-1H-cyclopenta[a]phénanthrèn-17-yl]éthyl]prop-2-ènamide |
| **70** | 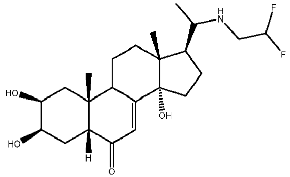 | (2S,3R,5R,10R,13R,14S,17S)-17-[1-(2,2-difluoroéthylamino)éthyl]-2,3,14-trihydroxy-10,13-diméthyl-2,3,4,5,9,11,12,15,16,17-décahydro-1H-cyclopenta[a]phénanthrèn-6-one |
| **71** | 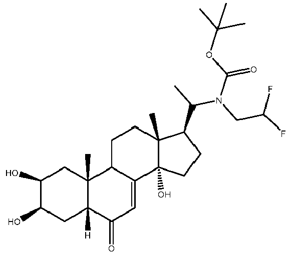 | N-(2,2-difluoroéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]carbamate de tert-butyle |
| **72** | 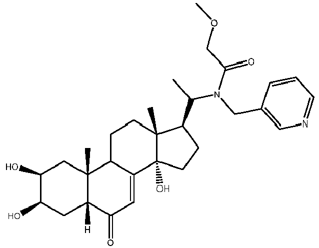 | 2-méthoxy-N-(3-pyridylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide |
| **73** | 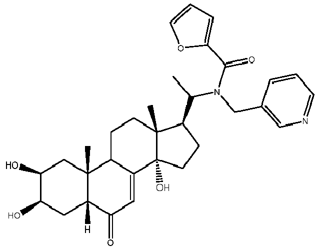 | N-(3-pyridylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide |
| **74** | 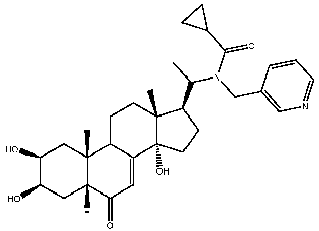 | N-(3-pyridylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]cyclopropanecarboxamide |
| **75** | 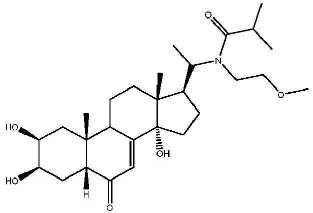 | N-(2-méthoxyéthyl)-2-méthyl-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]propanamide |
| **76** | 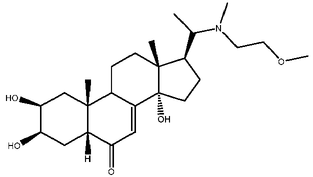 | (2S,3R,5R,10R,13R,14S,17S)-2,3,14-trihydroxy-17-[1-(2-méthoxyéthyl(méthyl)amino)éthyl]-10,13-diméthyl-2,3,4,5,9,11,12,15,16,17-décahydro-1H-cyclopenta[a]phénanthrèn-6-one |
| **77** | 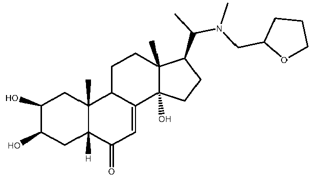 | (2S,3R,5R,10R,13R,14S,17S)-2,3,14-trihydroxy-10,13-diméthyl-17-[1-(méthyl(tétrahydrofuran-2-ylméthyl)amino)éthyl]-2,3,4,5,9,11,12,15,16,17-décahydro-1H-cyclopenta[a]phénanthrèn-6-one |
| **78** | 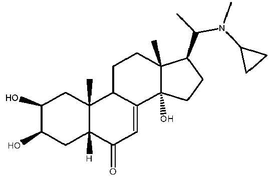 | (2S,3R,5R,10R,13R,14S,17S)-17-[1-(cyclopropyl(méthyl)amino)éthyl]-2,3,14-trihydroxy-10,13-diméthyl-2,3,4,5,9,11,12,15,16,17-décahydro-1H-cyclopenta[a]phénanthrèn-6-one |
| **79** | 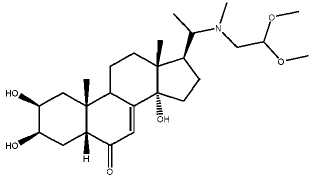 | (2S,3R,5R,10R,13R,14S,17S)-17-[1-(2,2-diméthoxyéthyl(méthyl)amino)éthyl]-2,3,14-trihydroxy-10,13-diméthyl-2,3,4,5,9,11,12,15,16,17-décahydro-1H-cyclopenta[a]phénanthrèn-6-one |
| **80** | 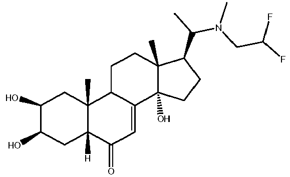 | (2S,3R,5R,10R,13S,17S)-2,3-dihydroxy-10,13-diméthyl-17-[(E)-3-(1-méthylpyrrol-2-yl)prop-2-enoyl]-1,2,3,4,5,9,11,12,14,15,16,17-dodécahydrocyclopenta[a]phénanthrèn-6-one |
| **81** | 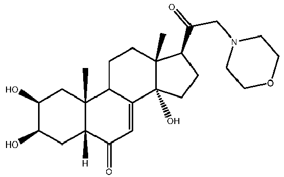 | (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one |
| **82** | 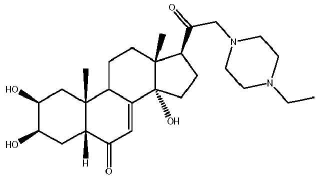 | (2S,3R,5R,10R,13R,14S,17S)-17-[2-(4-éthylpipérazin-1-yl)acétyl]-2,3,14-trihydroxy-10,13-diméthyl-2,3,4,5,9,11,12,15,16,17-décahydro-1H-cyclopenta[a]phénanthrèn-6-one |
| **83** | 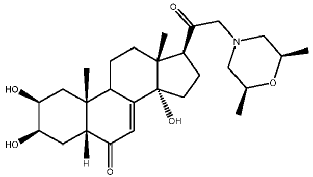 | (2S,3R,5R,10R,13R,14S,17S)-17-[2-[(2S,6R)-2,6-diméthylmorpholin-4-yl]acétyl]-2,3,14-trihydroxy-10,13-diméthyl-2,3,4,5,9,11,12,15,16,17-décahydro-1H-cyclopenta[a]phénanthrèn-6-one |
| **84** | 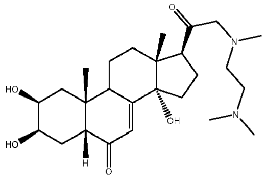 | (2S,3R,5R,10R,13R,14S,17S)-17-[2-(2-diméthylaminoéthyl(méthyl)amino)acétyl]-2,3,14-trihydroxy-10,13-diméthyl-2,3,4,5,9,11,12,15,16,17-décahydro-1H-cyclopenta[a]phénanthrèn-6-one |
| **85** | 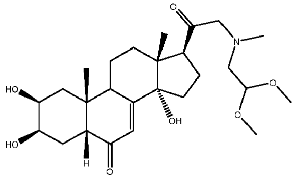 | (2S,3R,5R,10R,13R,14S,17S)-17-[2-(2,2-diméthoxyéthyl(méthyl)amino)acétyl]-2,3,14-trihydroxy-10,13-diméthyl-2,3,4,5,9,11,12,15,16,17-décahydro-1H-cyclopenta[a]phénanthrèn-6-one |
| **86** | 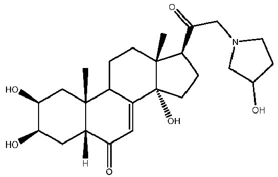 | (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one |
| **87** | 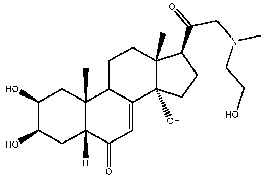 | (2S,3R,5R,10R,13R,14S,17S)-2,3,14-trihydroxy-17-[2-(2-hydroxyéthyl(méthyl)amino)acétyl]-10,13-diméthyl-2,3,4,5,9,11,12,15,16,17-décahydro-1H-cyclopenta[a]phénanthrèn-6-one |
| **88** | 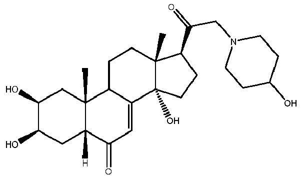 | (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one |
| **89** | 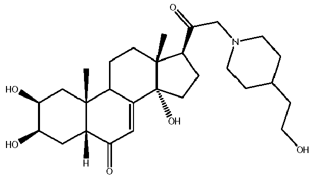 | (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one |
| **90** | 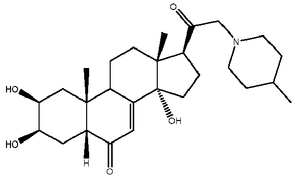 | (2S,3R,5R,10R,13R,14S,17S)-2,3,14-trihydroxy-10,13-diméthyl-17-[2-(4-méthyl-1-pipéridyl)acétyl]-2,3,4,5,9,11,12,15,16,17-décahydro-1H-cyclopenta[a]phénanthrèn-6-one |
| **91** | 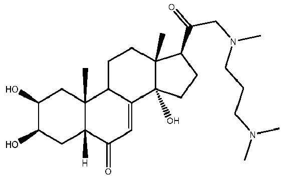 | (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one |
| **92** | 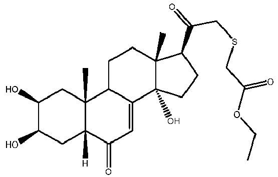 | 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle |
| **93** | 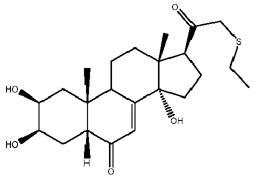 | (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one |
| **94** | 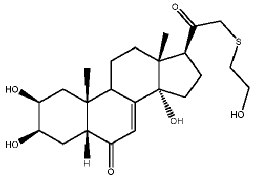 | (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one |
| **95** | 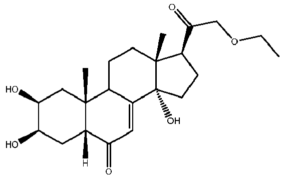 | (2S,3R,5R,10R,13R,14S,17S)-17-(2-éthoxyacétyl)-2,3,14-trihydroxy-10,13-diméthyl-2,3,4,5,9,11,12,15,16,17-décahydro-1H-cyclopenta[a]phénanthrèn-6-one |
| **96** | 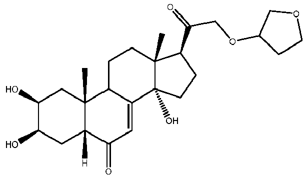 | (2S,3R,5R,10R,13R,14S,17S)-2,3,14-trihydroxy-10,13-diméthyl-17-(2-tétrahydrofuran-3-yloxyacétyl)-2,3,4,5,9,11,12,15,16,17-décahydro-1H-cyclopenta[a]phénanthrèn-6-one |
| **97** | 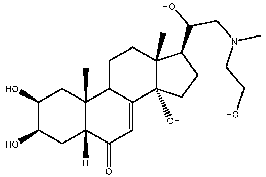 | (2S,3R,5R,10R,13R,14S,17S)-2,3,14-trihydroxy-17-[1-hydroxy-2-(2-hydroxyéthyl(méthyl)amino)éthyl]-10,13-diméthyl-2,3,4,5,9,11,12,15,16,17-décahydro-1H-cyclopenta[a]phénanthrèn-6-one |
| **98** | 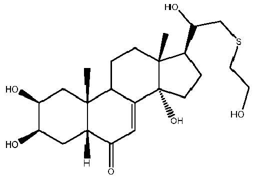 | (2S,3R,5R,10R,13R,14S,17S)-2,3,14-trihydroxy-17-[1-hydroxy-2-(2-hydroxyéthylsulfanyl)éthyl]-10,13-diméthyl-2,3,4,5,9,11,12,15,16,17-décahydro-1H-cyclopenta[a]phénanthrèn-6-one |
| **99** | 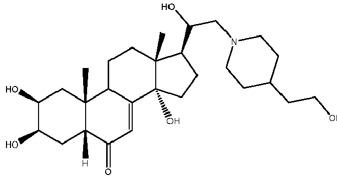 | (2S,3R,5R,10R,13R,14S,17S)-2,3,14-trihydroxy-17-[1-hydroxy-2-[4-(2-hydroxyéthyl)-1-pipéridyl]éthyl]-10,13-diméthyl-2,3,4,5,9,11,12,15,16,17-décahydro-1H-cyclopenta[a]phénanthrèn-6-one |
| **100** | 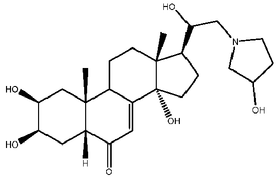 | (2S,3R,5R,10R,13R,14S,17S)-2,3,14-trihydroxy-17-[1-hydroxy-2-(3-hydroxypyrrolidin-1-yl)éthyl]-10,13-diméthyl-2,3,4,5,9,11,12,15,16,17-décahydro-1H-cyclopenta[a]phénanthrèn-6-one |
| **101** | 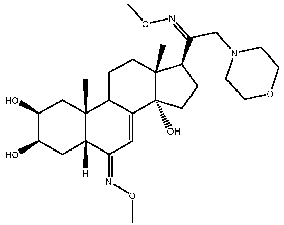 | (2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one |
| **102** | 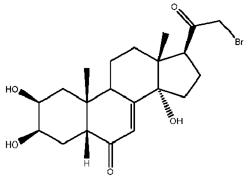 | (2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one |

### Exemple 1 : Schéma A ;

### Préparation des composés n°1 et n°2 :

### (2S,3R,5R,10R,13S,14S,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one et (2S,3R,5R,10R,13S,14R,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one

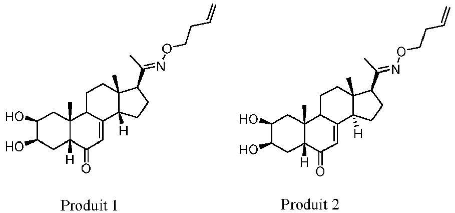

### Etape 1 : Préparation du (2S,3R,5R,10R,13S,17S)-2,3-dihydroxy-10,13-diméthyl-17-[(1R,2R)-1,2,5-trihydroxy-1,5-diméthyl-hexyl]-1,2,3,4,5,9,11,12,14,15,16,17-dodécahydrocyclopenta[a]phénanthrèn-6-one

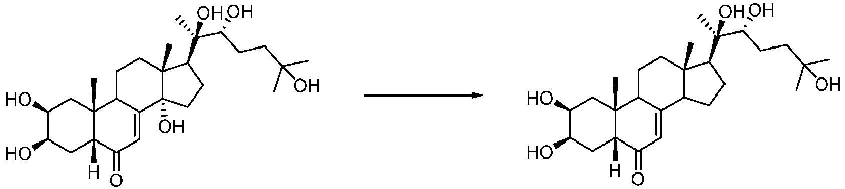

20 g (41.6 mmol) de 20-hydroxyecdysone (commercialement disponible) sont solubilisés dans 280 mL d'acide acétique et la solution est chauffée à 67 °C. 27.2 g (416 mmol) de poudre de zinc sont ajoutés par portions et le milieu réactionnel est chauffé à 67 °C durant 18h. Puis, la solution est filtrée à 20 °C à travers un gâteau de Célite qui est lavé avec 50 mL de méthanol. Le filtrat est évaporé pour donner 33.7 g d'huile brune qui est purifiée par chromatographie flash sur cartouche de gel de silice (Dichlorométhane/Méthanol, 90/10) pour donner 9.52 g de poudre jaune (Rdt: 49 %) de (2S,3R,5R,10R,13S,17S)-2,3-dihydroxy-10,13-diméthyl-17-[(1R,2R)-1,2,5-trihydroxy-1,5-diméthyl-hexyl]-1,2,3,4,5,9,11,12,14,15,16,17-dodécahydrocyclopenta[a]phénanthrèn-6-one.
LC-MS: m/z = 465.3 (MH⁺) pureté UV à 254 nm = 99 %.
RMN ¹H (300 MHz, DMSO-*d₆*)δ 5.72-5.43 (m, 1H(C7)), 4.42-4.32 (m, 2H), 4.13 (s, 1H), 3.76-2.62 (m, 2H), 3.2-3.1 (m, 2H), 2.21-2.14 (m, 2H), 1.90-1.02 (m, 28H), 1.03-0.77 (m, 6H).

### Etape 2 : Préparation du (2S,3R,5R,10R,13S,17S)-17-acétyl-2,3-dihydroxy-10,13-diméthyl-1,2,3,4,5,9,11,12,14,15,16,17-dodécahydrocyclopenta[a]phénanthrèn-6-one

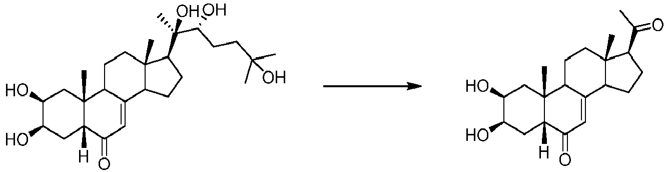

9,52 g (20,28 mmol) de (2S,3R,5R,10R,13S,17S)-2,3-dihydroxy-10,13-diméthyl-17-[(1R,2R)-1,2,5-trihydroxy-1,5-diméthyl-hexyl]-1,2,3,4,5,9,11,12,14,15,16,17-dodécahydrocyclopenta[a]phénanthrèn-6-one sont solubilisés dans 46 mL de pyridine et 276 mL de dichlorométhane. 6,69 g (30,4 mmol) de Pyridinium Chlorochromate sont ajoutés par portions en 10 min et le milieu réactionnel est agité à 20 °C pendant 2h30. La pyridine et le dichlorométhane sont ensuite évaporés sous vide et le résidu est purifié par chromatographie flash sur cartouche de gel de silice (Dichlorométhane/Méthanol, 95/5) pour donner 4 g de poudre beige (Rdt : 56%) de (2S,3R,5R,10R,13S,17S)-17-acétyl-2,3-dihydroxy-10,13-diméthyl-1,2,3,4,5,9,11,12,14,15,16,17-dodécahydrocyclopenta[a]phénanthrèn-6-one. LC-MS: m/z = 347.2 (MH⁺) pureté UV à 254 nm = 99 %.
RMN ¹H (300 MHz, DMSO-*d₆*) δ 5.68-5.46 (m, 1H(C7)), 4.41-4.37 (m, 2H), 3.76-3.55 (m, 2H), 2.83-2.54 (m, 2H), 2.33-1.95 (m, 6H), 1.90-1.30 (m, 10H), 1.28-1.18 (m, 1H), 0.88-0.42 (m, 6H).

### Etape 3 : Préparation des épimères (2S,3R,5R,10R,13S,14S,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one et (2S,3R,5R,10R,13S,14R,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one

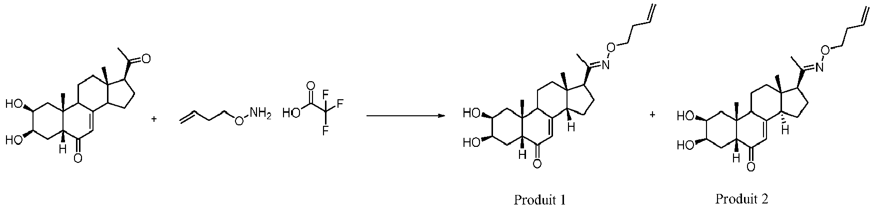

328 mg (0,947 mmol) de (2S,3R,5R,10R,13S,17S)-17-acétyl-2,3-dihydroxy-10,13-diméthyl-1,2,3,4,5,9,11,12,14,15,16,17-dodécahydrocyclopenta[a]phénanthrèn-6-one (14-désoxy-poststérone) préparés à l'étape 2 sont solubilisés dans 1,2 mL d'éthanol et 200 mg (0,994 mmol) de 2,2,2-trifluoroacétate de but-3-énoxyammonium sont ajoutés par portion. Le milieu réactionnel est porté au reflux 20 h. Le solvant est évaporé et le résidu est purifié par chromatographie préparative sur colonne C18 (Acétonitrile/eau, 60/40) pour donner 24 mg de poudre beige (Rdt : 6%) de composé n°**1** (2S,3R,5R,10R,13S,14S,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one et 57 mg de poudre beige (Rdt: 14 %) de composé n°**2** (2S,3R,5R,10R,13S,14R,17S)-17-(N-but-3-énoxy-C-méthyl-carbonimidoyl)-2,3-dihydroxy-10,13-diméthyl-1,2,3,4,5,9,11,12,14,15,16,17-dodécahydrocyclopenta[a]phénanthrèn-6-one.

### Composé n°1 :

LC-MS: m/z = 416.2 (MH⁺) pureté UV à 254 nm = 99%.
RMN ¹H (300 MHz, DMSO-*d₆*) - épimère béta en C14 - δ 5.83-5.72 (m, 1H), 5.70 (s, 1H(C7)), 5.1-5 (m, 2H), 4.40-4.36 (m, 2H), 4 (t, 2H), 3.77-3.71 (m, 2H), 2.80-2.60 (m, 1H), 2.40-1.20 (m, 20H), 0.82-0.74 (m, 6H).

### Composé n°2 :

LC-MS: m/z = 416.2 (MH⁺) pureté UV à 254 nm = 99%.
RMN ¹H (300 MHz, DMSO-D6) - épimère alpha en C14 - δ 5.87-5.72 (m, 1H), 5.48 (s, 1H(C7)), 5.1-4.9 (m, 2H), 4.40-4.36 (m, 2H), 4 (t, 2H), 3.77-3.71 (m, 2H), 2.80-2.60 (m, 1H), 2.44-1.23 (m, 20H), 0.83 (s, 3H), 0.47 (s, 3H).

Les composés n°**3** à **6** ont été préparés selon le même schéma, sous forme de mélange d'épimères C14 alpha et C14 béta.

| **N°** | **PM g/mol** | **Apparence** | **Pureté¹ (%)** | **SM m/z MH⁺** | **RMN ¹H (300 MHz,** DMSO-*d₆***) δ** |
|---|---|---|---|---|---|
| **3** | 492.6 | poudre beige | 92 | 493.2 | 8.56 (s, 1H), 7.78 (s, 1H), 6.34-5.47 (m, 1H(C7)), 4.48 (m, 4H), 3.72 (m, 3H), 2.28-1.30 (m, 20H), 0.85-0.44 (m, 6H) |
| **4** | 419.5 | poudre beige | 92 | 420.2 | 5.69-5.46 (m, 1H(C7)), 4.43 (s, 2H), 3.74-3.64 (m, 3H), 2.80-2.60 (m, 1H), 2.31-1.16 (m, 19H), 0.85-0.44 (m, 6H) |
| **5** | 389.5 | poudre blanche | 94 | 390.2 | 5.70-5.47(m,1H(C7)),4.39-4.36(m,2H),4.02-3.95(q,2H),3.76-3.60(m,2H),2.80-2.60(m,1H),2.41-1.2(m,18H),1.15(t,3H),0.83-0.47(m,6H) |
| **6** | 361.5 | poudre blanche | 93 | 362.2 | 10.44-10.39 (m, 1H), 5.67-5.47 (m, 1H(C7)), 4.37-4.35 (m, 2H), 3.75-3.60 (m, 2H), 2.80-2.60 (m, 1H), 2.45-1.1 (m, 18H), 0.83-0.45 (m, 6H) |

| | | | | | |
|---|---|---|---|---|---|
| ¹Pureté LCMS, UV à 254 nm | | | | | |

### Exemple 2 : Schéma B ;

### Préparation du composé n°7 : [1-[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]éthanone oxime] et du composé n°19 : [(2S,3R,5R,6E,10R,13R,14S,17S)-17-(N-(2-méthoxyéthoxy)-C-méthyl-carbonimidoyl)-6-méthoxyimino-10,13-diméthyl-2,3,4,5,9,11,12,15,16,17-décahydro-1H-cyclopenta[a]phénanthrène-2,3,14-triol]

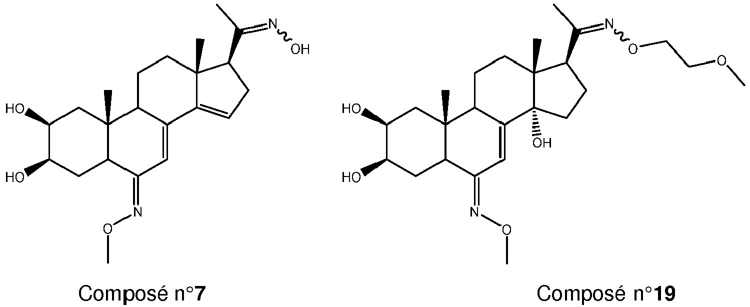

### Préparation du composé n°7

### Etape 1 : Préparation du composé (a) [(2S,3R,5R,6E,10R,13R,14S,17S)-6-méthoxyimino-10,13-diméthyl-17-[(1R,2R)-1,2,5-trihydroxy-1,5-diméthyl-hexyl]-2,3,4,5,9,11,12,15,16,17-décahydro-1H-cyclopenta[a]phénanthrène-2,3,14-triol] et du composé (b) [(2R,3R)-2-[(2S,3R,5R,6Z, 10R,13R,17S)-2,3-dihydroxy-6-méthoxyimino-10,13-diméthyl-1,2,3,4,5,9,11,12,16,17-décahydrocyclopenta[a]phénanthrèn-17-yl]-6-méthyl-heptane-2,3,6-triol]

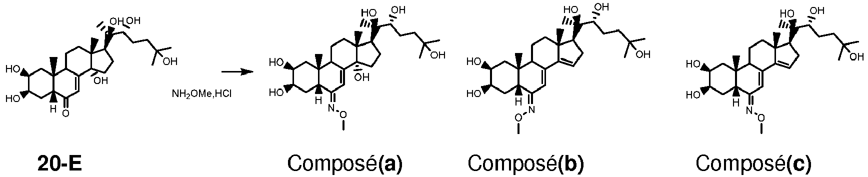

Suivant le même mode opératoire que celui décrit à l'étape 3 du schéma A, 788 mg de poudre beige (Rdt : 37%) du composé **(a)** [(2S,3R,5R,6E,10R,13R,14S,17S)-6-méthoxyimino-10,13-diméthyl-17-[(1R,2R)-1,2,5-trihydroxy-1,5-diméthyl-hexyl]-2,3,4,5,9,11,12,15,16,17-décahydro-1H-cyclopenta[a]phénanthrène-2,3,14-triol] ont été préparés à partir de 20-hydroxyecdysone et de O-méthylhydroxylamine hydrochloride. 667 mg (Rdt : 32 %) du composé d'élimination **(b)** [(2R,3R)-2-[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]-6-méthyl-heptane-2,3,6-triol] ont également pu être isolés ainsi que 34 mg (Rdt : 2 %) du composé d'élimination **(c)** [(2R,3R)-2-[(2S,3R,5R,6E,10R,13R,17S)-2,3-dihydroxy-6-méthoxyimino-10,13-diméthyl-1,2,3,4,5,9,11,12,16,17-décahydrocyclopenta[a]phénanthrèn-17-yl]-6-méthyl-heptane-2,3,6-triol] ont également pu être également isolés.

### Composé (a) :

LC-MS: m/z = 510.2 (MH⁺) pureté UV à 254 nm = 99%.
RMN ¹H (300 MHz, DMSO-*d₆*)δ 6.25 (s, 1H(C7)), 4.45-4.35 (m, 3H), 4.31-4.29 (m, 1H), 4.14 (s, 1H), 3.74-3.69 (m, 4H), 3.6-3.5 (m, 1H), 3.17-3.08 (m, 1H), 2.87-2.75 (m, 1H), 2.26-2.20 (m, 2H), 2.05-1.1 (m, 15H), 1.1-0.98 (m, 11H), 0.73 (s, 6H).

### Composé (b) :

LC-MS: m/z = 492.2 (MH⁺) pureté UV à 254 nm = 99 %.
RMN ¹H (300 MHz, DMSO-*d₆*)δ 6.04 (s, 1H), 5.77 (s, 1H), 4.45-4.30 (m, 2H), 4.25 (s, 1H), 4.11 (s, 1H), 3.75-3.65 (m, 5H), 3.63-3.55 (m, 1H), 3.20-3.08 (m, 2H), 2.17-1.90 (m, 3H), 1.70-1.20 (m, 11H), 1.15-0.93 (m, 14H), 0.74 (s, 3H).

### Composé (c) :

LC-MS: m/z = 492.2 (MH⁺) pureté UV à 254 nm = 99 %.
RMN ¹H (300 MHz, DMSO-*d₆*)δ 6.55 (s, 1H), 5.81 (s, 1H), 4.44-4.26 (m, 3H), 4.09 (s, 1H), 3.79-3.67 (m, 5H), 3.62-3.54 (m, 1H), 3.16-3.08 (m, 1H), 2.30-1.90 (m, 4H), 1.70-1.20 (m, 11H), 1.15-0.92 (m, 14H), 0.73 (s, 3H).
En partant du composé (b) isolé :

### Etape 2a : Préparation du composé (d): [1-[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]éthanone]

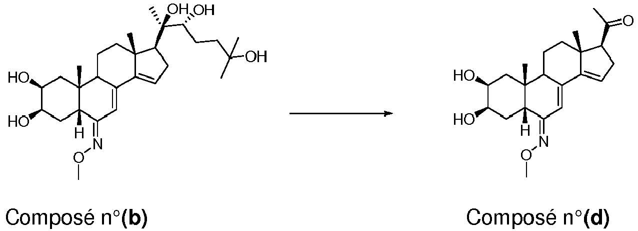

Suivant le même mode opératoire que celui décrit à l'étape 2 du schéma A, 267 mg de poudre beige (Rdt : 55 %) de composé **(d)** [1-[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]éthanone] ont été préparés à partir du composé **(b).**

### Composé (d) :

LC-MS: m/z = 374.2 (MH⁺) pureté UV à 254 nm = 99 %.
RMN ¹H (300 MHz, DMSO-*d₆*)δ 6.09 (s, 1H), 5.81-5.75 (m, 1H), 4.39-4.37 (m, 1H), 4.30-4.26 (m, 1H), 3.76 (s, 3H), 3.72-3.68 (m, 1H), 3.65-3.55 (m, 1H), 3.2-3 (m, 2H), 2.75-2.60 (m, 1H), 2.29-2.10 (m, 5H), 1.74-1.23 (m, 8H), 0.74-0.70 (m, 6H).

### Etape 3a : Préparation du composé n°7 : [1-[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]éthanone oxime]

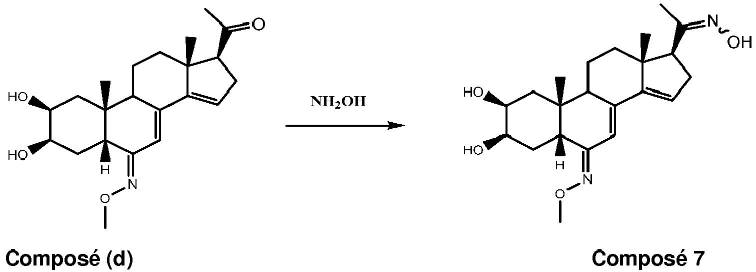

Suivant le même mode opératoire que celui décrit à l'étape 3 du schéma A, 81 mg de poudre blanche (Rdt : 71 %) de -[(2S,3R,5R,6Z,10R,13R,17S)-2,3-dihydroxy-6-méthoxyimino-10,13-diméthyl-1,2,3,4,5,9,11,12,16,17-décahydrocyclopenta[a]phénanthrèn-17-yl]éthanone oxime ont été préparés à partir du composé **(d).**

### Composé n°7 :

LC-MS: m/z = 389.2 (MH⁺) pureté UV à 254 nm = 99 %.
RMN ¹H (300 MHz, DMSO-*d₆*)δ 10.53 (s, 1H), 6.09 (s, 1H), 5.04 (s, 1H), 4.37 (d, 1H), 4.30-4.26 (m, 1H), 3.77-3.67 (m, 4H), 3.65-3.55 (m, 1H), 3.15-3.03 (m, 1H), 2.80-2.65 (m, 2H), 2.25-2.12 (m, 1H), 2.05-1.99 (m, 1H), 1.79 (s, 3H), 1.74-1.20 (m, 8H), 0.76-0.66 (m, 6H).

### Préparation du composé n°19 en partant du composé (a) (2S,3R,5R,6E,10R,13R,14S,17S)-6-méthoxyimino-10,13-diméthyl-17-[(1R,2R)-1,2,5-trihydroxy-1,5-diméthyl-hexyl]-2,3,4,5,9,11,12,15,16,17-décahydro-1H-cyclopenta[a]phénanthrène-2,3,14-triol isolé :

### Etape 2b : Préparation des composés (e): [1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthanone] et (f) : [1-[(2S,3R,5R,6Z,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthanone].

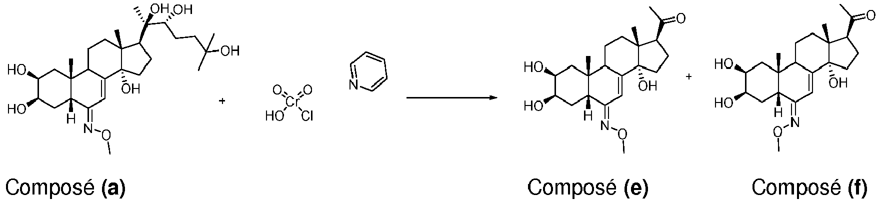

Suivant le même mode opératoire que celui décrit à l'étape 2 du schéma A, 891 mg de poudre beige (Rdt : 36 %) de composé **(e)** [1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthanone] ont été isolés ainsi que 23 mg (Rendement : 0.9 %) du composé **(f)** : [1-[(2S,3R,5R,6Z,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthanone] à partir de 3.5 g de composé **(a)** [2S,3R,5R,6E,10R,13R,14S,17S)-6-méthoxyimino-10,13-diméthyl-17-[(1R,2R)-1,2,5-trihydroxy-1,5-diméthyl-hexyl]-2,3,4,5,9,11,12,15,16,17-décahydro-1H-cyclopenta[a]phénanthrène-2,3,14-triol] isolé.

### Composé (e) :

LC-MS: m/z = 392.2 (MH⁺) pureté UV à 254 nm = 99%.
RMN ¹H (300 MHz, DMSO-*d₆*)δ 6.28 (s, 1H(C7)), 4.74 (s, 1H), 4.42-4.36 (m, 1H), 4.32-4.28 (m, 1H), 3.76-3.70 (m, 4H), 3.68-3.52 (m, 1H), 3.20-3.12 (m, 1H), 2.90-2.76 (m, 1H), 2.30-2.00 (m, 5H), 1.90-1.50 (m, 8H), 1.49-1.24 (m, 3H), 0.72 (s, 3H), 0.45 (s, 3H).

### Composé (f) :

LC-MS: m/z = 392.2 (MH⁺) pureté UV à 254 nm = 99 %.
RMN ¹H (300 MHz, DMSO-*d₆*)δ 5.71 (s, 1H(C7)), 4.45 (s, 1H), 4.45-4.41 (m, 1H), 4.26-4.23 (m, 1H), 3.76-3.70 (m, 4H), 3.65-3.55 (m, 1H), 3.18-3.09 (m, 1H), 2.90-2.80 (m, 1H), 2.22-2.00 (m, 5H), 1.88-1.22 (m, 11H), 0.73 (s, 3H), 0.47 (s, 3H).

### Etape 3b : Préparation du composé n°19 : [(2S,3R,5R,6E,10R,13R,14S,17S)-17-(N-(2-méthoxyéthoxy)-C-méthyl-carbonimidoyl)-6-méthoxyimino-10,13-diméthyl-2,3,4,5,9,11,12,15,16,17-décahydro-1H-cyclopenta[a]phénanthrène-2,3,14-triol]

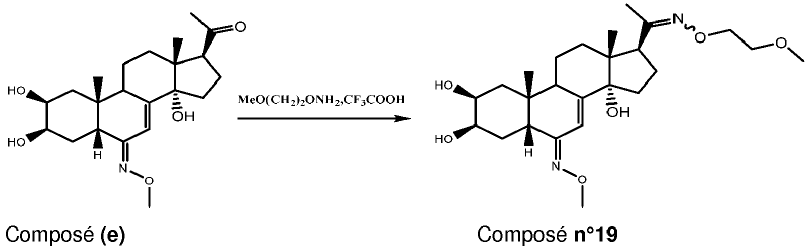

Suivant le même mode opératoire que celui décrit à l'étape 3 du schéma A, 46 mg de poudre blanche (Rendement : 48 %) de composé n°**19** [(2S,3R,5R,6E,10R,13R,14S,17S)-17-(N-(2-méthoxyéthoxy)-C-méthyl-carbonimidoyl)-6-méthoxyimino-10,13-diméthyl-2,3,4,5,9,11,12,15,16,17-décahydro-1H-cyclopenta[a] phénanthrène-2,3,14-triol] ont été préparés à partir de 233 mg du composé **(e).**

### Composé n°19 :

LC-MS: m/z = 465.2 (MH⁺) pureté UV à 254 nm = 99 %.
RMN ¹H (300 MHz, DMSO-*d₆*)δ 6.28 (s, 1H(C7)), 4.66 (s, 1H), 4.44-4.38 (m, 1H), 4.34-4.28 (m, 1H), 4.10-4.01 (m, 2H), 3.75-3.70 (m, 4H), 3.65-3.45 (m, 3H), 3.24 (s, 3H), 2.98-2.76 (m, 2H), 2.30-1.90 (m, 4H), 1.80-1.24 (m, 12H), 0.73 (s, 3H), 0.49 (s, 3H).

Le composé n°**21** a été préparé selon le même schéma.

| **N°** | **PM g/mol** | **Apparence** | **Pureté¹ (%)** | **SMm/z MH⁺** | **RMN ¹H (300 MHz, DMSO-*d₆*) δ** |
|---|---|---|---|---|---|
| **21** | 460.6 | poudre blanche | 99 | 461.3 | 10.35 (s, 1H), 6.38 (s, 1H), 5.39-5.27 (m, 1H), 4.58 (s, 1H), 4.49-4.27 (m, 1H), 4.25-4.22 (m, 1H), 3.74 (s, 1H), 3.65-3.55 (m, 1H), 2.96-2.77 (m, 2H), 2.3-1.22 (m, 24H), 0.72 (s, 3H), 0.49 (s, 3H). |

| | | | | | |
|---|---|---|---|---|---|
| ¹Pureté LCMS, UV à 254 nm | | | | | |

### Exemple 3 : Schéma C ;

### Préparation du composé n°23 :

### (2S,3R,5R,10R,13R,14S,17S)-17-(N-éthoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one

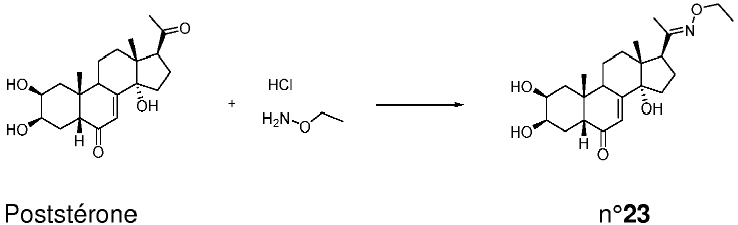

Suivant le même mode opératoire que celui décrit à l'étape 3 du schéma A, 64 mg de poudre blanche (Rendement : 22 %) de (2S,3R,5R,10R,13R,14S,17S)-17-(N-éthoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one ont été préparés à partir de la poststerone (obtenue par coupure oxydante de la chaine de la 20-hydroxyecdysone suivant le même mode opératoire que celui décrit à l'étape 2 du schéma B).

### Composé n°23 :

LC-MS: m/z = 406.2 (MH⁺) pureté UV à 254 nm = 93 %.
RMN ¹H (300 MHz, CD₃OD) δ 5.82 (s, 1H(C7)), 4.04 (q, 2H), 3.97-3.92 (m, 1H), 3.89-3.80 (m, 1H), 3.22-3.10 (m, 1H), 3.04 (t, 1H), 2.43-1.55 (m, 15H), 1.45-1.37 (m, 1H), 1.21 (t, 3H), 0.96 (s, 3H), 0.64 (s, 3H).

Les composés n°**24** à **36** ont été préparés selon le même schéma

| **N°** | **PM g/mol** | **Apparence** | **Pureté¹ (%)** | **SM m/z MH⁺** | **RMN ¹H (300 MHz, DMSO-*d₆*) δ** |
|---|---|---|---|---|---|
| **24** | 377.5 | poudre blanche | 99 | 378.1 | (in CD₃OD) δ 5.81 (s, 1H(C7)), 3.95 (s, 1H), 3.85-3.80 (m, 1H), 3.25-3.17 (m, 1H), 3.05 (t, 1H), 2.45-1.55 (m, 15H), 1.47-1.39 (m, 1H), 0.96 (s, 3H), 0.63 (s, 3H). |
| **25** | 391.5 | poudre blanche | 95 | 392.2 | (in CD₃OD) δ 5.81 (s, 1H(C7)), 3.95 (s, 1H), 3.88-3.75 (m, 4H), 3.23-3.12 (m, 1H), 3.03 (t, 1H), 2.41-1.55 (m, 15H), 1.45-1.39 (m, 1H), 0.96 (s, 3H), 0.63 (s, 3H). |
| **26** | 435.5 | poudre marron | 99 | 436.2 | (DMSO+D₂O) δ 5.66 (s, 1H(C7)), 4.41 (s, 1H), 3.77 (s, 1H), 3.68-3.58 (m, 1H), 3.03-2.96 (m, 1H), 2.92-2.84 (m, 1H), 2.23-1.21 (m, 16H), 0.8 (s, 3H), 0.47 (s, 3H). |
| **27** | 497.6 | poudre blanche | 92 | 498.2 | 7.30-7.24 (m, 2H), 6.94-6.92 (m, 3H), 5.65 (s, 1H(C7)), 4.95 (s, 1H), 4.50-4.40 (m, 2H), 4.29-4.25 (m, 2H), 4.18-4.12 (m, 2H), 3.78-3.74 (m, 1H), 3.63-3.57 (m, 1H), 3.15-2.80 (m, 2H), 2.25-1.1 (m, 16H), 0.82 (s, 3H), 0.49 (s, 3H). RMN ¹³C (75 MHz, DMSO-D6)δ 202.8 (C6), 177.8, 164.1, 158.6, 157.8, 129.6, 120.7, 114.5, 82.5, 51.2, 47.2, 45.7, 30.5, 21.1, 16.2, 6.2. |
| **28** | 431.6 | poudre blanche | 99 | 432.2 | 5.87-5.73 (m, 1H), 5.65 (s, 1H(C7)), 5.1-5 (m, 2H), 4.94 (s, 1H), 4.49 (d, 1H), 4.41-4.39 (m, 1H), 4.05-3.95 (m, 2H), 3.77 (s, 1H), 3.66-3.58 (m, 1H), 3.1-2.98 (m, 1H), 2.94 (t, 1H), 2.4-1.4 (m, 17H), 1.32-1.22 (m, 1H), 0.83 (s, 3H), 0.51 (s, 3H). RMN ¹³C (75 MHz, DMSO-*d₆*)δ 202.8 (C6), 164.2, 156.9, 116.6, 82.5, 71.7, 47.2, 37.7, 33.5, 31.6, 21.1. |
| **29** | 435.6 | poudre blanche | 99 | 436.2 | 5.65 (s, 1H(C7)), 4.94 (s, 1H), 4.50-4.48 (m, 1H), 4.42-4.39 (m, 1H), 4.10-4.02 (m, 2H), 3.8-3.72 (m, 1H), 3.7-3.55 (m, 1H), 3.52-3.48 (m, 2H), 3.24 (s, 3H), 3.08-3 (m, 1H), 2.94 (t, 1H), 2.28-2.03 (m, 3H), 1.92-1.42 (m, 12H), 1.34-1.20 (m, 1H), 0.83 (s, 3H), 0.51 (s, 3H). |
| **30** | 445.6 | poudre beige | 90 | 446.2 | 5.65 (s, 1H(C7)), 5.37-5.29 (m, 1H), 4.92 (s, 1H), 4.51-4.35 (m, 3H), 3.81-3.74 (m, 1H), 3.68-3.56 (m, 1H), 3.08-2.85 (m, 2H), 2.25-1.18 (m, 23H), 0.83 (s, 3H), 0.50 (s, 3H). |
| **31** | 490.6 | poudre blanche | 90 | 491.3 | 5.66 (s, 1H(C7)), 4.97 (s, 1H), 4.52-4.3 (m, 4H), 3.95-3.55 (m, 7H), 3.1-2.87 (m, 4H), 2.25-1.18 (m, 19H), 0.83 (s, 3H), 0.52 (s, 3H). |
| **32** | 476.7 | poudre blanche | 99 | 477.3 | 5.65 (s, 1H(C7)), 4.98 (s, 1H), 4.52 (d, 1H), 4.44-4.40 (m, 1H), 4.39-4 (m, 2H), 3.77 (s, 1H), 3.70-3.54 (m, 1H), 3.1-2.85 (m, 6H), 2.28-2.02 (m, 4H), 1.9-0.92 (m, 21H), 0.83 (s, 3H), 0.52 (s, 3H). |
| **33** | 479.6 | poudre orange | 99 | 480.2 | 7.34-7.24 (m, 2H), 6.97-6.89 (m, 3H), 6.14-6.08 (m, 1H), 5.57 (s, 1H), 4.48-4.15 (m, 6H), 3.66 (s, 1H), 3.47-3.37 (m, 1H), 2.35-1.95 (m, 4H), 1.92-1.65 (m, 8H), 1.62-1.43 (m, 4H), 0.97-0.94 (m, 6H). |
| **34** | 508.6 | poudre blanche | 96 | 509.2 | 8.57 (s, 1H), 6.35 (s, 1H(C7)), 4.72 (s, 1H), 4.47 (s, 4H), 3.74 (m, 3H), 2.28-1.25 (m, 19H), 0.75-0.65 (m, 3H), 0.5 (s, 3H). |
| **35** | 448.6 | poudre blanche | 97 | 449.2 | 5.66 (s, 1H(C7)), 4.97 (s, 1H), 4.55-4.25 (m, 4H), 3.77 (s, 1H), 3.68-3.56 (m, 1H), 3.12-2.9 (m, 3H), 2.77 (s, 6H), 2.28-2.05 (m, 4H), 1.9-1.4 (m, 12H), 1.34-1.21 (m, 1H), 0.84 (s, 3H), 0.54 (s, 3H). |
| **36** | 474.6 | poudre blanche | 96 | 475.2 | (in D₂O) δ 5.95 (s, 1H(C7)), 4.38-4.31 (m, 2H), 4.06-3.91 (m, 2H), 3.74-3.60 (m, 2H), 3.55-3.48 (m, 2H), 3.20-3.05 (m, 2H), 3.03-2.93 (m, 1H), 2.35-1.55 (m, 20H), 1.41-1.28 (m, 1H), 0.95 (s, 3H), 0.62 (s, 3H). |

| | | | | | |
|---|---|---|---|---|---|
| ¹Pureté LCMS, UV à 254 nm | | | | | |

### Exemple 4 : Schéma D ;

### Préparation du composé n°37 : N-(2,2-difluoroéthyl)-N-[1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthyl]furan-2-carboxamide

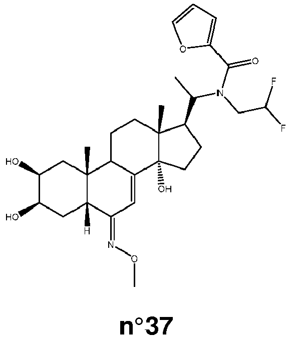

### Etape 1 : Préparation du composé n°39 : [(2S,3R,5R,6E,10R,13R,14S,17S)-17-[1-(2,2-difluoroéthylamino)éthyl]-6-méthoxyimino-10,13-diméthyl-2,3,4,5,9,11,12,15,16,17-décahydro-1H-cyclopenta[a]phénanthrène-2,3,14-triol].

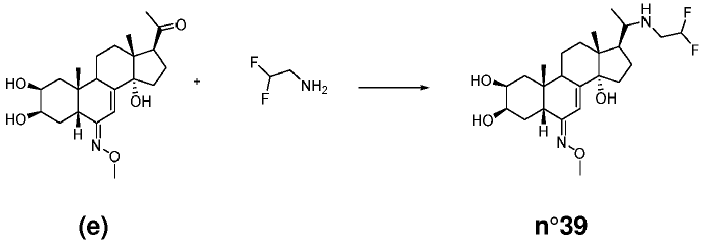

180 mg (0,46 mmol) de composé **(e)** [1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthanone] obtenu à l'étape 2b de la méthode B sont solubilisés dans 5 mL de méthanol et 0,21 mL (2,76 mmol) de 2,2-difluoroéthanamine sont ajoutés au milieu réactionnel. Le pH de la solution est ajusté à 6 à l'aide de la quantité suffisante d'acide acétique concentré. Puis 31,8 mg (0,506 mmol) de cyanoborohydrure de sodium sont ajoutés par portions et la suspension obtenue est chauffée au reflux 20 h. Le solvant est évaporé et le résidu obtenu est repris dans 20 mL d'eau et le pH est ajusté à 8 à l'aide d'une solution saturée de bicarbonate de sodium. Cette phase aqueuse est extraite avec deux fois 15 mL de butanol et la phase butanolique est séchée sur sulfate, filtrée et évaporer pour donner un solide jaune, qui, repris dans 30 mL d'éther isopropylique et filtré donne après séchage 134 mg (Rendement : 62 %) du composé n°**39** (2S,3R,5R,6E,10R,13R,14S,17S)-17-[1-(2,2-difluoroéthylamino)éthyl]-6-méthoxyimino-10,13-diméthyl-2,3,4,5,9,11,12,15,16,17-décahydro-1H-cyclopenta[a]phénanthrène-2,3,14-triol sous forme de poudre jaune.

### Composé n°39 :

LC-MS: m/z = 457.4 (MH⁺) pureté UV à 254 nm = 97 %.
RMN ¹H (300 MHz, DMSO-*d₆*)δ 6.30-6.23 (m, 1H), 5.95-5.70 (m, 1H), 4.43-4.25 (m, 3H), 3.72 (s, 3H), 3.65-3.55 (m, 1H), 3.42-3.32 (m, 1H), 2.88-2.76 (m, 2H), 2.29-2.23 (m, 1H), 1.99-1.15 (m, 16H), 1.05-0.82 (m, 3H), 0.73 (s, 3H), 0.61-0.53 (m, 3H).

### Etape 2 : Préparation du composé n°37 : N-(2,2-difluoroéthyl)-N-[1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthyl]furan-2-carboxamide

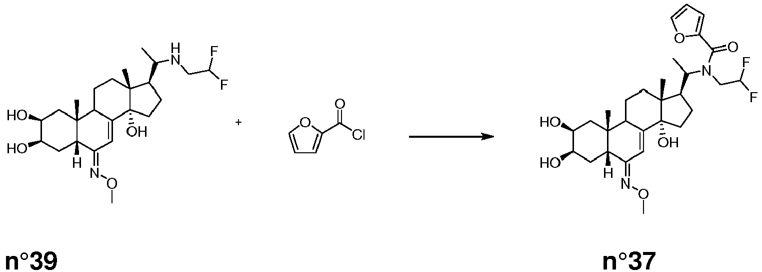

134 mg,(0,285 mmol) de composé n°**39** [(2S,3R,5R,6E,10R,13R,14S,17S)-17-[1-(2,2-difluoroéthylamino)éthyl]-6-méthoxyimino-10,13-diméthyl-2,3,4,5,9,11,12,15,16,17-décahydro-1H-cyclopenta[a]phénanthrène-2,3,14-triol] sont solubilisés dans 2 mL de THF et 52 mg (0,854 mmol) de bicarbonate de soude sont ajoutés au milieu réactionnel sous atmosphère d'Argon. 30 µL (0,299mmol) de chlorure de furoyle sont additionnés et le milieu réactionnel est agité 20 h à 20°C. La solution est ensuite versée sur 5 mL d'eau et extraite deux fois avec 10 mL de butanol. La phase butanolique est évaporée pour donner 118 mg de solide purifiée par chromatographie flash sur cartouche de gel de silice (Dichlorométhane/MeOH, 95/5) pour donner 100 mg de poudre blanche (Rendement : 60 %) du composé n°**37** :N-(2,2-difluoroéthyl)-N-[1-[(2S,3R,5R,6E,10R,13R,14S,17S)-2,3,14-trihydroxy-6-méthoxyimino-10,13-diméthyl-2,3,4,5,9,11,12,15,16,17-décahydro-1H-cyclopenta[a]phénanthrèn-17-yl]éthyl]furane-2-carboxamide.

### Composé n°37 :

LC-MS: m/z = 551.3 (MH⁺) pureté UV à 254 nm = 93 %.
RMN ¹H (300 MHz, DMSO-*d₆*) δ 7.87 (s, 1H), 7.03 (s, 1H), 6.64 (s, 1H), 6.25 (s, 1H), 4.58 (d, 1H), 4.43-4.27 (m, 3H), 3.95-3.83 (m, 1H), 3.75-3.65 (m, 4H), 3.63-3.49 (m, 2H), 2.85-2.68 (m, 1H), 2.31-2.18 (m, 1H), 2.01-1 (m, 17H), 0.73-0.15 (m, 6H).

Les composés n°**38** et **40** ont été préparés selon le même schéma.

| **N°** | **PM g/mol** | **Apparence** | **Pureté¹ (%)** | **SM m/z MH⁺** | **RMN ¹H (300 MHz, DMSO-*d₆*) δ** |
|---|---|---|---|---|---|
| **38** | 550.7 | poudre blanche | 93 | 551.5 | 6.27 (s, 1H(C7)), 4.55-4.22 (m, 4H), 3.78-3.67 (m, 4H), 3.62-3.54 (m, 1H), 3.32-3.22 (m, 6H), 2.95-2.70 (m, 2H), 2.30-2.19 (m, 1H), 2-0.9 (m, 25H), 0.79-0.40 (m, 6H). |
| **40** | 480.6 | poudre beige | 99 | 481.4 | 6.35-6.24 (m, 1H), 4.48-4.25 (m, 4H), 3.71 (s, 3H), 3.65-3.55 (m, 1H), 3.40-3.20 (m, 7H), 2.88-2.76 (m, 1H), 2.75-2.66 (m, 1H), 2.29-2.22 (m, 1H), 2-1.15 (m, 15H), 1.05-0.76 (m, 4H), 0.73 (s, 3H), 0.61-0.54 (m, 3H). |

| | | | | | |
|---|---|---|---|---|---|
| ¹Pureté LCMS, UV à 254 nm | | | | | |

### Exemple 5 : Schéma E ;

### Préparation du composé n°41 : 2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17yl]éthyl]acétamide

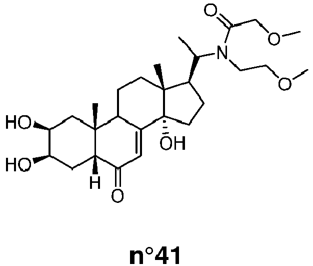

### Etape 1 : Préparation du composé n°42 : (2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one

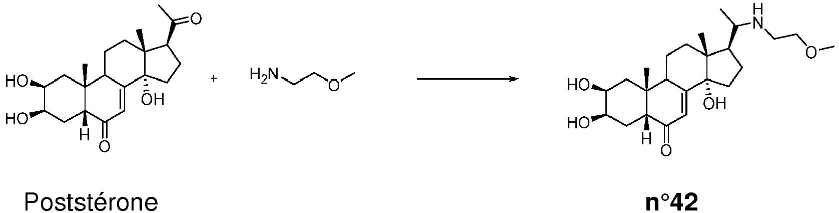

5 g (13,8 mmol) de poststérone (obtenue par coupure oxydante de la chaine de la 20-hydroxyecdysone suivant le même mode opératoire que celui décrit à l'étape 2 du schéma B) sont solubilisés dans 250 mL de méthanol et 7,2 mL (83 mmol) de 2-méthoxyéthylamine sont additionnés goutte à goutte. Le pH de la solution est ensuite amené à pH 6 par addition d'acide acétique concentré et 250 mL de THF sont ajoutés. 0,954 g de cyanoborohydrure de sodium sont ajoutés par portions et le milieu réactionnel est porté à reflux 20 h. Les solvants sont évaporés et le brut obtenu est repris dans 100 mL d'eau et le pH est ajusté à 8 par addition d'une solution saturée de bicarbonate de soude. Le milieu est extrait trois fois avec 80 mL de butanol et la phase butanolique est évaporée pour donner une mousse marron qui, reprise avec 5 mL d'acétate d'éthyle donne après filtration et séchage 3,32 g (Rendement: 57 %) du composé n°**42** : (2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one sous forme de poudre grise.

### Composé n°42 :

LC-MS: m/z = 422.2 (MH⁺) pureté UV à 254 nm = 95 %.
RMN ¹H (300 MHz, DMSO-*d₆*)δ 5.70-5.60 (m, 1H(C7)), 4.80-4.62 (m, 1H), 4.55-4.47 (m, 1H), 4.43-4.35 (m, 1H), 3.78-3.70 (m, 2H), 3.68-3.50 (m, 3H), 3.30-3.18 (m, 5H), 3.10-2.91 (m, 1H), 2.30-0.9 (m, 18H), 0.82 (s, 3H), 0.59 (s, 3H).
RMN ¹³C (75 MHz, DMSO-*d₆*)δ 202.9 (C6), 120.5, 82.9, 66.7, 58.1, 46.2, 37.8, 30.5, 23.9, 6.2.

### Etape 2 : Préparation du composé n°41 : 2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide

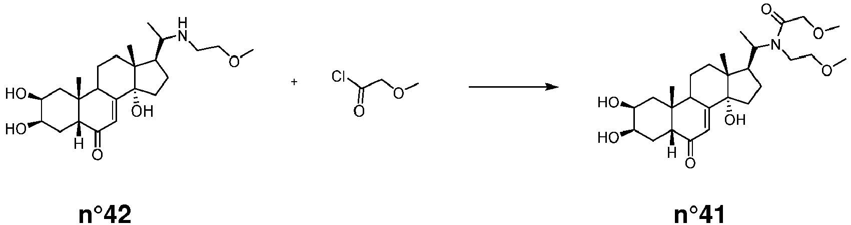

Suivant le même mode opératoire que l'étape 2 de l'exemple 5, 89 mg (Rendement : 58 %) de composé **n°41** [2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide] ont été obtenus sous forme de poudre orange à partir du composé n°**42**.

### Composé n°41 :

LC-MS: m/z = 494.4 (MH⁺) pureté UV à 254 nm = 94 %.
RMN ¹H (300 MHz, DMSO-*d₆*)δ 5.63 (s, 1H(C7)), 4.88-4.7 (m, 1H), 4.5-4.35 (m, 2H), 4.2-3.9 (m, 2H), 3.76 (s, 1H), 3.68-3.52 (m, 1H), 3.5-3.3 (m, 4H), 3.28-3.18 (m, 6H), 3.08-2.9 (m, 1H), 2.3-0.95 (m, 18H), 0.88-0.75 (m, 3H), 0.7-0.42 (m, 3H).

Les composés n°**43** à **75** ont été préparés selon le même schéma :

| **N°** | **PM g/mol** | **Apparence** | **Pureté¹ (%)** | **SM m/z MH⁺** | **RMN ¹H (300 MHz, DMSO-*d₆*) δ** |
|---|---|---|---|---|---|
| **43** | 454.6 | poudre blanche | 91 | 455.2 | 8.54-8.38 (m, 2H), 7.75-7.68 (m, 1H), 7.35-7.28 (m, 1H), 5.60 (s, 1H(C7)), 4.63 (s, 1H), 4.42 (d, 1H), 4.37-4.34 (m, 1H), 3.88-3.48 (m, 5H), 3.10-2.85 (m, 1H), 2.25-1.05 (m, 15H), 1.03 (d, 3H), 0.83 (s, 3H), 0.52 (s, 3H). |
| **44** | 476.7 | poudre blanche | 91 | 477.3 | 5.67-5.60 (m, 1H(C7)), 4.98-4.40 (m, 3H), 3.76 (s, 1H), 3.68-3.45 (m, 5H), 3.10-2.78 (m, 2H), 2.45-0.95 (m, 26H), 0.83 (s, 3H), 0.64-0.58 (m, 3H). |
| **45** | 403.6 | poudre blanche | 85 | 404.2 | 5.73-5.60 (m, 1H(C7)), 4.80-4.62 (m, 1H), 4.56-4.50 (m, 1H), 4.46-4.38 (m, 1H), 3.77 (s, 1H), 3.68-3.53 (m, 2H), 3.53-3.45 (m, 1H), 3.10-2.85 (m, 1H), 2.30-0.98 (m, 22H), 0.84 (s, 3H), 0.65-0.5 (m, 3H). |
| **46** | 447.6 | huile jaune | 92 | 448.2 | 5.69-5.61 (m, 1H(C7)), 4.92-4.75 (m, 1H), 4.48-4.33 (m, 2H), 4.18-3.55 (m, 4H), 3.1-2.7 (m, 3H), 2.3-1 (m, 24H), 0.85 (s, 3H), 0.65-0.55 (m, 3H). |
| | | | | | ¹³C NMR (75 MHz, DMSO-*d₆*)δ 202.8 (C6), 163.9, 120.5, 82.8, 67.5, 66.7, 50.1, 46.2, 37.7, 36.6, 33.3, 28.9, 25.2, 23.9, 20, 15.2. |
| **47** | 515.7 | poudre blanche | 93 | 516.1 | 5.68-5.60 (m, 1H(C7)), 4.87-4.65 (m, 1H), 4.48-4.35 (m, 2H), 4.08-3.88 (m, 1H), 3.81-3.50 (m, 4H), 3.35-3.25 (m, 2H), 3.08-2.9 (m, 1H), 2.3-0.95 (m, 23H), 0.9-0.4 (m, 10H). |
| **48** | 501.7 | poudre blanche | 97 | 502.4 | 6.85-6.65 (m, 1H), 6.20-5.9 (m, 1H), 5.70-5.50 (m, 2H), 4.85-4.70 (m, 1H), 4.48-4.30 (m,2H), 4.2-3.8 (m, 1H), 3.77-3.70 (m, 2H), 3.68-3.40 (m, 3H), 3.10-2.85 (m, 1H), 2.27-1 (m, 23H), 0.85-0.75 (m, 3H), 0.70-0.46 (m, 3H). |
| **49** | 515.6 | poudre blanche | 99 | 516.3 | 7.82 (s, 1H), 6.93 (s, 1H), 6.60 (s, 1H), 5.65-5.55 (m, 1H), 4.84 (s, 1H), 4.48-4.35 (m, 2H), 4.30-4.20 (m, 1H), 3.77-3.33 (m, 3H), 3.32-3.12 (m, 5H), 3.10-2.85 (m, 1H), 2.25-1 (m, 18H), 0.84-0.76 (m, 3H), 0.74-0.17 (m, 3H). |
| **50** | 503.7 | poudre mauve | 99 | 504.3 | 5.62 (s, 1H(C7)), 4.88-4.65 (m, 1H), 4.47-4.33 (m, 2H), 4.08-3.85 (m, 1H), 3.8-3.5 (m, 4H), 3.08-2.9 (m, 2H), 2.3-0.9 (m, 28H), 0.88-0.72 (m, 3H), 0.69-0.4 (m, 3H). |
| **51** | 519.7 | poudre blanche | 94 | 520.4 | 5.63 (s, 1H(C7)), 4.88-4.7 (m, 1H), 4.45-4.35 (m, 2H), 3.75 (s, 1H), 3.68-3.52 (m, 1H), 3.52-3.35 (m, 2H), 3.27-3.21 (m, 5H), 3.08-2.9 (m, 1H), 2.26-0.86 (m, 23H), 0.87-0.72 (m, 9H), 0.70-0.47 (m, 3H). |
| **52** | 503.7 | poudre orange | 99 | 504.4 | 5.91-5.75 (m, 1H), 5.68-5.60 (m, 1H(C7)), 5.07-4.89 (m, 2H), 4.83-4.65 (m, 1H), 4.45-4.28 (m, 2H), 3.76 (s, 1H), 3.68-3.52 (m, 1H), 3.46-3.35 (m, 2H), 3.30-3.15 (m, 5H), 3.08-2.09 (m, 1H), 2.47-0.92 (m, 22H), 0.90-0.77 (m, 3H), 0.7-0.45 (m, 3H). |
| **53** | 531.7 | poudre blanche | 99 | 532.4 | 7.76-7.70 (m, 1H), 7.39-7.32 (m, 1H), 7.12-7.08 (m, 1H), 5.59 (s, 1H(C7)), 4.81 (s, 1H), 4.43-4.22 (m, 2H), 3.78-3.53 (m, 2H), 3.52-3.32 (m, 2H), 3.28-3.08 (m, 5H), 3.05-2.85 (m, 1H), 2.25-1.15 (m, 18H), 0.83-0.76 (m, 3H), 0.70-0.15 (m, 3H). |
| **54** | 475.6 | poudre blanche | 99 | 476.3 | 5.63 (s, 1H(C7)), 4.78-4.68 (m, 1H), 4.45-4.32 (m, 2H), 4.28-4.08 (m, 2H), 3.8-3.7 (m, 1H), 3.68-3.54 (m, 1H), 3.28-3.18 (m, 5H), 3.05-2.85 (m, 1H), 2.25-1.09 (m, 17H), 0.88-0.7 (m, 7H), 0.68-0.52 (m, 3H). |
| **55** | 459.6 | poudre blanche | 99 | 460.3 | 5.63 (s, 1H(C7)), 4.78-4.52 (m, 1H), 4.48-4.32 (m, 2H), 3.76 (s, 1H), 3.68-3.54 (m, 1H), 3.05-2.85 (m, 1H), 2.48-1.08 (m, 21H), 1.02-0.92 (m, 3H), 0.90-0.69 (m, 7H), 0.68-0.52 (m, 3H). |
| **56** | 532.7 | poudre blanche | 99 | 533.3 | 7.89 (d, 2H), 7.60 (d, 2H), 5.66 (s, 1H(C7)), 4.81 (s, 1H), 4.49-4.32 (m, 2H), 3.76 (s, 1H), 3.68-3.54 (m, 1H), 3.05-2.85 (m, 1H), 2.80-2.72 (m, 1H), 2.27-1.18 (m, 18H), 0.85 (s, 3H), 0.73-0.22 (m, 7H). |
| **57** | 534.7 | poudre blanche | 99 | 535.3 | 5.62 (s, 1H(C7)), 4.78 (s, 1H), 4.46 (d, 1H), 4.39-4.35 (m, 1H), 3.75 (s, 1H), 3.68-3.4 (m, 5H), 3.28-3.13 (m, 5H), 3.10-2.82 (m, 7H), 2.4-1.1 (m, 18H), 0.88-0.75 (m, 3H), 0.65-0.51 (m, 3H). |
| **58** | 508.7 | poudre rose | 99 | 509.4 | 8.68-8.55 (m, 2H), 7.85-7.77 (m, 1H), 7.5-7.35 (m, 1H), 5.66 (s, 1H(C7)), 4.82 (s, 1H), 4.48-4.30 (m, 2H), 3.76 (s, 1H), 3.68-3.54 (m, 1H), 3.12-2.78 (m, 2H), 2.25-1.15 (m, 18H), 0.88-0.18 (m, 10H). |
| **59** | 537.7 | poudre blanche | 92 | 538.8 | 7.42 (t, 2H), 6.98-6.85 (m, 2H), 5.69-5.62 (m, 1H(C7)), 4.85-4.79 (m, 1H), 4.49-4.35 (m, 2H), 3.78-3.73 (m, 4H), 3.67-3.51 (m, 1H), 3.10-2.87 (m, 2H), 2.27-1.20 (m, 18H), 0.87-0.83 (m, 3H), 0.74-0.18 (m, 7H). |
| **60** | 545.7 | poudre blanche | 99 | 546.2 | 7.83 (s, 1H), 6.94 (s, 1H), 6.61 (s, 1H), 5.67-5.57 (m, 1H(C7)), 4.84 (s, 1H), 4.63-4.17 (m, 4H), 3.75 (s, 1H), 3.67-3.51 (m, 2H), 3.29-3.24 (m, 6H), 3.10-2.87 (m, 1H), 2.25-1.03 (m, 18H), 0.85-0.74 (m, 3H), 0.70-0.10 (m, 3H). |
| 61 | 505.7 | poudre blanche | 99 | 506.2 | 6.85-6.65 (m, 1H), 6.20-6.00 (m, 1H), 5.70-5.62 (m, 2H), 4.87-4.70 (m, 1H), 4.48-4.36 (m, 2H), 3.75 (s, 1H), 3.65-3.42 (m, 2H), 3.29-3.24 (m, 6H), 3.10-2.87 (m, 2H), 2.27-0.98 (m, 19H), 0.86-0.78 (m, 3H), 0.70-0.46 (m, 3H). |
| **62** | 519.7 | poudre blanche | 99 | 520.3 | 5.63 (s, 1H(C7)), 4.9-4.65 (m, 1H), 4.48-4.36 (m, 2H), 4.25-3.85 (m, 3H), 3.82-3.70 (m, 2H), 3.68-3.56 (m, 2H), 3.29-3.23 (m, 3H), 3.08-2.93 (m, 2H), 2.28-1.02 (m, 23H), 0.86-0.79 (m, 3H), 0.66-0.47 (m, 3H). |
| **63** | 541.7 | poudre blanche | 97 | 542.2 | 7.84-7.75 (m, 1H), 6.94-6.86 (m, 1H), 6.62-6.57 (m, 1H), 5.64-5.53 (m, 1H(C7)), 4.84 (s, 1H), 4.48-4.36 (m, 2H), 4.34-3.80 (m, 2H), 3.78-3.50 (m, 5H), 3.08-2.93 (m, 2H), 2.24-1.05 (m, 21H), 0.85-0.74 (m, 3H), 0.73-0.16 (m, 3H). |
| **64** | 561.7 | poudre blanche | 97 | pas de masse | 7.77-7.71 (m, 1H), 7.41-7.30 (m, 1H), 7.13-7.09 (m, 1H), 5.70-5.56 (m, 1H(C7)), 4.84 (s, 1H), 4.62-4.17 (m, 4H), 3.74-3.51 (m, 3H), 3.32-3.26 (m, 6H), 3.08-2.93 (m, 2H), 2.24-1.15 (m, 17H), 0.85-0.70 (m, 3H), 0.68-0.17 (m, 3H). |
| **65** | 519.7 | poudre blanche | 97 | 520.2 | 5.68-5.60 (m, 1H(C7)), 4.86-4.70 (m, 1H), 4.56-4.34 (m, 3H), 3.75 (s, 1H), 3.67-3.53 (m, 2H), 3.30-3.23 (m, 6H), 3.10-2.9 (m, 2H), 2.23-0.93 (m, 19H), 0.87-0.77 (m, 3H), 0.75-0.40 (m, 7H). |
| **66** | 521.7 | poudre blanche | 94 | 522.3 | 5.63 (s, 1H(C7)), 4.86-4.70 (m, 1H), 4.55-4.34 (m, 3H), 3.76 (s, 1H), 3.68-3.53 (m, 1H), 3.35-3.20 (m, 6H), 3.10-2.87 (m, 2H), 2.29-0.9 (m, 26H), 0.88-0.72 (m, 3H), 0.69-0.41 (m, 3H). |
| **67** | 521.6 | poudre blanche | 99 | 522.1 | 7.87 (s, 1H), 7.07-7.01 (m, 1H), 6.67-6.61 (m, 1H), 5.63-5.54 (m, 1H(C7)), 4.88-4.84 (m, 1H), 4.45-4.32 (m, 3H), 4.01-3.35 (m, 3H), 3.10-2.85 (m, 1H), 2.25-1.05 (m, 19H), 0.80-0.76 (m, 3H), 0.70-0.17 (m, 3H). |
| **68** | 495.6 | poudre blanche | 99 | 496.2 | 6.43-5.87 (m, 1H), 5.67-5.63 (m, 1H(C7)), 4.88-4.70 (m, 1H), 4.47-4.25 (m, 3H), 3.80-3.52 (m, 3H), 3.10-2.85 (m, 1H), 2.30-0.95 (m, 19H), 0.90-0.46 (m, 10H). |
| **69** | 481.6 | poudre blanche | 99 | 482.1 | 6.95-6.68 (m, 1H), 6.38-5.90 (m, 2H), 5.78-5.61 (m, 2H), 4.88-4.70 (m, 1H), 4.47-4.34 (m, 2H), 4.15-3.85 (m, 1H), 3.83-3.74 (m, 2H), 3.68-3.5 (m, 1H), 3.10-2.85 (m, 1H), 2.30-0.98 (m, 18H), 0.81-0.77 (m, 3H), 0.65 (s, 1H), 0.50(s,2H). |
| **70** | 427.5 | poudre jaune | 99 | 428.1 | 6.13-5.59 (m, 1H), 5.61 (s, 1H(C7)), 4.63 (s, 1H), 4.44 (s, 1H), 4.35 (s, 1H), 3.76 (s, 1H), 3.68-3.53 (m, 1H), 3.10-2.85 (m, 1H), 2.30-2.15 (m, 2H), 2-1.1 (m, 16H), 0.97 (s, 3H), 0.84 (s, 3H), 0.61 (s, 3H). |
| **71** | 527.6 | poudre jaune | 99 | 528.2 | 6.13-5.71 (m, 1H), 5.67 (s, 1H(C7)), 5.03-4.99 (m, 1H), 4.77 (s, 1H), 4.70-4.57 (m, 1H), 3.99 (s, 1H), 3.12-3 (m, 1H), 2.92-2.70 (m, 2H), 2.30-2.24 (m, 1H), 2.10-1.18 (m, 24H), 1.08-0.96 (m, 3H), 0.87 (s, 3H), 0.57 (s, 3H). |
| **72** | 526.7 | poudre jaune | 99 | 527.2 | 8.50-8.37 (m, 2H), 7.64-7.60 (m, 1H), 7.45-7.28 (m, 1H), 5.67-5.62 (m, 1H(C7)), 4.95-4 (m, 6H), 3.83-3.50 (m, 3H), 3.35-3.24 (m, 3H), 3.12-2.85 (m, 1H), 2.25-0.80 (m, 18H), 0.84 (s, 3H), 0.70 (m, 3H). |
| **73** | 548.7 | poudre jaune | 99 | 549.2 | 8.55-8.32 (m, 2H), 7.88-7.80 (m, 1H), 7.75-7.60 (m, 1H), 7.34-7.25 (m, 1H), 7.05-6.80 (m, 1H), 6.66-6.40 (m, 1H), 5.62 (s, 1H(C7)), 4.90-4.70 (m, 2H), 4.49-4.27 (m, 3H), 3.76 (s, 1H), 3.69-3.53 (m, 1H), 3.12-2.85 (m, 1H), 2.27-0.95 (m, 18H), 0.84-0.73 (m, 3H), 0.65-0.19 (m, 3H). |
| **74** | 522.7 | poudre jaune | 96 | 523.2 | 8.60-8.30 (m, 2H), 7.73-7.5 (m, 1H), 7.41-7.25 (m, 1H), 5.67-5.62 (m, 1H(C7)), 4.92-4.75 (m, 2H), 4.5-4.3 (m, 2H), 3.76 (s, 1H), 3.69-3.53 (m, 1H), 3.12-2.89 (m, 1H), 2.28-0.96 (m, 20H), 0.97-0.40 (m, 10H). |
| **75** | 491.7 | poudre blanche | 99 | 492.2 | 5.68-5.60 (m, 1H(C7)), 4.84 (s, 1H), 4.82-4.65 (m, 1H), 4.46-4.32 (m, 2H), 3.76 (s, 1H), 3.68-3.52 (m, 2H), 3.48-3.38 (m, 1H), 3.23-3.19 (m, 5H), 3.20-2.71 (m, 3H), 2.29-0.90 (m, 22H), 0.80-0.77 (m, 3H), 0.71-0.45 (m, 3H). |

| | | | | | |
|---|---|---|---|---|---|
| ¹Pureté LCMS, UV à 254 nm | | | | | |

### Exemple 6 : Schéma F ;

### Préparation du composé n°76 : (2S,3R,5R,10R,13R,14S,17S)-2,3,14-trihydroxy-17-[1-(2-méthoxyéthyl(méthyl)amino)éthyl]-10,13-diméthyl-2,3,4,5,9,11,12,15,16,17-décahydro-1H-cyclopenta[a]phénanthrèn-6-one

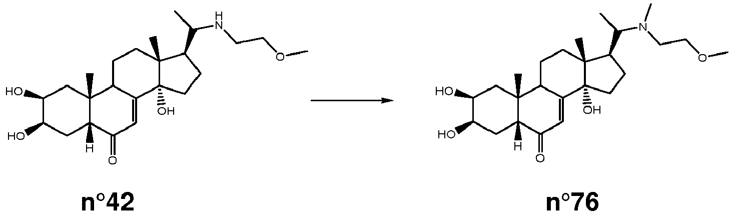

155 mg (0,368 mmol) de composé n°**42** [(2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one] préparés suivant la technique décrite à l'étape 1 de l'exemple 5 sont solubilisés dans 2,5 mL de DMF et 61,8 mg (0,735 mmol) de bicarbonate de soude sont ajoutés au milieu réactionnel ainsi que 0,034 mL (0,552 mmol) de iodométhane. La suspension obtenue est agitée à 20°C 20h.La solution est ensuite versée sur 15 mL d'eau et extraite trois fois avec 15 mL de butanol. La phase butanolique est évaporée pour donner 220 mg de poudre purifiée par chromatographie flash sur cartouche de gel de silice (Dichlorométhane/MeOH, 95/5) pour donner 40 mg de poudre blanche (Rendement: 25%) du composé n°**76** : (2S,3R,5R,10R,13R,14S,17S)-2,3,14-trihydroxy-17-[1-(2-méthoxyéthyl(méthyl)amino)éthyl]-10,13-diméthyl-2,3,4,5,9,11,12,15,16,17-décahydro-1H-cyclopenta[a]phénanthrèn-6-one sous forme de poudre blanche.

### Composé n°76 :

LC-MS: m/z = 436.3 (MH⁺) pureté UV à 254 nm = 99%.
RMN ¹H (300 MHz, DMSO-*d₆*)δ 5.61 (s, 1H(C7)), 4.64 (s, 1H), 4.47-4.34 (m, 2H), 3.75 (s, 1H), 3.67-3.50 (m, 1H), 3.25-3.16 (m, 5H), 3.05-2.85 (m, 1H), 2.27-1.15 (m, 20H), 0.90-0.70 (m, 6H), 0.59 (s, 3H).

Les composés n°**77** à **80** ont été préparés selon le même schéma.

| **N°** | **PM g/mol** | **Apparence** | **Pureté¹ (%)** | **SM m/z MH⁺** | **RMN ¹H(300 MHz, DMSO-*d₆*) δ** |
|---|---|---|---|---|---|
| **77** | 461.6 | poudre blanche | 86 | 462.3 | 5.68-5.58 (m, 1H(C7)), 4.50-4.30 (m, 2H), 3.85-3.50 (m, 4H), 3.10-2.90 (m, 2H), 2.25-1.10 (m, 25H), 0.90-0.75 (m, 6H), 0.65-0.45 (m, 3H). |
| **78** | 417.6 | poudre blanche | 99 | 418.3 | 5.63-5.58 (m, 1H(C7)), 4.65 (s, 1H), 4.42 (d, 1H), 4.38-4.33 (m, 1H), 3.75 (s, 1H), 3.67-3.53 (m, 1H), 3.04-2.90 (m, 1H), 2.96-2.67 (m, 1H), 2.23-1.12 (m, 18H), 0.89 (d, 3H), 0.82 (s, 3H), 0.57-0.15 (m, 7H). |
| **79** | 465.6 | poudre beige | 94 | 466.2 | 5.67-5.57 (m, 1H(C7)), 4.70-4.64 (m, 1H), 4.48-4.33 (m, 3H), 3.76 (s, 1H), 3.65-3.55 (m, 1H), 3.27-3.22 (m, 6H), 3.05-2.85 (m, 1H), 2.26-1.10 (m, 20H), 0.87-0.73 (m, 6H), 0.64-0.47 (m, 3H). |
| **80** | 441.6 | poudre jaune | 99 | 442.1 | 6.25-5.75 (m, 1H), 5.65-5.61 (m, 1H(C7)), 4.71-4.66 (m, 1H), 4.48-4.40 (m, 1H), 4.39-4.35 (m, 1H), 3.76 (s, 1H), 3.65-3.55 (m, 1H), 3.05-2.85 (m, 1H), 2.76-2.70 (m, 1H), 2.28-1.15 (m, 19H), 0.90-0.80 (m, 6H), 0.62-0.46 (m, 3H). |

| | | | | | |
|---|---|---|---|---|---|
| ¹Pureté LCMS, UV à 254 nm | | | | | |

### Exemple 7 : Schéma G ;

### Préparation du composé n°81 : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one

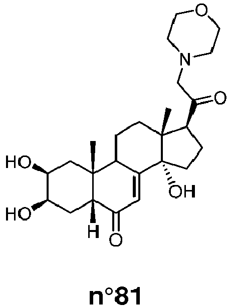

### Etape 1 : Préparation du composé n°702 : (2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one

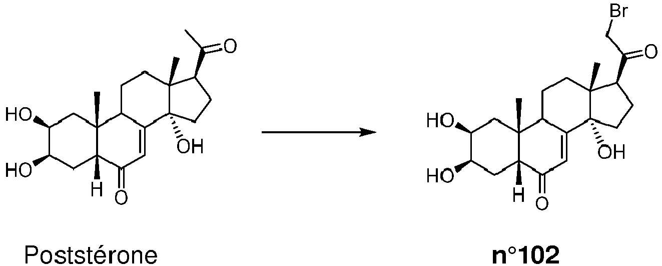

1 g (2,76 mmol) de poststérone (obtenue par coupure oxydante de la chaine de la 20-hydroxyecdysone suivant le même mode opératoire que celui décrit à l'étape 2 du schéma B) sont solubilisés dans 20 mL de méthanol. La solution est refroidie à 0°C et 0,284 mL (5,52 mmol) de brome sont ajoutés goutte à goutte et le milieu réactionnel est agité 1 h à cette température, puis laissée à température ambiante 16 h. Le milieu réactionnel est versé sur 50 mL d'une solution saturée de bicarbonate de soude et extraite trois fois avec 100 mL d'acétate d'éthyle. Les phases organiques sont lavées avec 50 mL de solution saturée de bicarbonate de soude, puis d'eau salée, séchées sur sulfate de sodium, filtrées et le solvant évaporé pour donner 833 mg de poudre, qui, reprise dans 30 mL de dichlorométhane, donne après filtration et dessiccation 412 mg (Rendement : 31 %) de composé n°**102** : 2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one sous forme de poudre jaune.

### Composé n°102 :

LC-MS: m/z = 443.1 (MH⁺) pureté UV à 254 nm = 91 %.
RMN ¹H (300 MHz,DMSO-*d₆*)δ 5.69-5.63 (m,1H(C7)), 5.08 (s, 1H), 4.42-4.35 (m, 3H), 4.33-4.22 (m, 1H), 3.77 (s, 1H), 3.66-3.58 (m, 1H), 3.39 (t, 1H), 3.10-2.95 (m, 1H), 2.25-1.20 (m, 13H), 0.83 (s, 3H), 0.51 (s, 3H).

### Etape 2 : Préparation du composé n°81 :(2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one

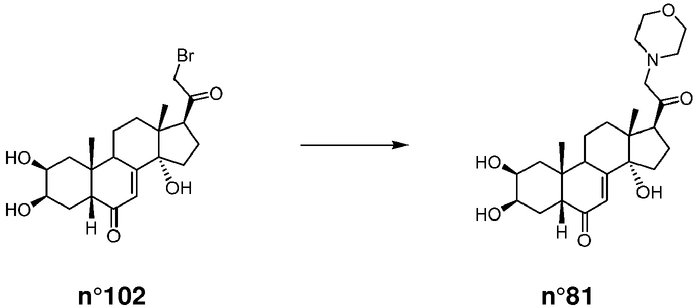

50 mg (0,103 mmol) de composé n°**102** [(2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one] sont solubilisés dans 1 mL de DMF et 42,7 mg de carbonate de potassium sont ajoutés ainsi que 10,78 µl (0,124 mmol) de morpholine. Après 18 h d'agitation à 20°C, le milieu réactionnel est versé sur 10 mL d'eau et cette phase aqueuse est extraite deux fois avec 15 mL de butanol. La phase organique est évaporée pour donner 71 mg de poudre purifiée par chromatographie flash sur cartouche de gel de silice (Dichlorométhane/MeOH, 90/10) pour donner 28 mg (Rendement : 60 %) du composé n°**81** :(2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one sous forme de poudre blanche.

### Composé n°81 :

LC-MS: m/z = 448.4 (MH⁺) pureté UV à 254 nm = 99 %.
RMN ¹H (300 MHz,DMSO-*d₆*)δ 5.65 (s, 1H(C7)), 5.02 (s, 1H), 4.45 (d, 1H), 4.40-4.37 (m, 1H), 3.77 (s, 1H), 3.68-3.53 (m, 5H), 3.34-3.24 (m, 4H), 3.08-2.95 (m, 1H), 2.45-1.17 (m, 16H), 0.82 (s, 3H), 0.48 (s, 3H).

Les composés n°**82** à **94** ont été préparés selon le même schéma.

| **N°** | **PM g/mol** | **Apparence** | **Pureté¹ (%)** | **SM m/z MH⁺** | **RMN ¹H (300 MHz, DMSO-*d₆*) δ** |
|---|---|---|---|---|---|
| **82** | 474.6 | poudre orange | 99 | 475.3 | 5.65 (s, 1H(C7)), 5.03 (s, 1H), 4.49-4.38 (m, 2H), 3.77 (s, 1H), 3.68-3.53 (m, 1H), 3.3-3.1 (m, 4H), 3.08-2.95 (m, 1H), 2.9-2.45 (m, 6H), 2.28-0.92 (m, 19H), 0.82 (s, 3H), 0.48 (s, 3H). |
| **83** | 475.6 | poudre blanche | 99 | 476.6 | 5.65 (s, 1H(C7)), 5.01 (s, 1H), 4.45 (d, 1H), 4.41-4.38 (m, 1H), 3.77 (s, 1H), 3.7-3.48 (m, 3H), 3.35-3.23 (m, 2H), 3.08-2.95 (m, 1H), 2.72-2.55 (m, 2H), 2.28-2 (m, 3H), 1.9-1.38 (m, 12H), 1.35-1.18 (m, 1H), 1.05-0.85 (m, 6H), 0.82 (s, 3H), 0.48 (s, 3H). |
| **84** | 462.6 | poudre jaune | 99 | 463.3 | 5.66 (s, 1H(C7)), 5.08 (s, 1H), 4.55-4.25 (m, 2H), 3.77 (s, 1H), 3.70-3.45 (m, 4H), 3.25-2.90 (m, 5H), 2.80-2.60 (m, 6H), 2.33-1.15 (m, 16H), 0.83 (s, 3H), 0.50 (s, 3H). |
| **85** | 479.6 | huile jaune | 99 | 480.3 | 5.65 (s, 1H(C7)), 5.01 (s, 1H), 4.48-4.37 (m, 3H), 3.77 (s, 1H), 3.70-3.52 (m, 1H), 3.25 (s, 6H), 3.25-2.90 (m, 1H), 2.57-2.47 (m, 2H), 2.3-1.19 (m, 19H), 0.82 (s, 3H), 0.48 (s, 3H). |
| **86** | 447.6 | huile orange | 99 | 448.2 | 5.67 (s, 1H(C7)), 5.44-5.39 (m, 1H), 5.14 (s, 1H), 4.5-4.15 (m, 5H), 3.77 (s, 1H), 3.68-3.55 (m, 1H), 3.27-3.15 (m, 4H), 3.25-2.90 (m, 1H), 2.25-1.17 (m, 16H), 0.83 (s, 3H), 0.54 (s, 3H). |
| **87** | 435.6 | huile incolore | 99 | 436.2 | 5.65 (s, 1H(C7)), 5.02 (s, 1H), 4.49-4.38 (m, 3H), 3.77 (s, 1H), 3.68-3.55 (m, 1H), 3.52-3.36 (m, 2H), 3.30-3.18 (m, 2H), 3.25-2.95 (m, 1H), 2.30-1.17 (m, 19H), 0.82 (s, 3H), 0.48 (s, 3H). |
| **88** | 461.6 | poudre blanche | 99 | 462.2 | 5.65 (s, 1H(C7)), 5.02 (s, 1H), 4.58 (s, 1H), 4.46 (s, 1H), 4.42-4.39 (m, 1H), 3.77 (s, 1H), 3.68-3.55 (m, 1H), 3.48-3.33 (m, 1H), 3.31-3.17 (m, 2H), 3.08-2.95 (m, 1H), 2.70-2.56 (m, 2H), 2.3-1.18 (m, 20H), 0.82 (s, 3H), 0.47 (s, 3H). |
| **89** | 489.7 | huile incolore | 99 | 490.3 | 5.65 (s, 1H(C7)), 5.01 (s, 1H), 4.48-4.30 (m, 3H), 3.77 (s, 1H), 3.68-3.55 (m, 1H), 3.45-3.35 (m, 2H), 3.28-3.11 (m, 2H), 3.08-2.95 (m, 1H), 2.85-2.58 (m, 2H), 2.28-1.03 (m, 23H), 0.82 (s, 3H), 0.47 (s, 3H). |
| **90** | 459.6 | huile incolore | 99 | 460.3 | 5.64 (s, 1H(C7)), 5.01 (s, 1H), 4.49-4.38 (m, 2H), 3.77 (s, 1H), 3.68-3.55 (m, 1H), 3.25-3.20 (m, 1H), 3.10-2.85 (m, 2H), 2.77-2.61 (m, 2H), 2.25-1.03 (m, 21H), 0.88 (d, 3H), 0.82 (s, 3H), 0.47 (s, 3H). |
| **91** | 476.7 | poudre jaune | 99 | 477.3 | 5.66 (s, 1H(C7)), 5.10 (s, 1H), 4.55-4.40 (m, 2H), 3.77 (s, 1H), 3.68-3.55 (m, 1H), 3.22 (t, 2H), 3.15-2.93 (m, 3H), 2.75 (s, 6H), 2.35-1.20 (m, 21H), 0.83 (s, 3H), 0.51 (s,3H). |
| **92** | 480.6 | poudre blanche | 99 | 481.1 | 5.65 (s, 1H(C7)), 5.06 (s, 1H), 4.46 (d, 1H), 4.41-4.38 (m, 1H), 4.08 (q, 2H), 3.77 (s, 1H), 3.68-3.51 (m, 3H), 3.32 (s, 2H), 3.08-2.90 (m, 1H), 2.28-1.25 (m, 14H), 1.19 (t, 3H), 0.82 (s, 3H), 0.49 (s, 3H). |
| **93** | 422.6 | poudre blanche | 99 | 423.1 | 5.65 (s, 1H(C7)), 5.06 (s, 1H), 4.46 (d, 1H), 4.41-4.38 (m, 1H), 3.77 (s, 1H), 3.68-3.51 (m, 1H), 3.50-3.36 (m, 2H), 3.08-2.90 (m, 1H), 2.48-2.40 (m, 2H), 2.28-1.20 (m, 14H), 1.14 (t, 3H), 0.82 (s, 3H), 0.51 (s, 3H). |
| **94** | 438.6 | poudre blanche | 99 | 439.2 | 5.65 (s, 1H(C7)), 5.05 (s, 1H), 4.81 (t, 1H), 4.46 (d, 1H), 4.41-4.38 (m, 1H), 3.77 (s, 1H), 3.68-3.55 (m, 1H), 3.52-3.40 (m, 4H), 3.08-2.90 (m, 1H), 2.58-2.50 (m, 2H), 2.23-1.20 (m, 14H), 0.82 (s, 3H), 0.50 (s, 3H). |

| | | | | | |
|---|---|---|---|---|---|
| ¹Pureté LCMS, UV à 254 nm | | | | | |

### Exemple 8 : Schéma H ;

### Préparation du composé n°95 : (2S,3R,5R,10R,13R,14S,17S)-17-(2-éthoxyacétyl)-2,3,14-trihydroxy-10,13-diméthyl-2,3,4,5,9,11,12,15,16,17-décahydro-1H-cyclopenta[a]phénanthrèn-6-one

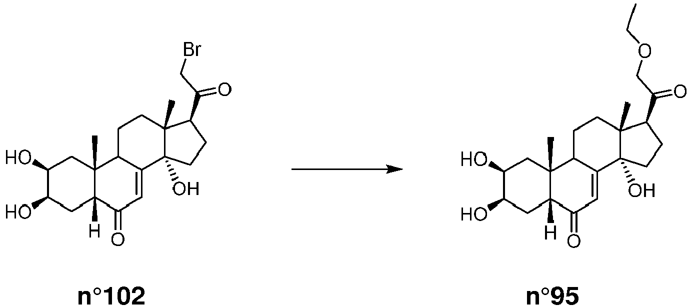

100 mg (0,227 mmol) de composé n°**102** [2S,3R,5R,10R,13R,14S,17S)-17-(2-bromoacétyl)-2,3,14-trihydroxy-10,13-diméthyl-2,3,4,5,9,11,12,15,16,17-décahydro-1H-cyclopenta[a]phénanthrèn-6-one] préparés à l'étape 1 de l'exemple 7 sont solubilisés dans 2 mL d'éthanol et 0,102 mL (0,272 mmol) d'une solution d'éthoxide de sodium à 21 % dans l'éthanol dilués dans 1 mL d'éthanol sont ajoutés goutte à goutte et la solution obtenue est portée au reflux 30 min. Le milieu réactionnel refroidit à 20°C est versé sur 25 mL d'eau et extrait avec deux fois 20 mL de butanol. La phase organique est évaporée pour donner 30 mg d'une huile purifiée par chromatographie flash sur cartouche de gel de silice (Dichlorométhane/MeOH, 95/5) pour donner 13,5 mg (Rendement: 14 %) du composé n°**95** : (2S,3R,5R,10R,13R,14S,17S)-17-(2-éthoxyacétyl)-2,3,14-trihydroxy-10,13-diméthyl-2,3,4,5,9,11,12,15,16,17-décahydro-1H-cyclopenta[a]phénanthrèn-6-one sous forme d'huile jaune.

### Composé n°95 :

LC-MS: m/z = 407.2 (MH⁺) pureté UV à 254 nm = 93 %.
RMN ¹H (300 MHz,DMSO-*d₆*)δ 5.65-5.59 (m, 1H(C7)), 4.96 (s, 1H), 4.46 (d, 1H), 4.41-4.36 (m, 1H), 4.03 (q, 2H), 3.77 (s, 1H), 3.68-3.55 (m, 1H), 3.08-2.90 (m, 1H), 2.75-2.62 (m, 1H), 2.3-2.15 (m, 2H), 1.92-1.42 (m, 13H), 1.18 (t, 3H), 0.83 (s, 3H), 0.58-0.49 (m, 3H).

Le composé n°**96** a été préparé selon le même schéma.

| **N°** | **PM g/mol** | **Apparence** | **Pureté¹ (%)** | **SM m/z MH⁺** | **RMN ¹H(300 MHz,** DMSO-*d₆***) δ** |
|---|---|---|---|---|---|
| **96** | 448.6 | Poudre blanche | 99 | 449.1 | 5.61 (s, 1H(C7)), 5.23-5.15 (m, 1H), 4.97 (s, 1H), 4.48-4.42 (m, 1H), 4.39-4.35 (m, 1H), 3.82-3.55 (m, 6H), 3.08-2.90 (m, 1H), 2.72-2.55 (m, 1H), 2.32-1.17 (m, 17H), 0.83 (s, 3H), 0.58 (s, 3H). |

| | | | | | |
|---|---|---|---|---|---|
| ¹Pureté LCMS, UV à 254 nm | | | | | |

### Exemple 9 : Schéma I ;

### Préparation du composé n°97 : (2S,3R,5R,10R,13R,14S,17S)-2,3,14-trihydroxy-17-[1-hydroxy-2-(2-hydroxyéthyl(méthyl)amino)éthyl]-10,13-diméthyl-2,3,4,5,9,11,12,15,16,17-décahydro-1H-cyclopenta[a]phénanthrèn-6-one

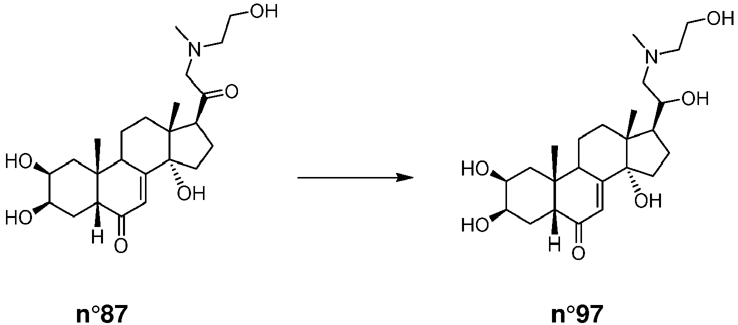

157 mg (0.360 mmol) de composé n°**87** [(2S,3R,5R,10R,13R,14S,17S)-2,3,14-trihydroxy-17-[2-(2-hydroxyéthyl(méthyl)amino)acétyl]-10,13-diméthyl-2,3,4,5,9,11,12,15,16,17-décahydro-1H-cyclopenta[a]phénanthrèn-6-one] obtenus suivant la méthode de l'étape 2 de l'exemple 7 sont solubilisés dans 7,5 mL d'éthanol et 21,14 mg (0,559 mmol) de borohydrure de sodium sont ajoutés par portions. Après 16 h d'agitation à 20 °C, le milieu réactionnel est versé sur 20 mL d'eau et extrait avec trois fois 15 mL de butanol. La phase organique est évaporée pour donner une poudre purifiée par chromatographie flash sur cartouche de gel de silice (Dichlorométhane/MeOH/NH₄OH, 85/14/1) pour donner 96 mg (Rendement : 60 %) du composé n°**97** : (2S,3R,5R,10R,13R,14S,17S)-2,3,14-trihydroxy-17-[1-hydroxy-2-(2-hydroxyéthyl(méthyl)amino)éthyl]-10,13-diméthyl-2,3,4,5,9,11,12,15,16,17-décahydro-1H-cyclopenta[a]phénanthrèn-6-one sous forme de poudre blanche.

### Composé n°97 :

LC-MS: m/z = 438.2 (MH⁺) pureté UV à 254 nm = 99 %.
RMN ¹H (300 MHz, DMSO-*d₆*)δ 5.6 (s, 1H(C7)), 5.32-5.2 (m, 2H), 4.77 (s, 1H), 4.47 (d, 1H), 4.42-4.38 (m, 1H), 3.92-3.57 (m, 4H), 3.3-2.95 (m, 4H), 2.82 (s, 3H), 2.31-1.18 (m, 16H), 0.85 (s, 3H), 0.69 (s, 3H).
RMN ¹³C (75 MHz, DMSO-*d₆*)δ 203.2, 164.9, 121.0, 82.8, 66.9, 59.0, 55.6, 50.6, 46.9, 40.7, 37, 34, 31.9, 31.1, 30.3, 24.5, 23.2, 20.4, 16.3.

Les composés n°**98** à **100** ont été préparés selon le même schéma.

| **N°** | **PM g/mol** | **Apparence** | **Pureté ¹ (%)** | **SM m/z MH⁺** | **RMN ¹H (300 MHz,** DMSO-*d₆***) δ** |
|---|---|---|---|---|---|
| **98** | 440.6 | poudre blanche | 99 | 441.1 | 5.6 (s, 1H(C7)), 4.76 (t, 1H), 4.65 (s, 1H), 4.47 (d, 1H), 4.43 (d, 1H), 4.36-4.33 (m, 1H), 3.76 (s, 1H), 3.82-3.55 (m, 1H), 3.53-3.45 (m, 3H), 3.08-2.90 (m, 1H), 2.65-2.55 (m, 3H), 2.45-1.10 (m, 15H), 0.84 (s, 3H), 0.63 (s, 3H). |
| **99** | 491.66 | poudre blanche | 99 | 492.2 | 5.61 (s, 1H(C7)), 4.70-4.62 (m, 1H), 4.48-4.28 (m, 3H), 3.76 (s, 1H), 3.65-3.55 (m, 1H), 3.53-3.31 (m, 3H), 3.03-2.84 (m, 2H), 2.78-2.65 (m, 1H), 2.25-1.04 (m, 26H), 0.84 (s, 3H), 0.70-0.55 (m, 3H). |
| **100** | 449.58 | huile orange | 98 | 450.2 | (in DMSO+D20) δ 5.65-5.58 (m, 1H(C7)), 4.23-4.13 (m, 1H), 3.75 (s, 1H), 3.04-2.93 (m, 1H), 2.87-2.65 (m, 2H), 2.25-1.10 (m, 22H), 0.83 (s, 3H), 0.63 (m, 3H). |

| | | | | | |
|---|---|---|---|---|---|
| ¹Pureté LCMS, UV à 254 nm | | | | | |

### Exemple 10 : Schéma J ;

### Préparation du composé n°101 : (2S,3R,5R,6E,10R,13R,14S,17S)-6-méthoxyimino-17-(N-méthoxy-C-(morpholinométhyl)carbonimidoyl)-10,13-diméthyl-2,3,4,5,9,11,12,15,16,17-décahydro-1H-cyclopenta[a]phénanthrène-2,3,14-triol

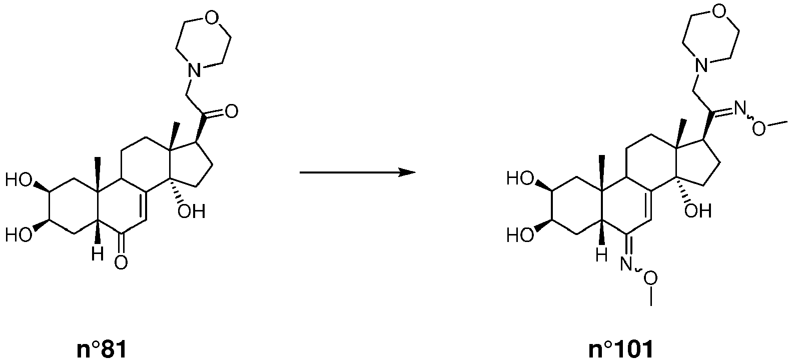

30 µl (0,301 mmol) de chlorhydrate de méthoxylamine sont solubilisés dans 0,6 mL de pyridine et 136 mg (0,301 mmol) de composé n°**81** [(2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one] préparés dans l'étape 2 de l'exemple 7 sont ajoutés par portions. Après 36 h d'agitation à 20°C, le milieu réactionnel est repris dans 10 mL de dichlorométhane et cette solution est lavée deux fois à l'eau salée, séchée sur sulfate de sodium, filtrée et évaporée pour donner une poudre purifiée par chromatographie flash sur cartouche de gel de silice (Dichlorométhane/MeOH, 90/10) pour donner 81 mg (Rendement : 53 %) du composé n°**101** : (2S,3R,5R,6E,10R,13R,14S,17S)-6-méthoxyimino-17-(N-méthoxy-C-(morpholinométhyl)carbonimidoyl)-10,13-diméthyl-2,3,4,5,9,11,12,15,16,17-décahydro-1H-cyclopenta[a]phénanthrène-2,3,14-triol sous forme de poudre jaune.

### Composé n°101 :

LC-MS: m/z = 506.2 (MH⁺) pureté UV à 254 nm = 99 %.
RMN ¹H (300 MHz, DMSO-*d₆*) δ Mélange de conformères (Z) et (E) en C6 : 6.28 (s,0.45H (C7-conformère E), 5.72 (s,0.55H (C7-conformère Z), 4.62 (s,0.45H-conformère E), 4.53 (s,0.55H-conformère Z), 4.47-4.35 (m,1H), 4.33-4.21(m,1H), 3.77-3.70 (m,7H), 3.60-3.48 (m,5H), 3.16-3.06 (m,1H), 2.90-2.70 (m,2H), 2.45-1.20 (m,18H), 0.72 (s,3H), 0.64-0.57 (m,3H).

### CASCADE DE SCREENING ET CARACTERISATION DES EFFETS BIOLOGIQUES DES DERIVÉS DE LA 20-HYDROXYECDYSONE

Le développement du test de criblage a été initié à partir des travaux de la littérature et basé sur les caractéristiques de la pathologie de la sarcopénie. Au niveau physiopathologique, cette maladie se caractérise par une diminution de la synthèse protéique et une augmentation de la protéolyse. Le développement de futurs médicaments doit donc être criblé sur des facteurs moléculaires en lien avec ces deux phénomènes.

Au niveau cellulaire, sur des cultures de cellules musculaires issues de la lignée murine C2C12, Gorelick-Feldman et al. (2008) ont montré qu'un traitement par les phytoecdysones augmente la synthèse protéique de +20 % en moyenne. Les premières études de mise au point se sont appuyées sur les conditions de culture et de traitement décrites par Gorelick-Feldman en présence de produits de références (IGF-1 et la 20-hydroxyecdysone ou 20E). Des mesures de l'incorporation de leucine tritiée dans ces cellules ont été réalisées pour évaluer la synthèse *de novo* de protéines. Ces premiers résultats ont permis de déterminer que la séquence optimale pour observer les effets des phytoecdysones sur la synthèse protéique était de différencier les cellules pendant 5 jours, puis de mettre de la leucine tritiée pendant 2h30 en présence d'IGF-1 ou de 20E.

L'analyse bibliographique a montré que des molécules telles qu'IGF-1 n'augmentaient la synthèse protéique que de 20 %, tout en activant des cibles de cette voie de signalisation de manière plus soutenue et pouvant atteindre des stimulations de l'ordre de 200 % [Kazi et al., 2010]. Ces cibles comprennent les phosphorylations activatrices de protéines telles que Akt ou la S6 kinase. D'autre part, dans le même système cellulaire C2C12, Zubeldia et al. (2012) ont analysé les phénomènes d'apoptose et de protéolyse. Dans leur étude, ils ont notamment rapporté que des extraits de plantes contenant les phytoecdysones comme la turkestérone ou la 20E étaient capables, après 24 h de traitement de cellules C2C12 différenciées, d'inhiber l'expression des gènes de la myostatine et de la caspase 3 d'un facteur 4 et 2, respectivement [Zubeldia et al., 2012].

Après plusieurs expériences dans lesquelles les cellules C2C12 différenciées en myotubes ont été incubées en présence d'IGF-1 ou de 20E pendant 2h30 ou 6 h, deux tests de criblage ont été développés. Ainsi, la phosphorylation de la protéine S6 kinase et l'expression du gène de la myostatine ont été étudiées afin de déterminer leur modulation par une hormone de croissance ou une ecdysone et de caractériser ces modulations d'un point de vue statistique.

### PROTOCOLES

### Inhibition de l'expression de la myostatine dans des cellules C2C12 :

Les cellules myoblastiques C2C12 (ATCC CRL-1772) sont ensemencées en plaques 24 puits à la densité de 30 000 cellules par puits et cultivées dans un milieu DMEM contenant du glucose à raison de 4.5 g/L et supplémenté en sérum de veau foetal (10%) et en antibiotiques (pénicilline et streptomycine). Quarante huit heures plus tard, les myoblastes sont induits en différenciation par privation partielle en sérum (2 % au lieu de 10 %) pendant 5 jours. Les cellules sont ensuite placées dans un milieu appauvri en glucose (DMEM contenant 1 g/L de glucose) et dépourvu de sérum en présence des molécules à tester ou des références (IGF-1 100 ng/mL ou 20E 10 µM) pendant 6 h. A la fin de l'expérience, les ARN messagers (ARNm) sont extraits en utilisant la méthodologie classique à base de phénol et chloroforme. Brièvement, les cellules sont lysées dans une solution de trizol (Sigma T9424) contenant un acide fort et du phénol. Les ARNm sont séparés des protéines par ajout de chloroforme puis centrifugation. Ils sont ensuite précipités dans l'isopropanol pour ensuite être suspendus à la concentration de 1µg/µL dans une eau ultra pure débarrassée des RNases et DNAses. 1 µg d'ARNm est alors converti par rétro transcription en ADN complémentaire par l'enzyme AMV en présence d'une amorce et d'un mélange de nucléotides selon le protocole donné par le fournisseur (Applied Biosystems 4368814). L'expression génique est étudiée par réaction en chaine initiée par une enzyme polymérase et couramment nommé PCR dans des conditions quantitatives, d'où le nom précis de qPCR. Les qPCR sont réalisées sur un 7900HT Fast real-Time PCR détection system (Applied Biosystems). Les conditions de programmation sont standard et consistent en 1 cycle à 95 °C pendant 15 min, suivi de 40 cycles à 95 °C pendant 15 s et à 60 °C pendant 1 min et on termine par une étape de courbe de fusion entre 60°C et 95°C. Les échantillons analysés contiennent tous 100 ng d'ADNc, un tampon de qPCR incluant l'enzyme, le mélange d'oligonucléotides et l'intercalant (le sybergreen ou SYBRgreen), et le couple d'amorces spécifiques du gène étudié, stratégiquement choisi entre deux séquences exoniques et à la concentration finale de 200 nM. Des sondes fluorescentes se fixent sur l'ADN double brin et ne fluorescent qu'une fois fixées à l'ADN. Un seuil de fluorescence est établi par le programme de la machine. Lorsque la quantité d'ADN permet à la sonde fluorescente de dépasser ce seuil, on obtient un numéro de cycle PCR appelé "Ct" pour "Cycle Threshold" ou cycle seuil. C'est cette valeur qui est à la base des calculs pour quantifier l'ADN de façon relative. On établit un rapport R entre la quantité d'ADN de départ d'un échantillon et celle d'un témoin, qui n'a pas subi de traitement (soit R=2^{-(Ct échantillon - Ct témoin)}) et cette mesure sera rapportée à celle d'un gène ménage connu pour ne pas être modulé par le traitement (soit R = 2^{-ΔΔACt}).

Les amorces utilisées sont consignées dans le tableau suivant :

**Tableau 3 : Amorces utilisées pour évaluer les modifications d'expression génique**

| Gène | Séquence 5' → 3' | Nb de bases | Tm | N° d'accession |
|---|---|---|---|---|
| Myostatine d | GAGTCTGACTTTCTAATGCAAG | 21 | 62 | souris: NM_010834 |
| Myostatine ind | TGTTGTAGGAGTCTTGACGG | 20 | 60 | rat: AF019624 |
| Atrogine d | AGAGTCGGCAAGTCTGTGCT | 20 | 62 | souris: AF441120 |
| Atrogine ind | GTGAGGCCTTTGAAGGCAG | 19 | 60 | humain: NM_058229 |
| beta-actine d | CTCTAGACTTCGAGCAGGAG | 20 | 62 | souris: X03672 |
| beta-actine ind | GGTACCACCAGACAGCACT | 19 | 60 | |

### Phosphorylation de la S6 kinase :

Les cellules myoblastiques C2C12 (ATCC CRL-1772) sont ensemencées en plaques 6 puits à la densité de 170 000 cellules par puits et cultivées dans un milieu DMEM contenant du glucose à raison de 4.5 g/L et supplémenté en sérum de veau foetal (10 %) et en antibiotiques (pénicilline et streptomycine). Quarante huit heures plus tard, les myoblastes sont induits en différenciation par privation partielle en sérum (2% au lieu de 10%) pendant 5 jours. Les cellules sont ensuite placées dans un milieu appauvri en glucose (DMEM contenant 1g/L de glucose) et dépourvu de sérum en présence des molécules à tester ou des références (IGF-1 100ng/mL ou 20E 10 µM) pendant 2 h. A la fin de l'expérience, les cellules sont lysées dans un tampon de lyse commercial (Invitrogen FNN0011) complété avec un mélange commercial d'anti-protéases (Roche 05056489001). Après centrifugation, la fraction cytoplasmique contenant les protéines solubles est gardée et la concentration en protéines est déterminée en utilisant un kit commercial (Biorad 500-0114) dont le principe est composé du dosage par la méthodologie de Lowry. Le dosage de la phosphorylation de S6 kinase est réalisé en utilisant un kit ELISA (Enzyme Linked ImmunoSorbent Assay) commercial (Cell signaling 7063). Brièvement, 50 µg de lysat protéique sont déposés dans le puits d'une microplaque 96 puits et incubés toute la nuit à 4 °C avec la solution d'antigène spécifique de l'anticorps pS6 kinase thréonine 389. La fixation de l'antigène sur le fond des puits se fait électrostatiquement. On incube ensuite à 37 °C dans les puits, la solution d'anticorps à doser (pS6K T389) pendant 2 heures. Les anticorps se fixent spécifiquement sur l'antigène. Les puits sont ensuite lavés pour éliminer avec du tampon de lavage les anticorps primaires spécifiques de l'antigène à doser qui sont en excès. La troisième étape consiste à fixer l'anticorps de détection. On incube à 37 °C dans les puits, la solution d'anticorps de détection pendant 1 heure. Les puits sont ensuite lavés pour éliminer les anticorps de détection en excès. Notons que les anticorps de détection sont couplés à une enzyme qui, en présence de son substrat, le transforme en produit de réaction détectable et mesurable grâce à l'apparition d'une coloration. La dernière étape consiste à révéler les anticorps fixés. On incube à 37 °C et à l'obscurité pendant 30 min, une solution révélatrice contenant le substrat pour l'enzyme, dans notre cas le TMB (3,3',5,5'-tétraméthylbenzidine). L'apparition d'une coloration bleue dans le substrat indique la présence de l'anticorps à doser. Pour éviter tout phénomène de saturation, une solution stop (contenant en général de la soude) est ajoutée et provoque un changement de coloration, qui de bleue devient jaune. L'intensité de celle-ci est proportionnelle à la quantité d'enzyme présente et donc à la concentration d'anticorps recherché. L'intensité du signal est mesurée par une spectrophotométrie à la longueur d'onde de 450 nm.

### Evaluation de l'effet des molécules dans un modèle de souris soumises à un régime hyperlipidique

La 20E en tant que composé comparatif et les composés selon l'invention (N°51 et 93) ont été administrées par voie orale, à la dose de 5 mg/kg de poids corporel, à des souris C57BL/6J de 12 semaines soumises à un régime hyperlipidique pendant 6 semaines. L'effet des composés sur le poids et la quantité de protéines du muscle *Soleus* ainsi que les transcrits de gènes impliqués dans la myogénèse ont été évalués.

La myogenèse, processus de formation des tissus musculaires, est contrôlée par plusieurs facteurs de transcription myogéniques qui agissent comme des effecteurs terminaux de la cascade de signalisation et produisent des transcrits impliqués dans les différents stades de développement. Les rôles des facteurs de transcription ont été bien décrits dans les différentes revues (Sabourin et Rudnicki 2000) et (Le Grand et Rudnicki 2007). La protéine Pax7 (Paired-box protein 7) maintient une population de cellules satellites en quiescence et, avec Myf5 (Myogenic factor 5), joue un rôle dans l'expansion de myoblastes activés. La protéine MyoD (Myoblast Détermination protein) semble déterminer le potentiel de différenciation de myoblastes activées, et coopère avec la myogénine et la protéine MEF2 (Myocyte Enhancer Factor 2) pour contrôler et entraîner la différenciation. Enfin, MRF4, (Muscle-specific Regulatory Factor 4) est requis pour l'hypertrophie, même si elle joue probablement d'autres rôles. De toute évidence, ces facteurs de transcription n'agissent pas seuls, mais existent dans le cadre de cascades de signalisation complexes qui contrôlent chaque étape de la myogenèse (Knight and Kothary, 2011).

La détermination de la quantité de protéines s'est faite en lysant d'abord les muscles prélevés dans une solution de NaOH 0.1N avec la technologie FastPrep. La quantification des protéines s'est faite par un dosage colorimétrique dérivé de la méthode de Lowry.

Pour réaliser l'analyse de l'expression des gènes, les tissus musculaires ont été homogénéisés dans une solution de Trizol (500 µl), et les ARN extraits et purifiés en utilisant la méthode au phénol / chloroforme. Une quantité de 1 pg d'ARN a été utilisée comme matrice pour la synthèse du premier brin d'ADNc en utilisant des oligo (dT) comme amorces et l'enzyme AMV reverse transcriptase comme décrit par le fabricant (Applied Biosystems 4368814). Les Q-PCR ont ensuite été effectuées en utilisant un appareil 7900HT muni d'un système rapide de détection en temps réel par PCR (Applied Biosystems) et le programme qPCR standard (1 cycle de 95°C 15min, 40 cycles 95°C 15s et 60°C 1min, une courbe de fusion 60-95°C pour les sondes de Sybergreen). Les expériences sont réalisées dans un master mix Sybergreen SYBR (Applied Biosystems) contenant les échantillons d'ADNc 100 ng et un ensemble d'amorces se fixant au niveau de 2 exons différents et à une concentration finale de 200 nM.

Les différences relatives de l'expression des gènes entre traitements sont exprimées en augmentation ou diminution des nombres des temps de cycle [Ct] comparé au groupe control. La valeur [Ct] de chaque gène ayant été normalisée avec le gène de la bêta actine.

### Etude de pharmacocinétique par voie orale des molécules chez le rat

La pharmacocinétique des composés a été évaluée par voie orale en utilisant des rats Wistar mâles (Charles River). La 20E en tant que composé comparatif a été administrée à une dose de 50 mg/kg de poids corporel. Les nouveaux composés selon l'invention ont été administrés à une dose de 10mg/kg de poids corporel sous la forme d'un mélange de 4 à 6 produits. Après administration, le sang a été prélevé au niveau de la queue à t = 0,25 h ; 0,5 h ; 1h ; 3h ; 6h et 8h. Les échantillons de sang ont été centrifugés et les plasmas prélevés. Le dosage des échantillons de plasma a permis la détermination des paramètres pharmacocinétiques, à savoir le Cₘₐₓ, qui correspond à la concentration maximale observée après l'administration de la molécule, le Tₘₐₓ qui est le temps requis pour atteindre la concentration maximale après administration de la molécule et l'AUC : l'aire sous la courbe composée des différentes concentrations de composés aux différents temps de prélèvements.

### RESULTATS

### • Les effets sur l'expression de la myostatine.

**Tableau 4 : Effets sur l'expression de la myostatine. Les résultats sont exprimés en pourcentage d'expression génique de la myostatine dans les cellules en contact avec les composés rapporté à l'expression dans les cellules contrôle. A représente un pourcentage inférieur à 70 %, B représente un pourcentage entre 71 % et 85 %. Les composés sont testés à la concentration de 10 µM.**

| Numéro | Expression génique Myostatine | Numéro | Expression génique Myostatine | Numéro | Expression génique Myostatine |
|---|---|---|---|---|---|
| **4** | A | **41** | A | **71** | A |
| **5** | A | **43** | A | **73** | B |
| **7** | A | **46** | A | **75** | A |
| **19** | B | **47** | A | **76** | A |
| **21** | A | **48** | B | **79** | A |
| **23** | B | **51** | A | **81** | A |
| **25** | A | **52** | A | **83** | B |
| **27** | A | **53** | A | **85** | B |
| **28** | A | **54** | A | **86** | A |
| **29** | A | **56** | B | **88** | B |
| **30** | B | **57** | B | **89** | A |
| **31** | A | **60** | B | **91** | B |
| **32** | A | **62** | A | **92** | A |
| **33** | A | **63** | A | **93** | A |
| **35** | B | **64** | A | **94** | A |
| **36** | B | **65** | A | **99** | A |
| **37** | B | **67** | A | **101** | A |
| **38** | A | **68** | A | | |

Les 38 composés suivants : 4, 5, 7, 21, 25, 27 à 29, 31 à 33, 38, 41, 43, 46, 47, 51 à 54, 62 à 65, 67, 68, 71, 75, 76, 79, 81, 86, 89, 92 à 94, 99 et 101 inhibent de façon très significative l'expression de la myostatine dans les cellules musculaires.
Les 15 composés suivants : 19, 23, 30, 35 à 37, 48, 56, 57, 60, 73, 83, 85, 88 et 91 inhibent de façon significative l'expression de la myostatine dans les cellules musculaires.

### • Les effets sur la synthèse protéique via la phosphorylation de S6K1.

**Tableau 5 : Effets sur la synthèse protéique. Les résultats sont exprimés en pourcentage d'augmentation de la phosphorylation de S6K dans les cellules musculaires. A représente des valeurs supérieures à 130 %, B des valeurs comprises entre 110 et 129 %. Les composés sont testés à la concentration de 10 µM.**

| Numéro | Synthèse protéique | Numéro | Synthèse protéique |
|---|---|---|---|
| **28** | A | **67** | A |
| **32** | B | **76** | B |
| **41** | B | **81** | B |
| **42** | A | **86** | A |
| **43** | B | **88** | B |
| **46** | B | **89** | A |
| **51** | B | **91** | B |
| **52** | B | **92** | B |
| **62** | A | **93** | A |
| **63** | B | **94** | A |

Les 8 composés suivants : 28, 42, 62, 67, 86, 89, 93 et 94 stimulent la phosphorylation de S6K1 de façon très significative à des niveaux équivalents à l'IGF-1 (130-140 %).
Les 12 composés suivants : 32, 41, 43, 46, 51, 52, 63, 76, 81, 88, 91 et 92 stimulent la phosphorylation de S6K1 de façon significative à des niveaux équivalents à celui de la 20E (120 %).

### • Etude des molécules dans un modèle de souris soumises à un régime hyperlipidique

L'étude *in vivo* est réalisée en évaluant l'effet de la 20E en tant que composé comparatif et des molécules selon l'invention (N°51 et 93) administrées par voie orale, à une dose de 5 mg/kg de poids corporel, à des souris C57BL/6 soumises à un régime hyperlipidique pendant 6 semaines. L'effet des molécules sur le poids et la quantité de protéines du muscle *Soleus* ainsi que les transcrits de gènes impliqués dans la myogénèse ont été évalués.

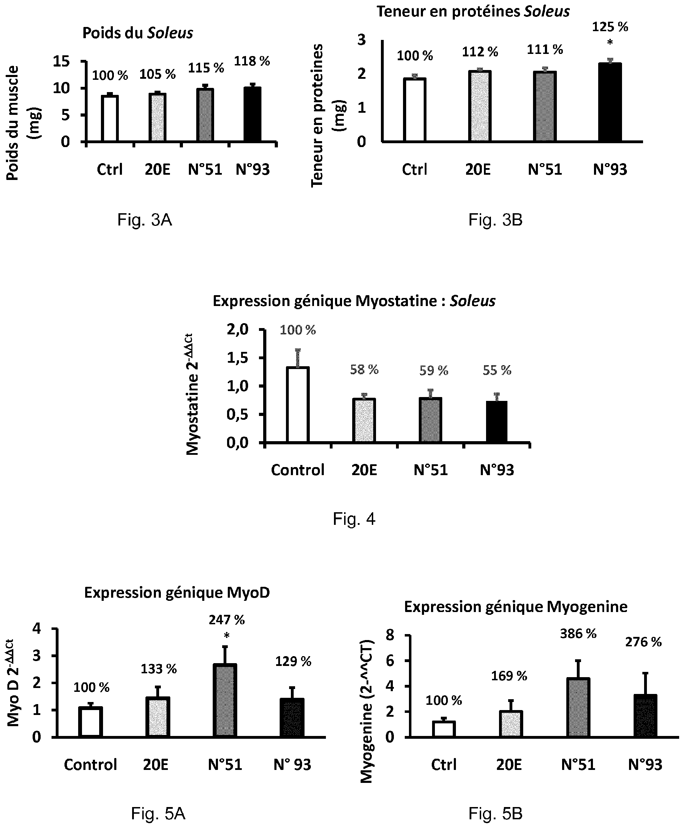

Les effets de la 20E en tant que composé comparatif et des composés selon l'invention n° 51 et 93 sur le poids du muscle sont illustrés en figure 3A et les effets de la 20E et des composés n° 51 et 93 sur la quantité de protéines du muscle *Soleus* sont illustrés en figure 3B.

La 20E et les composés administrés à 5 mg/kg induisent tous 3 des augmentations du poids et de la quantité de protéines du muscle *Soleus* comparé au groupe contrôle. Les composés conformes à l'invention montrent une efficacité aussi importante que celle de la 20E. Même, on note une augmentation significative de la teneur en protéines avec le composé n°93.

Les effets de la 20E en tant que composé comparatif et des composés selon l'invention administrés n° 51 et n° 93, sur le transcrit de la myostatine du muscle *Soleus,* sont illustrés en figure 4.

La 20E et les composés n°51 et 93 inhibent de manière comparable l'expression de la myostatine dans le muscle *Soleus.* Ces molécules inhibaient également le transcrit de la myostatine dans les études *in vitro* des lignées cellulaires C2C12 comme présenté dans le tableau 4 ci-avant.

Les effets de la 20E en tant que composé comparatif et des composés selon l'invention n° 51 et 93 sur les transcrits de MyoD et de la Myogenine, gènes impliqués dans la myogénèse du muscle *Soleus,* sont respectivement illustrés en figure 5A et 5B.

La 20E et les composés N°51 et 93 induisent l'augmentation des transcrits des gènes MyoD qui détermine le potentiel de différentiation et Myf5 impliqué dans la prolifération des myocytes. Ils induisent également l'augmentation du transcrit du gène de la Myogénine impliqué dans la différentiation précoce des myocytes.

### • Etude pharmacocinétique des molécules chez le rat

La pharmacocinétique de la 20E et de composés selon l'invention a été évaluée chez le rat par administration orale à la dose de 10 mg/kg dans le cas des composés et 50mg/kg dans le cas de la 20E.

**Tableau 6 : Principaux paramètres de pharmacocinétique (Tmax ; Cmax et AUC) de la 20E et des composés testés chez le rat Wistar**

| **Composé** | **Dose (mg/kg)** | **Tₘₐₓ (h)** | **Cₘₐₓ (ng/ml)** | **AUC (ng.h/ml)** | **Cexp** |
|---|---|---|---|---|---|
| 20E | 50 | 0.5 | 68 | 382 | 1.0 |
| 31 | 10 | 0.25 | 105 | 281 | 3.7 |
| 46 | 10 | 0.25 | 40 | 202 | 2.6 |
| 51 | 10 | 0.25 | 174 | 273 | 3.6 |
| 93 | 10 | 0.5 | 230 | 785 | 10.3 |

En tenant compte de la dose 5 fois plus importante de 20E (50 mg/kg) par rapport à celle des composés selon l'invention (10 mg/kg), le coefficient d'exposition Cexp [Cexp= (Dose_{20E} x AUC_{composé}) : (Dose_{composé} x AUC_{20E})] met en évidence l'amélioration du profil pharmacocinétique de tous composés testés par rapport à la 20E. Ainsi cette étude chez le rat montre que les composés n° 31 ; 46 ; 51 et 93 présentent une meilleure exposition plasmatique par rapport à la 20E.

### • Bilan

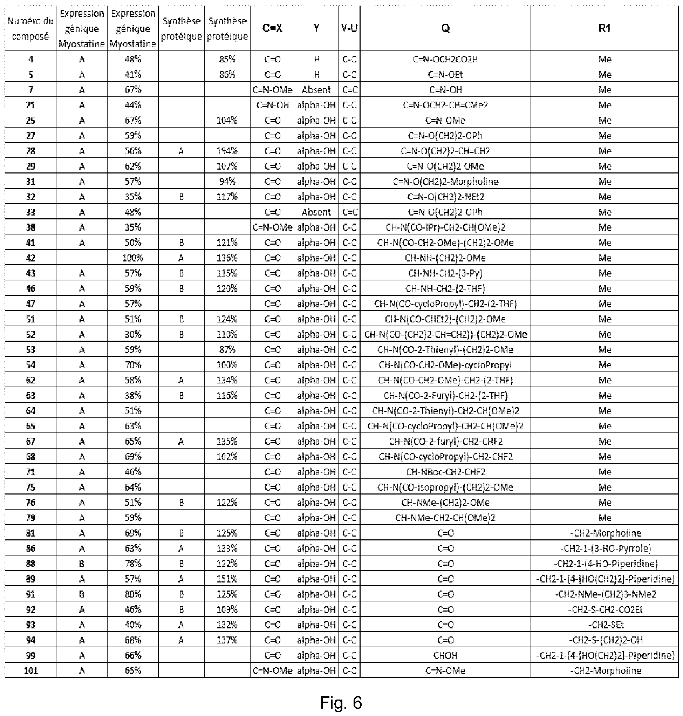

Le tableau illustré en figure 6 expose les résultats obtenus pour des composés de la présente invention lors des expériences d'analyse de l'expression génique de la Myostatine et de la synthèse protéique.

Concernant l'expression génique de la myostatine, les résultats sont exprimés en pourcentage d'expression génique de la myostatine dans les cellules en contact avec les composés rapporté à l'expression dans les cellules contrôle. A représente un pourcentage inférieur à 70 %, B représente un pourcentage entre 71 % et 85 %.

Concernant l'analyse de la synthèse protéique les résultats sont exprimés en pourcentage d'augmentation de la phosphorylation de S6K dans les cellules musculaires. A représente des valeurs supérieures à 130 %, B des valeurs comprises entre 110 et 129 %.

**Tableau 7 : Bilan sur les résultats illustrés en figure 6.**

| **Q** | Nombre d'exemples avec une expression génique Myostatine Catégorie A | Nombre d'exemples avec une synthèse protéique Catégorie A /B | Numéro des meilleurs composés AA | Numéro des meilleurs composés AB |
|---|---|---|---|---|
| C=N**OR⁵** | **12** | **1/1** | **28** | **32** |
| CH**NR²R³** | **19** | **3/7** | **62 et 67** | **41, 43, 46, 51, 63 et 76** |
| **Carbonyle** | **6** | **4/4** | **86, 89, 93 et 94** | **81 et 92** |
| **CHOH** | **1** | | | |

Les produits les plus attractifs sont de catégorie AA ou AB, à savoir de catégorie A pour leur expression génique sur la myostatine associée à une catégorie A ou B en synthèse protéique.

### BIBLIOGRAPHIE

Arounleut P, Bialek P, Liang LF, et al. 2013. A myostatin inhibitor (propeptide-Fc) increases muscle mass and muscle fiber size in aged mice but does not increase bone density or bone strength. Exper Gerontol 48:898-904.
Aubertin-Leheudre M, Lord C, Khalil A, Dionne IJ. 2007. Six months of isoflavone supplement increases fat-free mass in obese-sarcopenic postmenopausal women: a randomized double-blind controlled trial. Eur J Clin Nutr 61 :1442-1444.
Aussel C, Woelffle E, Lemoigne P, Depailler L, Bouillanne O. 2013. Une nouvelle stratégie nutritionnelle pour lutter contre la dénutrition et la sarcopénie : le régime protéique pulsé. Cahiers Nutrition Diététique 48:33-40. Azizov AP, Seifulla RD, Ankudinova IA, Kondrat'eva II, Borisova IG. 1998. Effect of the antioxidants elton and leveton on the physical work capacity of athletes. Eksp Klin Farmakol 61(1):60-62.
Baptista IL, Leal ML, Artioli GG, et al. 2010. Leucine attenuates skeletal muscle wasting via inhibition of ubiquitin ligases. Muscle Nerve 41(6):800-808.
Báthori M, Tóth N, Hunyadi A, Márki A, Zador E. (2008). Phytoecdysteroids and anabolic-androgenic steroids - Structure and effects on Humans. Current Medicinal Chemistry 15:75-91*.*
Bennett BT, Mohamed JS, Alway SE. 2013. Effects of resveratrol on the recovery of muscle mass following disuse in the plantaris muscle of aged rats. PLoS One 8(12):e83518.
Boirie Y, Gachon P, Beaufrère B. 1997. Splanchnic and whole body leucine kinetics in young and elderly men. Am J Clin Nutr. 65:489-495.
Bonnefoy M, Constans T, Ferry M. 2000. Dénutrition du sujet âgé. Influence de la nutrition et de l'activité physique sur le muscle au grand âge. Presse Med 29:2177-2182.
Bonnefoy M. 2008. Interventions pour restaurer la masse musculaire chez le sujet âgé. Nutr Clin Metab 22:80-83.
Buehring B, Binkley N. 2013. Myostatin - the holy grail for muscle, bone, and fat? Curr Osteoporos Rep 11(4):407-414.
Castan-Laurell I, Dray C, Knauf C, Kunduzova O, Valet P. 2012. Apelin, a promising target for type 2 diabetes treatment? Trends Endocrinol Metab 23(5):234-241.
Chermnykh NS, Shimanovsky NL, Shutko GV, Syrov VN. 1988. Effects of methandrostenolone and ecdysterone on physical endurance of animais and protein metabolism in the skeletal muscles. Farmakologiya i Toksikologiya6: 57-62.
Coëffier M, Petit A, Déchelotte P. 2009. Quelle pharmaconutrition pour lutter contre la sarcopénie ? Nutrition Clinique Métabolisme 23:76-79. Collins-Hooper H, Sartori R, Macharia R, et al. 2014. Propeptide-mediated inhibition of myostatin increases muscle mass through inhibiting proteolytic pathways in aged mice. J Gerontol A Biol Sci Med Sci, doi:10.1093/gerona/gh170.
Crenn P. 2013. Sarcopénie et cachexie : approche médicamenteuse. Nutrition Clinique Métabolisme 27:69-73.
de Jager N, Hudson NJ, Reverter A, et al. 2011. Chronic exposure to anabolic steroids induces the muscle expression of oxytocin and a more than fiftyfold increase in circulating oxytocin in cattle. Physiol Genomics 43:467-478.
Dumonceaux J, Marie S, Beley C, Trollet C, Vignaud A, Ferry A, Butler-Browne G, Garcia L. 2010. Combination of myostatin pathway interference and dystrophin rescue enhances tetanic and specific force in dystrophic mdx mice. Mol Ther 18(5): 881-887.
Fiatarone MA, Marks EC, Ryan ND, Meredith CN, Lipsitz LA, Evans WJ. 1990. High-intensity strength training in nonagerians. JAMA 263: 3029-3034. Foucault AS, Mathé V, Lafont R, Even P, Dioh W, Veillet S, Tomé D, Huneau D, Hermier D, Quignard-Boulangé A. 2012. Quinoa extract enriched in 20-hydroxyecdysone protects mice from diet-induced obesity and modulates adipokines expression. Obesity 20:270-277.
Foucault AS, Dioh W, Lafont R, Veillet S, Tomé D, Quignard-Boulangé A, Clément K, Rizkalla S. 2014. 20-Hydroxyecdysone increases android fat mass loss with no significant effect on muscle mass loss during a weight loss program in obese and overweight subjects. ICFSR 2014 International Conference of Frailty and Sarcopenia Research, Barcelone, 12-14/03/2014.
Gadzhieva RM, Portugalov SN, Paniushkin VV, Kondrat'eva II. 1995. A comparative study of the anabolic action of ecdysten, leveton and Prime Plus, preparations of plant origin. Eksp Klin Farmakologiya 58(5): 46-48.
Gilson H, Schakman O, Combaret L, et al. 2007. Myostatin gene deletion prevents glucocorticoid-induced muscle atrophy. Endocrinology 148:452-460. Gorelick-Feldman J, MacLean D, Ilic N, Poulev A, Lila MA, Raskin I. 2008. Phytoecdysteroids increase protein synthesis in skeletal muscle cells. J. Agric. Food Chem. 56: 3532-3537.
Gorelick-Feldman J, Cohick W, Raskin I. 2010. Ecdysteroids elicit a rapid Ca2+ flux leading to Akt activation and increased protein synthesis in skeletal muscle cells. Steroids 70: 632-637.
Greenberg SA. 2012. Pathogenesis and therapy of inclusion body myositis. Curr Opin Neuroil 25(5): 630-639.
Han HQ, Mitch WE. 2011. Targeting the myostatin signaling pathway to treat muscle wasting diseases. Curr Opin Support Palliat Care 5(4):334-341. Hung TJ, Chen WM, Liu SF, et al. 2012. 20-Hydroxyecdysone attenuates TGF-β1-induced renal cellular fibrosis in proximal tubule cells. J Diabetes Complications 26(6):463-469.
Kazi AA, Lang CH 2010. PRAS40 Regulates Protein Synthesis and Cell Cycle in C2C12 Myoblasts Mol Med.16(9-10):359-371*.*
Kizelsztein P, Govorko D, Komarnytsky S, Evans A, Wang Z, Cefalu WT, Raskin I. 2009. 20-Hydroxyecdysone decreases weight and hyperglycemia in a diet-induced obesity mice model. Am. J. Physiol. Endocrinol. Metab. 296:E433-E439.
KnightJDR, Kothary R. 2011. The myogenic kinome: protein kinases critical tomammalian skeletal myogenesis Skeletal Muscle, 1:29, http://www.skeletalmusclejournal.com/content/1/1/29
Lafont R, Clément K, Rizkalla S, Foucault AS, Veillet S, Dioh W. 2013 Phytoecdysones for use in weight stabilization after a weight-loss diet. Demande de brevet PCT WO 2013/068704
Lafont R, Harmatha J, Marion-Poll F, Dinan L, Wilson ID. 2002. Ecdybase, a freeecdysteroiddatabase. http://ecdybase.org
Larock RC. 1989. Comprehensive organic transformations: A guide to functional group preparations. VCH Publishers, New York.
Lawrence MM. 2012. Ajuga turkestanica as a countermeasure against sarcopenia and dynapenia. Ms thesis, Appalachian State University.
Le Grand F, Rudnicki MA. 2007.Skeletal muscle satellite cells and adultmyogenesis. Curr Opin Cell Biol, 19:628-633.
Léger B, Derave W, De Bock K; Hespel P, Russell AP. 2008. Human sarcopenial reveals an increase in SOCS-3 and myostatin and a reduced efficiency of Akt phosphorylation. Rejuvenation Res 11(1):163-175.
Li Z, Heber D. 2011. Sarcopenic obesity in the elderly and strategies for weight management. Nutrition Reviews 70(1):57-64.
Li ZB, Kollias HD, Wagner KR. 2008. Myostatin directly regulates skeletal muscle fibrosis. J. Biol. Chem. 283(28):19371-19378.
Little JP, Phillips SM. 2009. Résistance exercise and nutrition to counteract muscle wasting. Appl Physiol Nutr Metab 34:817-828.
Liu W, Thomas SG, Asa SL, et al. 2003. Myostatin is a skeletal muscle target of growth hormone anabolic action. J Clin Endocr Metab 88(11):5490-5496. Macell TJ, Harman SM, Urban RJ, et al. 2001. Comparison of GH, IGF-I, and testosterone with mRNA of receptors and myostatin in skeletal muscle in older men. Am J Physiol Endocrinol Metab 281 :E1159-E1164.
Murad MH, Elamin KB, Abu Elnour NO, et al. 2011. Clinical review: the effect of vitamin D on falls: a systematic review and meta-analysis. J Clin Endocrinol Metab 96(10):2997-3006.
Murphy KT, Koopman R, Naim T, et al. 2010. Antibody-directed myostatin inhibition in 21-mo-old mice reveals novel roles for myostatin signaling in skeletal muscle structure and function. FASEB J 24:4433-4442.
Pierno S, Tricarico D, Liantonio A, et al. 2014. An olive oil-derived antioxidant mixture ameliorates the age-related decline of skeletal muscle function. AGE 36:73-88.
Quillot D, Böhme P, Malgras P, Malgras A, Ziegler O. 2013. L'obésité du sujet âgé. Nutr Clin Métabolisme 27:95-101.
Ryall JG, Plant DR, Gregorevic P, Silence MN, Lynch GS. 2004. β2-Agonist administration reverses muscle wasting and improves muscle function in aged rats. J Physiol 555(1):175-188.
Ryall JG, Church JE, Lynch GS. 2007. A novel role of β-adrenoreceptor signalling in skeletal muscle growth, development and regeneration. Proc Australian Physiol Soc 40:103-108.
Ryan AS, Li G, Blumenthal JB, Ortmeyer HK. 2013. Aerobic exercise + weight loss decreases skeletal muscle myostatin expression and improves insulin sensitivity in older adults. Obesity 21 (7):1350-1356.
Sabourin LA, Rudnicki MA. 2000. The molecular regulation of myogenesis. Clin Genet 57:16-25.Saini A, Faulkner S, AL-Shanti N, Stewart C. 2009. Powerful signais for weak muscles. Ageing Res Rev 8:251-267.
Sakuma K, Yamaguchi A. 2012. Sarcopenia and age-related endocrine function. Int J Endocrinol, doi:10.1155/2012/127362.
Sattler FR. 2013. Growth hormone in the aging male. Best Practice Res Clin Endocr Metab 27:541-555.
Schaap LA, Pluijm SMF, Deeg DJH, et al. 2009. Higher inflammatory marker levels in older persons: associations with 5-year change in muscle mass and muscle strength. J Gerontol A Biol Sci Med Sci 64A(11):1183-1189. Seidlova-Wuttke D, Erhardt C, Wuttke W. 2010. Metabolic effects of 20-OH ecdysone in ovariectomized rats. J Steroid Biochem Mol Biol 119:121-126. Seidman SN. 2007. Androgens and the aging male. Psychopharmacol Bull 40:205-218.
Shadfar S, Couch ME, McKinney KA, et al. 2011. Oral resveratrol therapy inhibits cancer-induced skeletal muscle and cardiac atrophy in vivo. Nutr Cancer 63(5):749-762.
Simakin SYu, Panyushkin VV, Portugalov SN, Kostina LV, Martisorov EG. 1988. Combined application of preparation Ecdysten and product Bodrost during training in cyclic sports. Sports Science Bulletin N°2, 29-31.
Stenholm S, Alley D, Bandinelli S, et al. 2009. The effect of obesity combined with low muscle strength on decline in mobility in older persons: results from the InCHIANTI study. Int J Obesity 33:635-644.
Syrov VN. 2000. Comparative experimental investigations of the anabolic activity of ecdysteroids and steranabols. Pharm Chem Journal 34(4):193-197.
Syrov VN, Saatov V, Sagdullaev ShSh, Mamatkhanov AU. (2001). Study of the structure - anabolic activity relationship for the phytoecdysteroids extracted from some plants of central Asia. Pharmaceutical Chemistry Journal35: 667-671.
Tchoukouegno Ngueu S. 2013. Estrogenic, cytotoxic and anabolic effects on estrogen target organs of an extract of Erythrina excelsa and ecdysterone. PhD thesis, German Sports University of Cologne.
Tisdale MJ. 2001. Facteurs lipolytiques et protéolytiques de la cachexie cancéreuse. Nutr Clin Métabol 15:266-272.
Todorov IN, Mitrokhin Yul, Efremova OI, Sidorenko LI. 2000. The effect of ecdysterone on the biosynthesis of proteins and nucleic acids in mice. Pharmaceut Chem J 34(9):455-458.
Tóth N, Szabó A, Kacsala P, Héger J, Zádor E. 2008. 20-Hydroxyecdysone increases fiber size in a muscle-specific fashion in rat. Phytomedicine 15:691-698.
Verghese J, Holtzer R, Oh-Park M, et al. 2011. Inflammatory markers and gait speed decline in older adults. J Gerontol A Biol Sci Med Sci 66A:1083-1089.
Walston JD. 2012. Sarcopenia in older adults. Curr. Opinion Rheumatol. 24:623-627.
White JP, Gao S, Puppa MJ, et al. 2013. Testosterone regulation of Akt/mTORC1/FoxO3a signaling in skeletal muscle. Mol Cell Endocrinol 365:174-186.
White TA, LeBrasseur, NK. 2014. Myostatin and sarcopenia: opportunities and challenges - a mini-review. Gerontology, doi:10.1159/000356740. Wuttke W, Seidlovà-Wuttke D. 2013. Pflanzliche Präparate für die Therapie klimaterischer und postmenopausaler Beschwerden und Erkrankungen. Frauenartz 54:580-587.
Zhao J, Brault JJ, Schild A, Goldberg AL. 2008. Coordinate activation of autophagy and the proteasome pathway by FoxO transcription factor. Autophagy 4(3):378-380.
Zhu WM, Zhu HJ, Tian WS, Hao XJ, Pittman Jr CU. 2002. The selective dehydroxylation of 20-hydroxyecdysone by Zn powder and anhydrous acetic acid. Synthetic Communications 32:1385-1391.
Zubeldia JM, Hernández-Santana A, Jiménez-de-Rio M, et al. 2012. In vitro characterization of the efficacy and safety profile of a proprietary Ajuga turkestanica extract. Chinese Medicine 3:215-222.

## Revendications

1. Composé de formule générale (**I**) suivante : 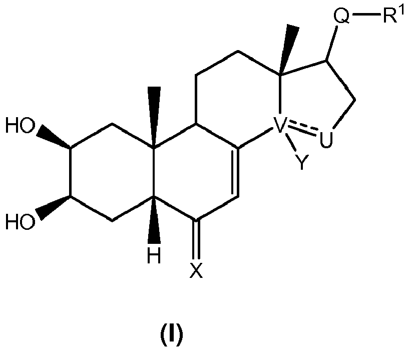 dans laquelle :
**V-U** est une liaison simple carbone-carbone et **Y** est un groupement hydroxyle ou un hydrogène, ou **V-U** est une liaison éthylénique C=C ;
**X** est choisi parmi : un oxygène ; un groupement N-O**R**⁵,
**R⁵** étant alors choisi parmi : un hydrogène ; un groupement alkyle en C₁-C₆ possédant ou non des insaturations sur la chaîne ; un groupement (C₁-C₆)CO2**R⁶** avec **R⁶** pouvant être un hydrogène ou un groupement en C₁-C₆; un groupement (C₁-C₆)O**R⁷**, **R⁷** étant un cycle aromatique ou hétéroaromatique mono ou polysubstitué ou non par un groupement alkyl ou alkoxyl, CF₃, Cl ;un groupement (C₁-C₆)N**R⁸R⁹**, **R⁸** et **R⁹** étant des groupements en C₁-C₆, ou des groupements en (C₁-C₆)N(C₁-C₆) ou des groupements en (C₁-C₆)N(C₁-C₆)O**R⁶** avec **R⁶** défini comme ci-dessus, N**R⁸R⁹** peut aussi être un hétérocycle ;
et dans laquelle :
**Q** est choisi parmi : un groupement C=N**OR⁵**, **R⁵** étant défini comme précédemment ; un groupement CH**NR²R³**,
avec **R¹** étant un groupement alkyle en (C₁-C₆)
avec **R²** et **R³,** identiques ou différents, étant choisis chacun parmi : un atome d'hydrogène ; un groupement alkyle en (C₁-C₆) ; un groupement (C₁-C₆)**W**(C₁-C₆); un groupement cycloalkyle ; un groupement (C₁-C₆)CHF₂ ; un groupement (C₁-C₆)**A** avec **A** représentant un hétérocycle défini comme précédemment ; un groupement de type **COR⁴,**
**R⁴** étant choisi parmi : un groupement alkyle ou cycloalkyle en (C₁-C₆) éventuellement insaturé ; un groupement hétérocyclique de type **A** comme défini précédemment, un groupement aromatique ou hétéroaromatique substitué ou non par un groupement de type OH, OMe, (C₁-C₆), N(C₁-C₆), CO₂(C₁-C₆), CF₃, OCF₃, CN, Cl, F; un groupement (C₁-C₆)**W**(C₁-C₆) ;
**W** étant un hétéroatome choisi parmi N, O et S ;
le composé étant sous forme d'un énantiomère, d'un diastéréoisomère, d'un hydrate, d'un solvate, d'un tautomère, d'un mélange racémique ou d'un sel pharmaceutiquement acceptable.

2. Composé selon la revendication 1, dans lequel dans la formule (I), **Q** représente un groupement C=N**OR⁵**, **R⁵** étant défini comme précédemment.

3. Composé selon la revendication 2, dans lequel dans la formule (I) :
**X** est un oxygène ;
**V-U** est une liaison simple carbone-carbone ;
**Y** est un groupement hydroxyle ;
**R¹** est un groupe méthyle.

4. Composé selon la revendication 1, dans lequel dans la formule (I), **Q** représente un groupement CH**NR²R³**, avec **R²** et **R³**, identiques ou différents, étant choisis chacun parmi : un atome d'hydrogène ; un groupement alkyle en (C₁-C₆) ; un groupement (C₁-C₆)**W**(C₁-C₆); un groupement cycloalkyle ; un groupement (C₁-C₆)CHF₂ ; un groupement (C₁-C₆)**A** avec **A** représentant un hétérocycle défini comme précédemment ; un groupement de type **COR⁴,**
**R⁴** étant choisi parmi : un groupement alkyle ou cycloalkyle en (C₁-C₆) éventuellement insaturé ; un groupement hétérocyclique de type **A** comme défini précédemment, un groupement aromatique ou hétéroaromatique substitué ou non par un groupement de type OH, OMe, (C₁-C₆), N(C₁-C₆), CO₂(C₁-C₆), CF₃, OCF₃, CN, Cl, F ; un groupement (C₁-C₆)**W**(C₁-C₆).

5. Composé selon la revendication 4, dans lequel dans la formule (I) :
**X** est un oxygène ;
**V-U** est une liaison simple carbone-carbone ;
**Y** est un groupement hydroxyle ;
**R¹** est un groupe méthyle ;
**W** est un hétéroatome choisi parmi N, O et S.

6. Composé selon la revendication 1, dans lequel dans la formule (I), **V-U** est une liaison éthylénique C=C.

7. Composé selon la revendication 1, dans lequel dans la formule (I), **X** est un groupement N-O**R⁵**.

8. Composé selon l'une quelconque des revendications 1 à 7, choisi parmi les composés suivants :
- **n°28** : (2S,3R,5R,10R,13R,14S,17S)-17-(N-but-3-énoxy-C-méthyl-carbonimidoyl)-2,3,14-trihydroxy-10,13-diméthyl-2,3,4,5,9,11,12,15,16,17-décahydro-1H-cyclopenta[a]phénanthrèn-6-one
- **n°32** : (2S,3R,5R,10R,13R,14S,17S)-17-(N-(2-diéthylaminoéthoxy)-C-méthyl-carbonimidoyl)-2,3,14-trihydroxy-10,13-diméthyl-2,3,4,5,9,11,12,15,16,17-décahydro-1H-cyclopenta[a]phénanthrèn-6-one
- **n°41** : 2-méthoxy-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide
- **n°42** : (2S,3R,5R,10R,13R,14S,17S)-2,3,14-trihydroxy-17-[1-(2-méthoxyéthylamino)éthyl]-10,13-diméthyl-2,3,4,5,9,11,12,15,16,17-décahydro-1H-cyclopenta[a]phénanthrèn-6-one
- **n°43** : (2S,3R,5R,10R,13R,14S,17S)-2,3,14-trihydroxy-10,13-diméthyl-17-[1-(3-pyridylméthylamino)éthyl]-2,3,4,5,9,11,12,15,16,17-décahydro-1H-cyclopenta[a]phénanthrèn-6-one
- **n°46** : (2S,3R,5R,10R,13R,14S,17S)-2,3,14-trihydroxy-10,13-diméthyl-17-[1-(tétrahydrofuran-2-ylméthylamino)éthyl]-2,3,4,5,9,11,12,15,16,17-décahydro-1H-cyclopenta[a]phénanthrèn-6-one
- **n°51** : 2-éthyl-N-(2-méthoxyéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]butanamide
- **n°62** : 2-méthoxy-N-(tétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]acétamide
- **n°63** : N-(tetratétrahydrofuran-2-ylméthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide
- **n°67** : N-(2,2-difluoroéthyl)-N-[1-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]furan-2-carboxamide
- **n°76** : (2S,3R,5R,10R,13R,14S,17S)-2,3,14-trihydroxy-17-[1-(2-méthoxyéthyl(méthyl)amino)éthyl]-10,13-diméthyl-2,3,4,5,9,11,12,15,16,17-décahydro-1H-cyclopenta[a]phénanthrèn-6-one

9. Composé selon l'une quelconque des revendications 1 à 8, en tant que médicament.

10. Composé selon l'une quelconque des revendications 1 à 9, pour son utilisation dans le traitement et/ou la prévention de la sarcopénie et en particulier de l'obésité sarcopénique, de ses complications et/ou pathologies associées telles que la perte de force, de masse musculaire, de performances et capacité physiques et de mobilité chez le mammifère.

11. Composé selon l'une quelconque des revendications 1 à 9, pour son utilisation dans le traitement et/ou la prévention de l'obésité et de ses complications et/ou des pathologies associées, avantageusement du diabète de type 2 ou du syndrome métabolique chez le mammifère.
